# EUROPEAN PATENT SPECIFICATION

(11) **EP 3 626 721 B1**
(45) Date of publication and mention of the grant of the patent: **06.09.2023**
(21) Application number: 19189318.9
(22) Date of filing: 31.07.2019
(51) Int. Cl.: C07D 499/78, C07D 501/16, C07D 501/60, A61P 33/00, A61P 35/00

(54) **SYNTHESIS OF ESTER AND AMIDE DERIVATIVES OF BETA-LACTAM NUCLEI**
SYNTHESE VON ESTER- UND AMID-DERIVATEN VON BETA-LACTAM-KERNEN
SYNTHÈSE DE DÉRIVÉS D'ESTER ET D'AMIDE DE NOYAUX BETA LACTAME

(30) Priority: 31.07.2018 IT 201800007656
(43) Date of publication of application: 25.03.2020
(73) Proprietor: Bonomi, Paolo, 94700 Maisons Alfort (FR)
(72) Inventor: Bonomi, Paolo, 94700 Maisons Alfort (FR)
(74) Representative: De Gregori, Antonella

(56) References cited:
- EP-A1- 0 331 395
- EP-A2- 0 059 554
- WO-A1-2015/049546
- WO-A1-2017/153892
- JP-B1- S5 116 434
- D. C. HUMBER ET AL: "Synthesis and Structure Activity Relationships of a Series of Penicillin-Derived Pseudosymmetric Inhibitors of HIV-1 Proteinase", ANTIVIRAL CHEMISTRY & CHEMOTHERAPY, vol. 5, no. 3, 1 June 1994 (1994-06-01), pages 197-200, XP055662186, England ISSN: 2040-2066, DOI: 10.1177/095632029400500311

## Description

The present invention relates to a synthetic multi-step strategy for the preparation of new ester and amide derivatives of beta-lactam nuclei with the aim of creating a library of new molecules with a possible pharmacological activity, and the compounds thus obtained. The present invention relates to a method for the synthesis of ester derivatives of β-lactam nuclei comprising one or two azide groups.

The present invention relates to a method for the synthesis of amide derivatives of β-lactam nuclei, namely of secondary amide derivatives of β-lactam nuclei comprising an azide group and tertiary amide derivatives of β-lactam nuclei containing two azide groups or two propargyl groups (Scheme 1).

The present invention relates to the cycloaddition reaction catalyzed by copper to give new beta-lactam derivatives starting from azide esters and amides and from dipropargyl tertiary amides to obtain new derivatives of β-lactam nuclei containing differently functionalized triazoles (Scheme 1).

The present invention also relates to a process for the synthesis of β-lactam nuclei containing differently functionalized triazoles or epoxides starting from said precursors, the relative precursors and derivatives.

The present invention overcomes the problems relating to the preparation of a library of new beta-lactam derivatives (scheme 1) from two different points of view:
- overcoming the limits that characterize the invention described in WO 2017/153892 A1 (with respect to both the synthesis of derivatives of the Cephalosporanic nucleus (II), and also with respect to the stability of the products. Amide derivatives are more resistant than ester derivatives)
- creating new difunctional derivatives never previously described in the state of the art.

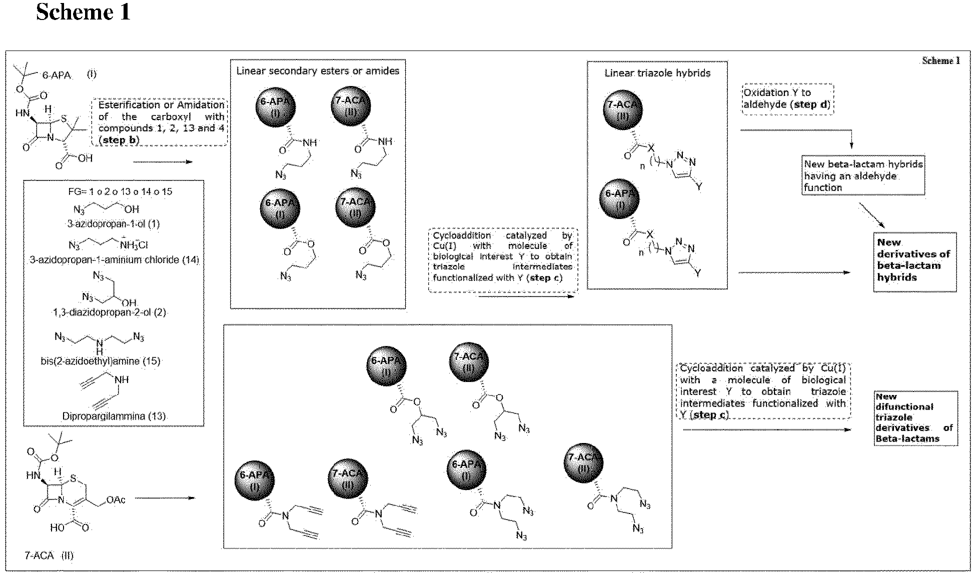

Antibiotic resistance by bacteria and fungi represents a problem of ever-increasing importance and is one of the most important challenges to be faced in the years to come. (*http:*//*www.who.int*/*medicines*/*publications*/*global-priority-list-antibiotic-resistant-bacteria*/*en*/*.*) The indiscriminate use of antibiotics, in fact, especially in the past, has led to the formation of new bacteria resistant not only to a specific antibiotic, but also to different classes of antibiotics. It is estimated that in the next few decades the number of deaths due to bacterial infections that cannot be controlled with currently available antibiotics can increase exponentially, to the point of reporting the number of non-curable infections in the period prior to the discovery of penicillins (Lee Ventola, MS.; « The Antibiotic Resistance Crisis Part 1: Causes and Threats »Pharmacology and Therapeutics 2015, 40 (4), 277-283*)*.

New strategies must therefore be sought for creating new antibiotics, or remodelling "old" antibiotics in order to create more efficient ones (Pedro MSD Cala et al.; "Trends in therapeutic drug conjugates for bacterial diseases: a patent review" Expert Opinion on Therapeutic Patents, 2017 vol. 27, No. 2, 179-189*).*

New beta-lactamase inhibitors, other than beta-lactam nuclei, have recently been introduced onto the market, such as, for example, Avibactam (D. Y. Wang et al.; "The road to avibactam: the first clinically useful non-β-lactam working somewhat like a β-lactam" Future Medicinal Chemistry 2016, 8 (10), 1063-1084). Also in this case, the first signs of bacterial resistance against this inhibitor have been found (RM Humphries et al.; "First Report of Ceftazidime-Avibactam Resistance in a KPC-3-Expressing Klebsiella pneumoniae Isolate" Antimicrobial Agents and Chemotherapy 2015 Vol. 59 Number 10, 6605-6607).

The possibility of chemically modifying the β-lactam nuclei is therefore extremely important for synthesizing new compounds with an antibiotic activity, capable of counteracting these new bacteria or multi-resistant modified bacteria.

In this context, this invention is based on a synthetic strategy comprising multiple steps, for modifying the beta-lactam nuclei 6-APA (I) and 7-ACA (II), protected as tertbutyl carbonates on the amino group, with the addition of molecules having their own biological activity in the carboxyl position.

This synthetic platform must make the preparation of new molecules or bio-adducts accessible, specifically containing the beta-lactam nucleus intact, but functionalized with molecules having a different or the same pharmacological activity, with the objective of both enhancing and modulating their activity, efficacy and absorption with respect to bacteria, mycobacteria, fungi and parasites, and increasing their resistance to bacterial defense systems.

The antibiotic activity of β-lactam derivatives is historically and scientifically associated with the chemical reactivity of the β-lactam ring, but it also varies depending on the substituents present on this ring, in particular in relation to the substituents of the amino group.

It is known however that the nature of the substituents in positions C-3 and C-4 also affects the biological activity of the β-lactam ring (N. Arya, European Journal of Medicinal Chemistry, 2014, 74, 619-656).

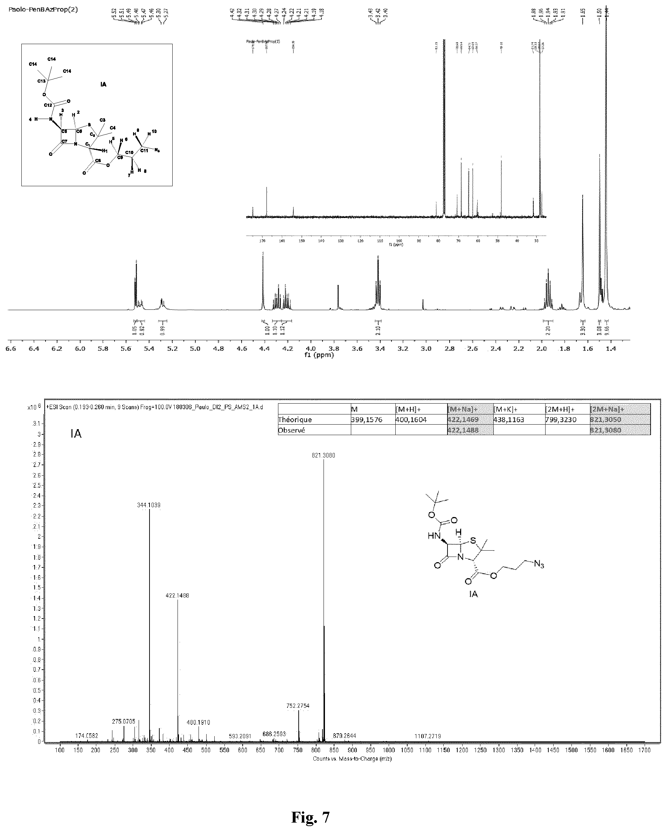

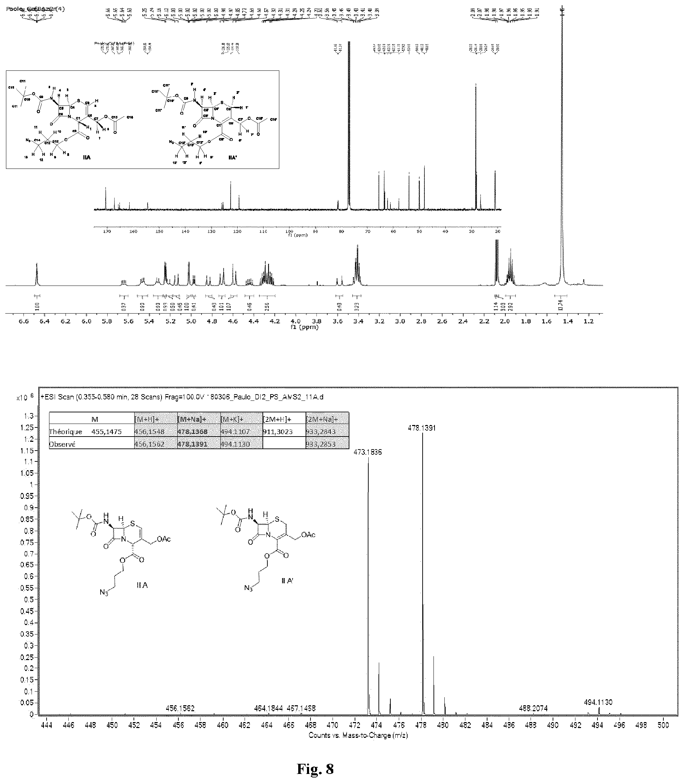

Bicyclic β-lactam antibiotics, such as Penicillins and Cephalosporins, are, in fact, composed of a β-lactam ring fused with a thiazolidine ring having 5 or 6 atoms, with the protons all in cis position with respect to each other. The structural tension due to the fusion of the two cycles is of great importance for the reactivity of these antibiotics. There are also an amide group in position 6 or 7 and a carboxyl group (the positions are numbered following the number shown in Figure 7 for nucleus I and in Figure 8 for nucleus II). In particular, the activity of penicillins strongly depends on the substituents in position 6, whereas the carboxylic group is relevant for allowing modifications to be made to the molecule and generating pro-drugs.

With respect to the relationship between structure and activity of penicillins, the most important characteristics are therefore:
- the cis relationship between the protons in position 5 and 6 on the beta-lactam ring;
- the bicyclic structure;
- the carboxyl group;
- the substituents of the amino group in position 6 for nucleus I and in position 7 for nucleus II.

In general, hydrophobic substituent groups tend to increase the activity of penicillin towards Gram+ bacteria, whereas hydrophilic substituent groups increase the activity towards Gram- bacteria.

The attachment of a substituent on the carboxyl group allows, for example, pro-drugs to be generated (Karen Bush "CHAPTER 14 - β-Lactam antibiotics: penicillins" Antibiotic and Chemotherapy (Ninth Edition) 2010, Pages 200-225).

In general, β-lactam antibiotics have a free carboxyl and negatively charged substituents, but the spreading of bacterial strains resistant to many of these antibiotics has made it necessary to identify synthetic routes and precursors or derivatives of β-lactam compounds having suitable structural characteristics, which allow their use as a starting point for generating new drugs and also for identifying the relationships between these compounds and bacterial enzymes.

In addition to the antibacterial activity indicated above, β-lactam derivatives are, in fact, characterized by also having other interesting properties at a biological level: they act as inhibitors of cholesterol absorption, as specific antigens when bound to proteins and are also syntons for the preparation of anticancer compounds such as Taxol.

Finally, for some β-lactam derivatives a direct activity in the treatment of cancer has also been demonstrated (DB, Boggian et al. "A solid- and solution phase-based library of 2β-methyl substituted penicillin derivatives and their effects on growth inhibition of tumor cell lines»Med. Chem. Commun., 2015, 6, 619-625, Geesala, R. et al. «2-Azetidinones: Synthesis and biological evaluation as potential anti-breast cancer agents.» Eur..Med. Chem. 2016, 124, 544-558, R. Reddy, CB et al. "HDAC and NF-κB mediated cytotoxicity induced by novel N-Chloro β-lactams and benzisoxazole derivatives." Chem-Biol., 246, 69-76, Galletti, P. et al. "Targeting integrins αvβ3 and α5β1 with new β-lactam derivatives." Eur. J. Med. Chem. 2014, 83, 284-293).

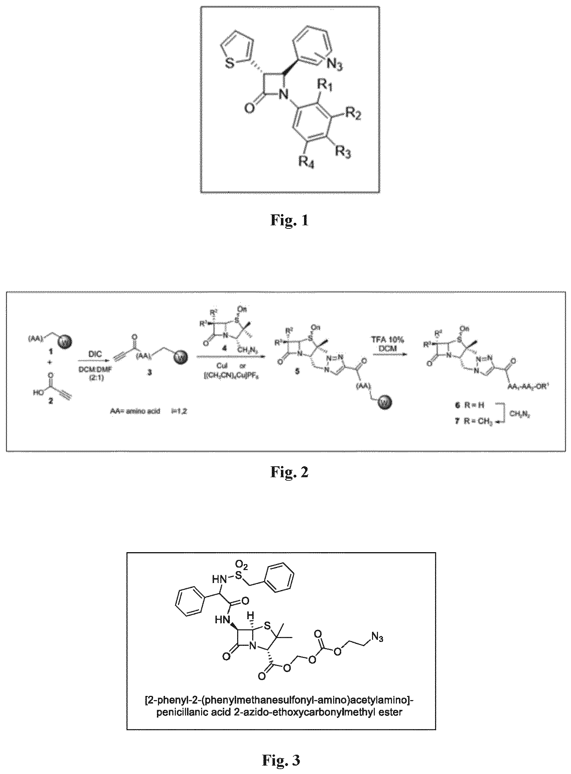

In 2017 Dong-Jun Fu and colleagues investigated the anticancer properties of azide derivatives of monolactams of which a general formula is provided in Figure 1. The azide group seems necessary for the effective inhibition of the polymerization of tubulin in cancer cells with consequent apoptosis of tumour cells (Dong-Jun Fu et al.; «Structure-Activity Relationship Studies of β-Lactam-azide Analogues as Orally Active Antitumor Agents Targeting the Tubulin Colchicine Site »Scientific Reports, 2017, | 7: 12788 | 1-12*)*.

Following recent discoveries concerning the anticancer properties of some beta-lactam derivatives, the possibility of obtaining new molecules containing this structure can allow the preparation of new anticancer molecules.

In general, the preparation of β-lactam derivatives follows some fundamental synthesis strategies:
- chemical synthesis exerted through the Staudinger reaction: a [2+2] cycloaddition of a ketene with an imine (C. Palomo et al., "Asymmetric Synthesis of β-Lactams by Staudinger Ketene-Imine Cicloaddition Reaction" 1999 , Eur.J. Org. Chem., 3223-3235) and many other synthetic strategies as described in the work (CR Pitts and T. Lectka "Chemical Synthesis of β-Lactams: Asymmetric Catalysis and Other Recent Advances", Chem. Rev., 2014, 114 (16), pages. 7930-795);
- chemo-enzymatic synthesis mediated by Penicillin G. Acylase (PGA) by E. Coli, which focuses, above all, on the acetylation of the amino group (P. Bonomi et al., «Modulation of the Microenvironment Surrounding the Active Site of Penicillin G Acylase Immobilized on Acrylic Carriers Improves the Enzymatic Synthesis of Cephalosporins »Molecules, 2013, 18, 14349-14365*;*
- biosynthesis mediated by enzymes such as Isopenicillin N Synthase (N.I. Burzlaff et al, "The reaction cycle of isopenicillin N synthase observed by X-ray diffraction." Nature, 1999, 401, 721-724);
- solid phase synthesis with the introduction of triazole groups on the carboxyl, previously transformed into an alcohol function. In particular, a library of triazolyl aminoacyl (peptidyl) penicillins was produced and its antineoplastic activity was studied (PG Cornier, et al., «In vitro anticancer activity and SAR studies of triazolyl aminoacyl (peptidyl) penicillins» Med. Chem Community, 2014, 5, 214-218*).* These derivatives were obtained through a "click reaction" mediated by copper I, where, however, the azide was introduced with carbonyl reduction according to the reaction scheme shown in Figure 2.

Very few examples of functionalization of the carboxyl group with compounds containing an azide group are reported in literature. In particular, the nuclei 6-APA (I) and 7-ACA (II) have been functionalized with a « 2-azido-ethoxycarbonyloxymethyl ester» as described in Astra Lakemedel Aktiebolag "Penicillin esters and methods and compositions for treating infectious diseases "US3931405, A, SE Dahlenet al.," Dipenicillin and dicephalosporin ester antibiotics "1975, US3928595, A, An example of these compounds is shown in Figure 3.

The search for new antibacterial drugs is currently of vital importance and the possibility of synthesizing new non-traditional molecules allows new therapeutic agents to be designed and obtained (Pedro M. S. D. Cala et al.; "Trends in therapeutic drug conjugates for bacterial diseases: a patent review" Expert Opinion on Therapeutic Patents, 2017 Vol. 27, No. 2, 179-189*).*

The patent application *"Synthesis process of precursors of derivatives of β-lactam nuclei and precursors and derivatives thereof"* WO 2017/153892 A1, previously mentioned, describes the possibility of preparing a propargyl ester of two protected beta-lactam nuclei such as di-tert-carbamates. In addition, the penicillanic nucleus, having an alkyne function, was subjected to the 1-3 dipolar cycloaddition reaction catalyzed by copper, with the objective of introducing a triazole having interesting pharmacological activities at the carboxyl level (*s.*Malik et al. "Pharmacological Importance of Triazole Derivatives: A Review" IJESC, 2016, Vol 6 Issue No. 8, 2799-2803*).* This work, which is based on the reactivity of the alkyne group inserted at the level of the carboxyl, can be implemented and overcome by inserting, for example, a different reactive group that can be used in both cycloaddition reactions and for other reactions otherwise impossible with the propargyl group alone.

In particular it should be pointed out that in WO'892 the difficulties encountered in producing and isolating the propargyl ester of the nucleus II did not allow any triazole derivative to be obtained.

The Applicant, however, has surprisingly found that the drawbacks of the prior art have been overcome, as indicated above, by the process for the synthesis of the precursors of derivatives of beta-lactam compounds according to the enclosed claims 3-10, i.e. by transforming the carboxyl group of nuclei I and II into secondary esters and amides, containing an azide group, using 3-azido-1-propanol (1) or 3-azidopropan-1-ammonium chloride (14) as nucleophile, according to what is reported in the following scheme 2.

Furthermore, the carboxyl groups of nuclei I and II were then esterified with 1,3-diazidoisopropanol (2) (according to what is reported in the following scheme 2b) or transformed into tertiary amides containing two azide groups using bis (2-azidoethyl)amine (compound 15, scheme 2b) or, not according to the invention into tertiary amides containing two propargyl groups with the use of dipropargylamine (compound 13, Scheme 2b), thus obtaining β-lactam derivatives activated with two azide groups (the esters IJ, IIJ and IIJ ' and the tertiary amides IN, IIN and IIN') or, not according to the invention with two propargyl groups (IG, IIG and IIG '). The presence of this double functionalization in these precursors, i.e. di-azide esters IJ, IIJ and IIJ' and the tertiary amides IN, IIN and IIN' (and IG, IIG and IIG' not according to the invention), allows branched structures of beta-lactam antibiotics to be prepared, not described in the state of the art, containing a double triazole functionality.

It should also be noted that the substituted 1,2,3-triazoles are extremely important in coordinating transition metals for preparing new molecules with interesting therapeutic activities (K. Bozorov et al.; "1,2,3-Triazole-containing hybrids as leads in medicinal chemistry: A recent overview "Bioorganic & Medicinal Chemistry, https://doi.org/10.1016/j.bmc.2019.07.005).

The compounds IJ, IN, IIN and IIN', (IG, IIG and IIG' not according to the present invention), are the first examples of beta-lactam antibiotics containing precursors for a double triazole functionality and therefore for new compounds with a high chelating activity for transition metals. Branched structures are emerging as a class of compounds of high pharmacological interest and to date no beta-lactam compounds with a branched structure of this type are known.

It should be pointed out that a branched structure such as polyamidoamine (PAMAM) dendrimers allows pharmacological properties to be obtained that are useful for different applications in the biomedical field (Amy M. Holmes et al. "Antimicrobial efficacy and mechanism of action of poly(amidoamine) (PAMAM) dendrimers against opportunistic pathogens "2019, International Journal of Antimicrobial Agents, 53, 500-507).

In the light of the reaction yields and considering the reaction conditions described in WO'892 for the esterification (diethyl ether as solvent and paratoluenesulfonyl chloride, methanesulfonyl chloride or diethyl phosphate chloride as activating reagent of the carboxyl group), this result is absolutely surprising.

A skilled person in the field would in fact be led to discard the use of azidopropanol as nucleophile. One would expect, in fact, that the polarity of the azide group in such a small saturated aliphatic chain (3 carbon atoms) could cause an interaction with the chloride of the three different activators (para-toluenesulfonylchloride, mesylenechloride and diethyl phosphate chloride) with a consequent deterioration in the esterification yield.

Furthermore, the low propensity of diethyl ether, with respect to a dichloromethane, to solvate compounds having a more marked polarity, would induce the choice of an alcohol containing a propargyl group (propargyl alcohol) as nucleophile rather than an alcohol with a group having a greater polarity such as azide (azidopropanol, compound 1).

Organic chemistry has a wide variety of examples in which similar functional groups have a completely different reactivity due to internal structural differences, or reactions that take place in a solvent, whereas they are impossible or give different results in other solvents. In literature, esterification reactions are mainly carried out in solvents such as dichloromethane and not in diethyl ether.

The physico-chemical properties of the solvent in the case of the cephalosporan nucleus (7-ACA), for example, affect the relationship between the two stereoisomers formed in the esterification or amidation reaction. In the case of esterification in diethyl ether, there is, in fact, a mixture containing 85% of the isomer IIA with respect to IIA' whereas, in the case of the amidation process in dichloromethane, 70% of the isomer IIL' is obtained with respect to IIL (Scheme 2).

In conclusion, in the light of the state of the art and his own general knowledge, a skilled person in the field would expect that in the esterification process on the cephalosporanic nucleus, a greater reaction yield would be obtained with the use of propargyl alcohol as described in WO'892 for two reasons:
- the lower polarity of the propargyl group with respect to the azide group would favour a greater esterification reaction yield, reducing the dipolar interaction with the sulfonyl chloride or phosphate chloride of the activating groups and
- the possible intermolecular interaction of the triple bond of propargyl alcohol with the double bond of the cephalosporanic nucleus (Van der Waals mainly due to the π-π interactions) could facilitate the positioning of the alcohol group of propargyl alcohol, in the attachment to the activated carboxyl group of the beta-lactam nucleus. π-π interactions, known since the beginning of the twentieth century, control many molecular phenomena: the tertiary structure of proteins, the preferential conformation and the "binding" properties of polyaromatic macrocycles, the vertical interaction between the DNA bases, etc. (C.A Hunter et al.; "The Nature of π-π interactions" 1990, JACS, 112, 5525-5534)*.* In the case of terminal alkynes, the solvent can determine a different orientation of the alkyne groups on the surface of pyrolytic graphite causing a different reaction outcome: polymerization or dimerization (X. Zhang et al.; « Polymerization or Cyclic Dimerization: Solvent Dependent Homo-Coupling of Terminal Alkynes at HOPG Surface» 2014, SCIENTIFIC REPORTS|4:3899|DOI:10.1038/srep03899).

The process according to the present invention is also particularly versatile and flexible and allows simple dimers of beta-lactam derivatives (IC, IIC and IIC') scheme 5 to be obtained, or beta-lactam intermediates with a branched structure (IJ, IIJ and IIJ', IN, IIN and IIN', (IG, IIG and IIG', not according to the invention,) useful for developing possible macrocyclic structures:

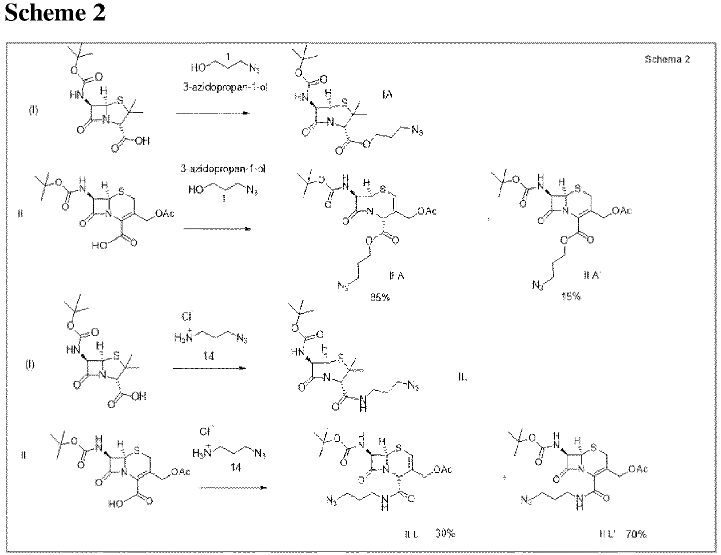

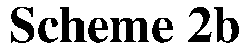

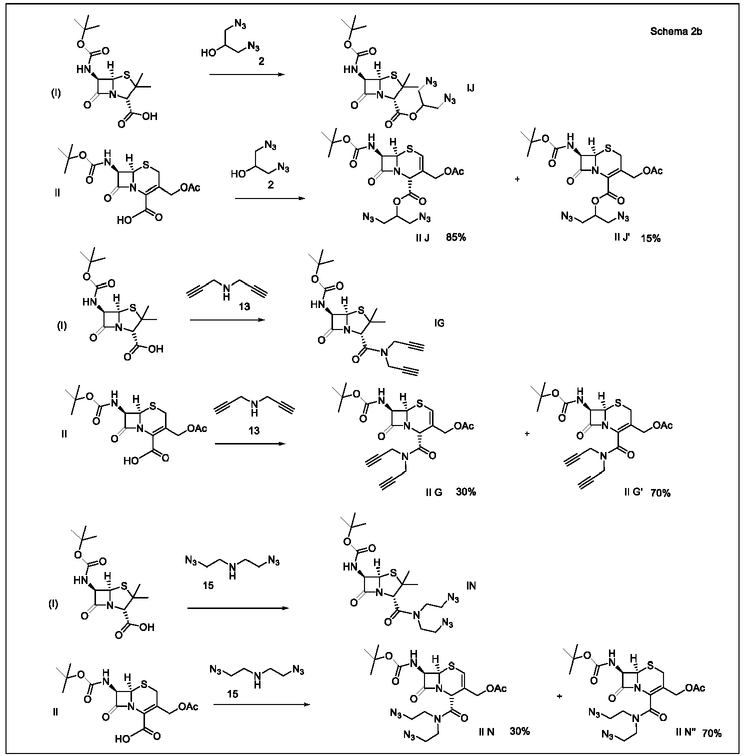

With respect to the importance of the azide group, first of all, the recent discoveries on the anticancer activity of some azide derivatives of monolactams (Figure 1) should be recorded (Dong-Jun Fu et al.; «Structure-Activity Relationship Studies of β-Lactam-azide Analogues as Orally Active Antitumor Agents Targeting the Tubulin Colchicine Site »Scientific Reports 2017,|7:12788| 1-12).

Furthermore, it should be remembered that certain pharmacologically interesting substrates can be more easily functionalized with an alkyne (to give cycloadditions with an azide as shown in scheme 1) rather than with an azide useful for giving cycloaddition with the propargyl ester of I and II described in *"Synthesis process of precursors of derivatives of β-lactam nuclei and precursors and derivatives thereof*" WO 2017/153892 Al.

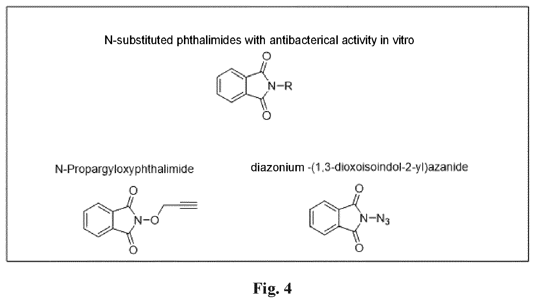

In this respect, some phthalimide derivatives (Figure 4) have recently been evaluated for their antibacterial properties (PF Lamie et al.; "Design, Synthesis and Evaluation of Novel Phthalimide Derivatives as in Vitro Anti-Microbial, Anti-Oxidant and Anti-Inflammatory Agents "Molecules 2015, 20, 16620-16642)*.*

The propargyl derivative *«N-Propargylphthalimide»* is available on the market, whereas the corresponding azide derivative «diazonium-(1,3-dioxoisoindol-2-yl)azanide» is not currently commercially available. In addition, the possibility of having the azide derivatives of beta-lactam nuclei reduces costs, as in the case of the compound shown in Figure 4.

It is important, in fact, to have the possibility of finding on the market and at a reasonable price, compounds that react with the functional group (azide or propargyl) inserted on the beta-lactam nucleus, in order to have rapid and economically sustainable access to a library of molecules with a possible and different pharmacological activity.

The extreme importance of having an ester or a secondary amide containing the azide group is linked to the possibility of synthesizing new beta-lactams that could not otherwise be prepared. The compounds described hereunder IC, IIC and IIC' (diagram 4), for example, could not be synthesized unless at least one azide derivative (ester or amide) is available.

Furthermore, it should be pointed out that the azide group not only gives access to the cycloaddition reaction, but also allows reactions that are not possible with the propargyl group. The Villarasa-Staudinger reaction, for example, allows the formation of an amide group from an azide and a carboxylic acid (J. Vilarrasa et al., "Catalytic Staudinger-Vilarrasa Reaction for the Direct Ligation of Carboxylic Acids and Azides" J. Org. Chem., 2009, 74, 2203-2206*).*

The Staudinger reaction, on the other hand, reduces the azide to amide. This reaction is extremely important, for example, for the selective functionalization of carbohydrates (S. Liu et al.; "Staudinger Reactions for Selective Functionalization of Polysaccharides: A Review" Biomacromolecules, 2015, 16 (9), pp. 2556-2571).

Finally, the advantage relating to the preparation of beta-lactam derivatives I and II containing two azide groups or two propargyl groups is to be able to prepare branched structures capable of giving a cycloaddition reaction catalyzed by copper (I) which, to date, are still unknown. This "branching" allows not only more complex structures with different functionalizations to be created, but also macrocyclic structures with significant effects on the structure-activity relationship of the molecules.

The chelating capacity of triazoles against metals, for example, is well-known and documented (D. Huang et al.; "Catalysis by 1,2,3-triazole- and related transition-metal complexes" Coordination Chemistry Reviews 272 (2014) 145 -165).

1,2,3 substituted triazoles have recently been studied for their capacity of chelating zinc II (HA Michaels et al.; "2-Anthryltriazolyl-Containing Multidentate Ligands: Zinc-Coordination Mediated Photophysical Processes and Potential in Live-Cell Imaging Applications" Inorg. Chem. 2010, 49, 4278-4287).

The presence of two triazoles is of extreme interest as it allows a tetradentate coordination which is not possible to obtain with a single triazole.

In this respect, it should be remembered that the most dangerous metal beta-lactamases are those capable of chelating metals, in particular zinc II, to exert their hydrolytic reaction on beta-lactam nuclei (T. Palzkill "Metallo-β-lactamase structure and function" Ann. NY Acad. Sci. 2013 January; 1277:91-104. Doi:10.1111/j.1749-6632.2012.06796.x).

Consequently, beta-lactam derivatives with a dual incorporated triazole function could be of great help in inhibiting this type of metal-beta-lactamase.

It is extremely important to note, for example, that in the search for new classes of antibiotics, a new class of molecules known as "dendrimeric peptides" has recently raised great interest. These peptides bound to a branched nucleus have antibiotic, antifungal and even anticancer activities (M.A.Scorciapino et al; "Antimicrobial Dendrimeric Peptides: Structure, Activity and New Therapeutic Applications" 2017, Int. J. Mol. Sci., 18, 542).

It is well known, for example, that branched amino acids have a better capacity of recognizing enveloped RNA and binding to it. This property is of vital importance for discovering new drugs against HIV (J.E.Wynn and W.L. Santos al., "HIV-1 drug discovery: targeting folded RNA structures with branched peptides". Biomol. Chem., 2015, 13, 5848 -5858). Branched oligosaccharides of «Fucoidan» show more marked biological activities than the linear structure (MJ.Clément et al.; «NMR characterization and molecular modeling of fucoidan showing the importance of oligosaccharide branching in its anticomplementary activity» 894). The branching of peptides allows their biological activity to be increased and also their resistance to proteolysis (C. Falciani et al.; "Synthesis and biological activity of stable branched neurotensin peptides for tumor targeting" 2007, Mol.Cancer. Ther. 6 (9), 2441-2447). Furthermore the branching can deform the supramolecular structure of the lipopolysaccharides of the bacterial extracellular membrane. The ramification of peptides can therefore be a method for creating new antibiotics (R. Lakshminarayanan et al.; "Branched Peptide, B2088, Disrupts the Supramolecular Organization of Lipopolysaccharides and Sensitizes the Gram-negative Bacteria" 2016, Scientific Reports volume 6, Article number:25905). Branched fatty acids are capable of inhibiting Menaquinone (MK) in Helicobacter Pylori (R. Tanaka et al. "Branched fatty acids inhibit the biosynthesis of menaquinone in Helicobacter pylori" 2011, The Journal of Antibiotics volume 64, pages 151- 153).

In the case of polyester compounds, as reported in literature in the work of R. D'Arcy of 2017, creating a branched structure involves a series of advantages: a) a higher degree of functionalization, which is not only essential for a better conjugation/complexation of a compound with a therapeutic activity, but also for a better exposure of the portions of the molecule necessary for the recognition of a precise target, b) greater stability of micellar systems, c) reduction of viscosity at high concentrations (R. D'Arcy and others "Branched polyesters: Preparative strategies and applications". 2016, Adv. Drug. Delivery Rev. Vol.107, 60- 81).

In spite of ample literature relating to the importance of "branching" for modulating the biological activity of compounds with therapeutic application, very surprisingly no structures are reported in literature of beta-lactam derivatives functionalized with double azide or propargyl units.

Consequently, in solving some of the problems and drawbacks found in WO'892 and in filling evident gaps in the field of beta-lactam derivatives, the present invention involves the creation of advantages such as:
1) the possibility of creating new beta-lactam derivatives starting from nuclei I and II through the esterification or amidation with an azide group (different from the propargyl group) and the preparation of esters containing a double azide group or tertiary amides containing a double azide group or a double propargyl group in order to obtain branched structures. These new products can be either molecules with their own pharmacological activity, or structures that can be used as starting material to create new beta-lactam derivatives through subsequent steps to create an economical and versatile synthetic platform;
2) the formation of beta-lactam derivatives otherwise impossible to obtain, such as IC and IIC;
3) improved esterification yield, especially with respect to the cephalosporan nucleus II;
4) the preparation of secondary and tertiary amides by means of an easy amidation reaction using thionyl chloride described in literature. (A. Leggio et al., "One-pot synthesis of ammides from carboxylic acids activated using thionyl chloride" RSC Adv., 201.6, 34468-34475*).*

With respect to point 1, it has been shown that this synthetic route allows the preparation of new hybrids that can also be subjected to subsequent modifications in order to introduce new reactive groups.

Consequently, in schemes 2 and 2b indicated above, the esterification of the above-mentioned beta-lactam nuclei is described with 3-azido-1-propanol (1) and 1,3-diazidopropan-2-ol (2) to obtain compounds IA, IIA and IIA' and IJ, IIJ and IIJ'.

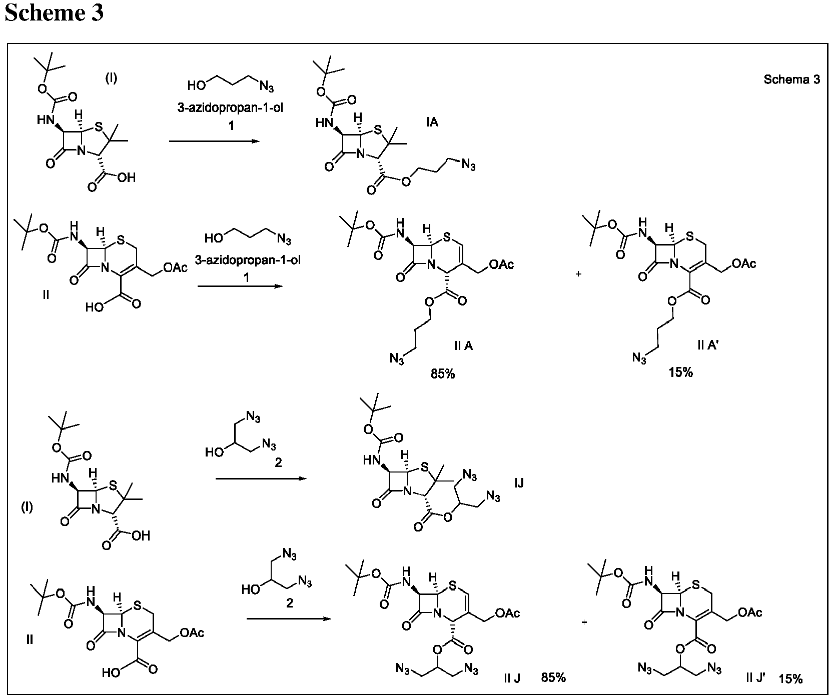

The following are cycloaddition reactions with different substrates to create new beta-lactam derivatives IB, IIB and IIB' (scheme 4), IC, IIC and IIC' (scheme 5), ID, IID and IID' (Scheme 6).

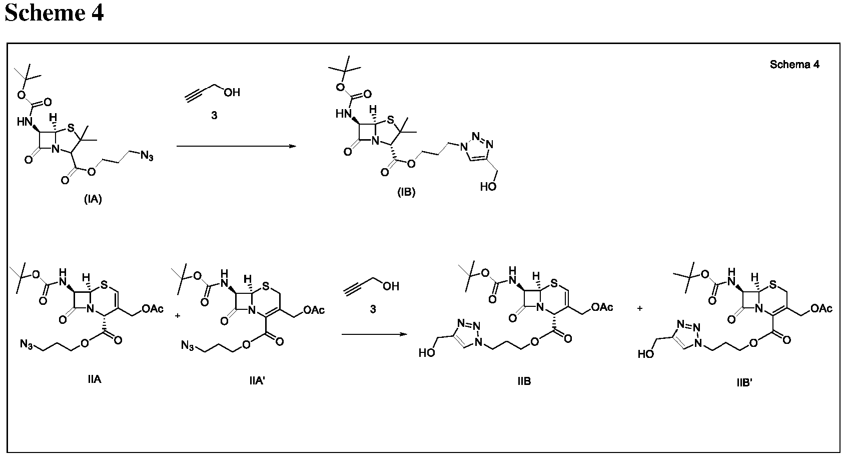

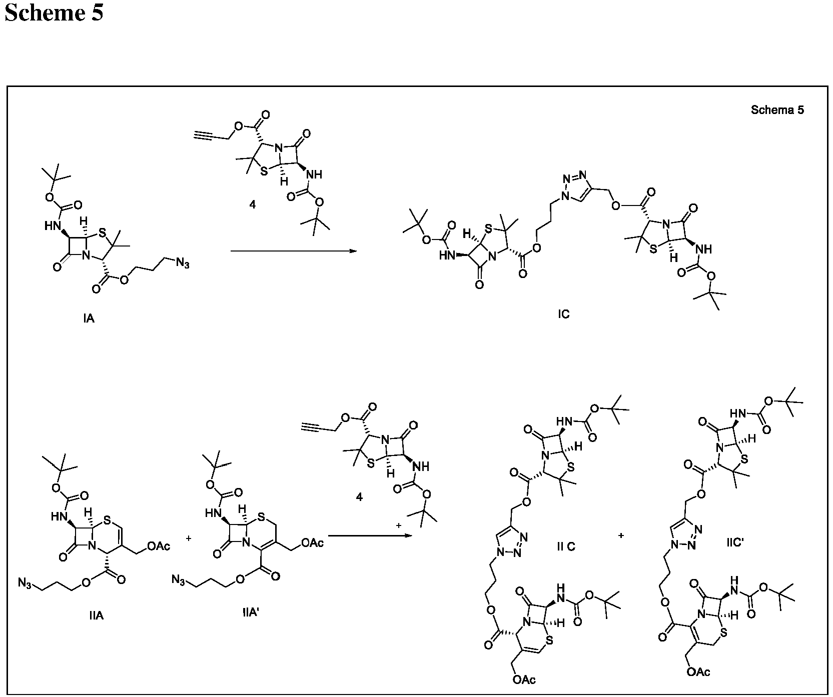

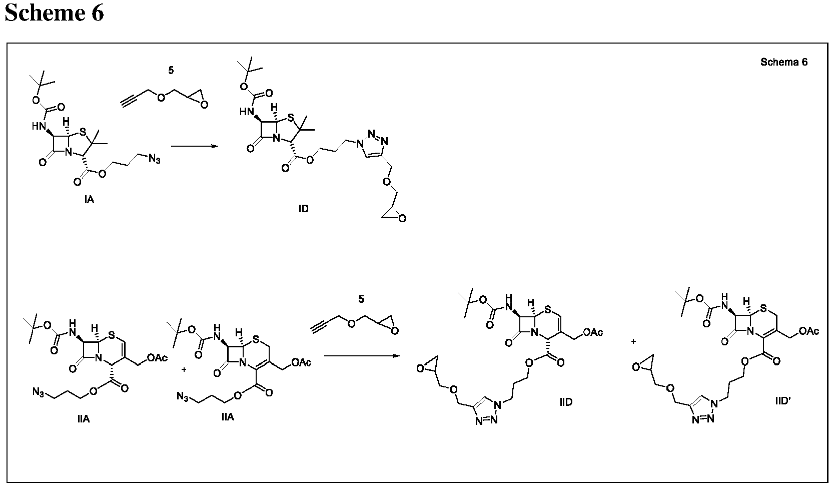

It should be noted that the cycloaddition reactions of IA, IIA and IIA' with the propargyl derivative of nucleus I (compound 4, scheme 4) demonstrate the need to synthesize the azide ester of I and II. In order to obtain a simple triazole functionalized with two beta-lactam rings, it is, in fact, also necessary to avail of an ester or azide amide.

Subsequent oxidation of the beta-lactam derivatives IB, IIB and IIB' in order to introduce an aldehyde group for producing the derivatives IF, IIF and IIF' (Scheme 7).

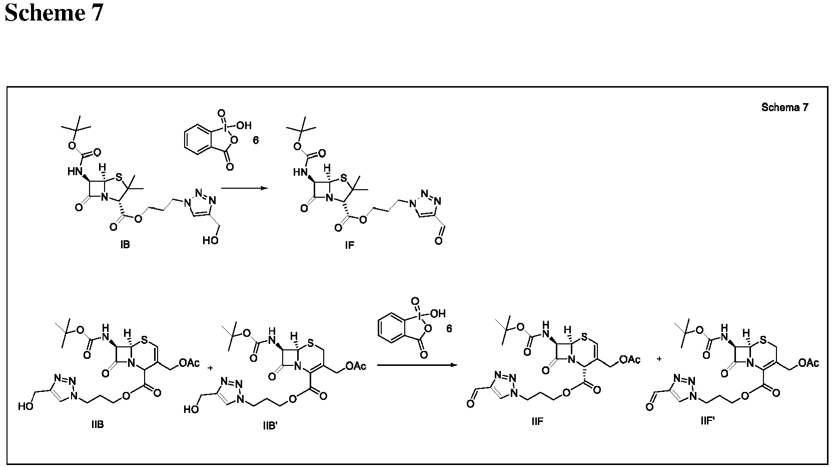

The aldehyde group, in fact, is important both for its therapeutic value and for its chemical reactivity. It is found in numerous compounds having a pharmacological activity including some molecules with an antimicrobial activity. Their antimicrobial activity appears to be linked to their capacity of interacting with the cytoplasmic membrane (K.W. Hunter et al.; "Preparation of microparticulate β-glucan from Saccharomyces cerevisiae for use in immune potentiation" Letters in Applied Microbiology 2002, 35, 267-271).

Furthermore, the aldehyde reacts with amino groups to form Schiff bases. These bases have been extensively studied for their versatility for different applications (Wail Al Zoubi, "Biological Activities of Schiff Bases and Their Complexes: A Review of Recent Works International Journal of Organic Chemistry" 2013, 3, 73-9*).*

As far as the esterification conditions are concerned (Point 2), efforts have been made to increase the reaction yield, in particular as regards nucleus II.

The conditions used in WO2017/158392 A1 do not in fact allow the propargyl derivative of the 7-aminocephalosporanic nucleus to be obtained in significant quantities.

In the synthesis process according to the present invention, the use of a nucleophile other than propargyl alcohol, together with the use of a reduced quantity of anhydrous dioxane and above all 3A molecular sieves, allowed the azide derivative of the nucleus 7-ACA (II) to be isolated with much higher yields and similar to those of the nucleus 6-APA (I). For this purpose, the use of 3A molecular sieves and 0.25 ml of anhydrous 1,4-dioxane per mmole of nuclei I and II not only allowed the volume of diethyl ether to be reduced from 15 ml to 10 ml, but also increased the reaction yield up to 30-40% also in the case of the 7-aminocephalosporanic nucleus.

With respect to the preparation of the ester derivative of nucleus I containing two azide groups (IJ scheme 3), the same esterification methodology indicated above allowed the desired product to be obtained with much lower reaction rates ranging from 5-10%. This esterification yield is justified by the greater steric hindrance exerted by the two azide groups of the propanol azide. It is incredibly important to observe how to date there are no beta-lactam branched antibiotics containing functionalizations that can directly produce triazole groups.

For this reason and in order to solve the problem of a greater stability of beta-lactam derivatives (the ester groups are more labile than the amide groups), secondary amide derivatives containing an azide group (IL, IIL and IIL') and tertiary amides containing two azide groups (IN, IIN and IIN') (or not according to the invention two propargyl groups (IG, IIG and IIG')) were synthesized, as shown in Scheme 8.

Now, although the reaction conditions described above proved to be effective or sufficient, these conditions did not allow the amide derivatives described hereunder to be produced, for obtaining the ester derivatives containing one or two azide groups.

In this respect, the secondary and tertiary amides of nuclei I and II, protected as terbutylcarbamates (Scheme 8), were obtained by modifying the amidation conditions reported in a work published in 2016 (A. Leggio et al., «One-pot synthesis of amides from carboxylic acids activated using thionyl chloride »RSC Adv., 2016, 6, 34468*).*

This method provides for the addition of thionyl chloride, at room temperature and in an equimolar ratio with the acid and nucleophilic amine, to a reaction mixture containing carboxylic acid and the desired amine in an equimolar ratio, in the presence of three equivalents of triethylamine.

The reaction yield in the case of the products IL, IIL and IIL', however, ranged from 5-10% to be reduced to 3-5% in the case of IN, IIN and IIN', (IG, IIG and IIG' not according to the invention). It should be noted, however, that even under these reaction conditions, two isomers are formed in the case of the cephalosporanic nucleus 7-ACA due to the slippage of the double bond. The use of DCM as solvent, however, involves an inversion of the percentage of the two isomers of nucleus II. In particular, IIG is obtained at 70%, whereas IIG' is obtained at 30% (NMR analysis).

In the case of IIN and IIN', however, the difficult purification made it possible to isolate a 90% mixture of IIN' and 10% of IIN for a total yield of 5%. COZY, carbon and HSQC analyses certify the success of the amidation reaction.

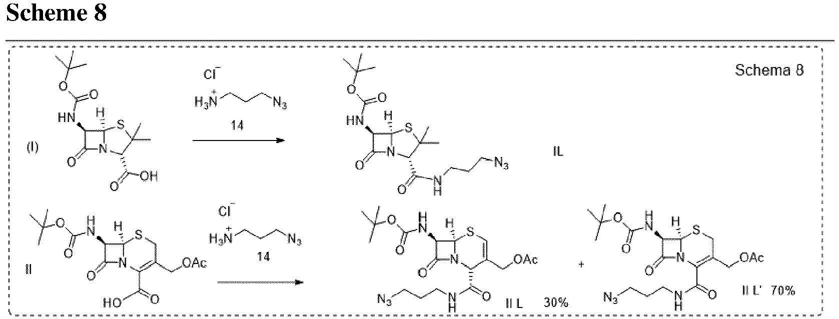

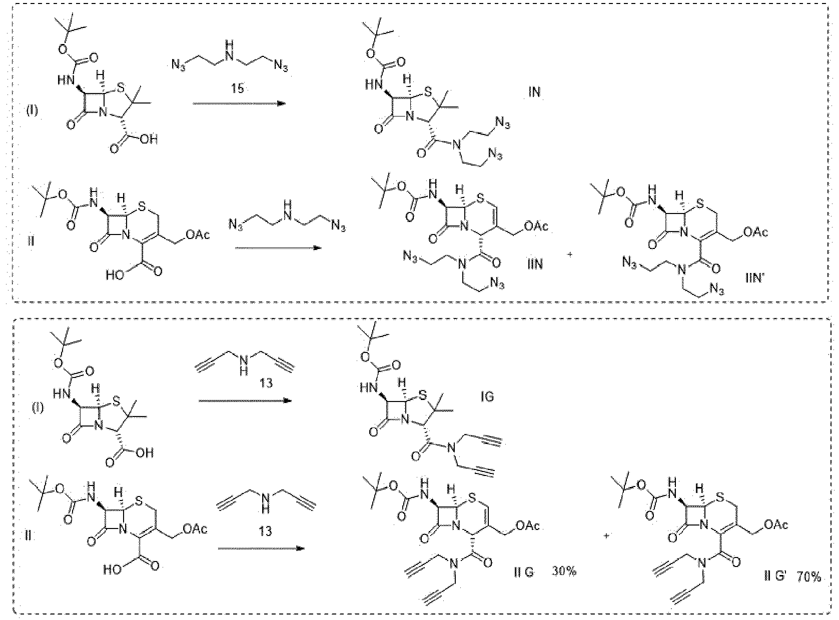

A modification of these reaction conditions allowed a yield of 23% to be obtained in the case of IL and 15% in the case of the mixture IIL and IIL', whereas a yield of 24% was obtained for IN and 5% for IIN', from 7 to 10% in the case of the IIG-IIG' mixture and 20% for IG. This modification consists in carrying out the reaction at 0°C, reversing the reaction order. Specifically, a solution of anhydrous DCM containing one millimole of carboxylic acid and one millimole of the primary or secondary amine in the presence of excess triethylamine or DIPEA (from3 to 5 equivalents) is added dropwise to a solution of one millimole of thionyl chloride in anhydrous DCM. The reaction is left under stirring and under anhydrous conditions for a time ranging from 1 hour to 24 hours.

With respect, on the other hand, to the synthesis of IL and IIL and IIL' derivatives, the reagent 14 was adopted, prepared using a synthesis sequence reported in literature (Huan Zhang et al., "Regioselective Rapid Synthesis of Fully-substituted 1,2,3-TriazolesMediated y Propargyl Cations »Org. Lett 2013, 15, 20, 5222-5225) for other purposes. The reagent 15 was obtained through a simple nucleophilic substitution with sodium azide, starting from the commercial reagent bis (2-chloroethyl) ammonium chloride.

The derivatives (IG not according to the invention), IL and IN were tested in the cycloaddition reaction with 3-azido-1-propanol and propargyl alcohol respectively to obtain the products IH (not according to the invention), IM and IP with yields within the range of 30-60% (Scheme 9). With respect to IP, the product obtained at 30% is difficult to purify due to the presence of impurities. The peaks relating to the triazole proton, however, integrate perfectly with the protons of nucleus I, confirming the success of the cycloaddition. Furthermore, COSY, carbon and HSQC analyzes confirm the structure of the derivative containing the two triazole groups and the intact structure of nucleus I.

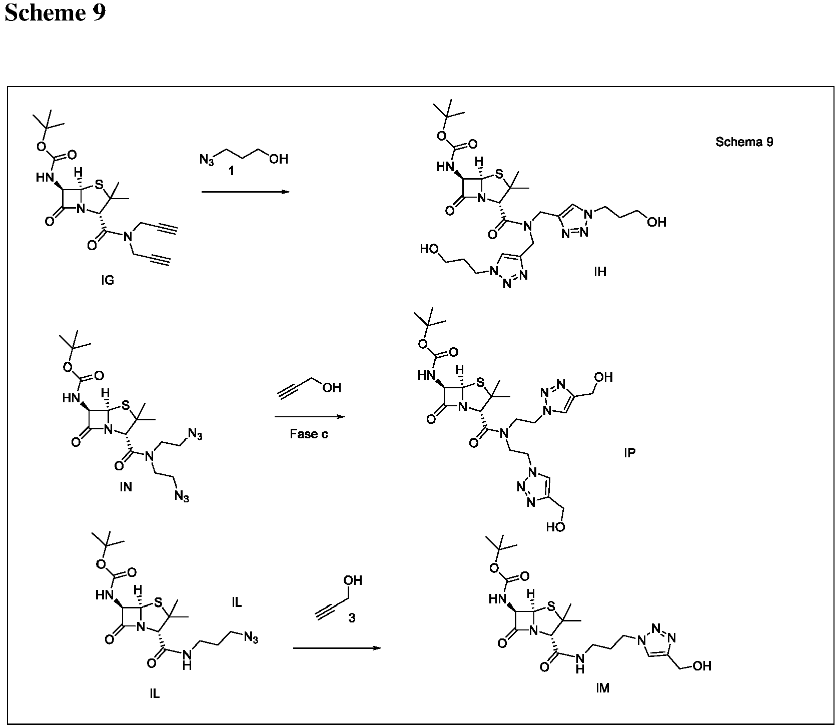

All the new products were characterized by NMR and mass spectroscopy using ultra highpressure liquid chromatography (UPLC 1290 Infinity) coupled with high resolution mass-spectrometry (HR-MS Q-TOF UHD 6538) of Agilent Technologies, with the exception of some products (IJ, IN, IIN' and IP) where mass spectroscopy was not effected due to instrumentation problems.

In conclusion, new beta-lactam derivatives were prepared with the synthesis process according to the present invention:
1. transforming nuclei I and II
   - into esters containing one or more azide groups by means of an azide alcohol containing one (IA, IIA, IIA') or two azide groups (IJ,) and
   - into secondary amides containing an azide group (IL, IIL, IIL'),
   - into tertiary amides containing two azide groups (IN, IIN)
      so as to introduce azide groups useful for both the cycloaddition reaction with alkynes and also for possible and different modifications of the azide group which do not provide for the formation of triazole;
2. introducing an epoxy group indirectly by means of the cycloaddition reaction of compounds IA and IIA and IIA' with epoxy propargylether (5) (Scheme 6);
3. introducing an aldehyde group into the nuclei I and II by means of the oxidation of compounds IB and IIB (Scheme 7);
4. preparing tertiary amides of the beta-lactam nuclei containing a double propargyl (scheme 9, compounds IG, IIG and IIG' not according to the invention); as previously pointed out, these derivatives allow branched structures of beta-lactam antibiotics, not described in the state of the art, containing a double triazole functionality, to be prepared;
5. cycloaddition catalyzed by the copper of a beta-lactam derivative with a double propargyl, compound IG not according to the invention, and a double azide, compound IN, to obtain a double functionalization with two triazole groups (respectively compound IH, not according to the invention, and compound IP) and a beta-lactam derivative containing an azide amide (IL) to obtain a beta-lactam amide functionalized with a single triazole IM, as shown in scheme 9.

The present invention therefore provides a method for modifying the beta-lactam nuclei 6-APA (I) and 7-ACA (II), protected as tertbutyl carbonates on the amino group, in the carboxyl position with the objective of making the preparation accessible of new molecules or bio-adducts containing, specifically, the beta-lactam nucleus intact, but functionalized with molecules having a different pharmacological activity with the aim of both enhancing and modulating their activity towards bacteria, mycobacteria, fungi and parasites, and also increasing their resistance towards bacterial defense systems.

In particular, the present invention provides a method for the functionalization at the level of the carboxyl group of the above-mentioned beta-lactam nuclei, with at least one reactive group different from the propargyl group previously indicated, thus introducing a group that allows a great versatility in the subsequent reactivity of the precursor.

The present invention therefore relates to a process for the synthesis of derivatives of β-lactam compounds selected from derivatives of 6-aminopenicillanic acid (6-APA) and 7-aminocephalosporanic acid (7-ACA) which comprises the following steps:
a) protection of the amino group in position 6 of 6-APA or in position 7 of 7-ACA via the formation of a carbamate;
b) esterification of the carboxyl group of the β-lactam compound obtained in step a) by reaction with an aliphatic alcohol, with a number of carbon atoms ranging from 3 to 10, preferably from 3 to 5, more preferably equal to 3, and containing at least one azide group or amidation of the carboxyl group of the β-lactam compound obtained in step a) by reaction with a primary aliphatic amine with a number of carbon atoms ranging from 3 to 10, preferably from 3 to 5, more preferably equal to 3, and containing an azide group or with a secondary aliphatic amine with a number of carbon atoms ranging from 4 to 10, preferably 4, and containing two azide groups to obtain a tertiary amide.

The general scheme of the process is indicated, for greater clarity, in schemes 2, 2b and 8 previously provided.

The present invention also relates to a process for the synthesis of triazole derivatives of β-lactam compounds wherein the precursor obtained at the end of step b) is subjected to the following step c):
c) cycloaddition reaction between the derivative obtained in step b) having at least one azide group and a compound having at least one alkyne group (C=C) with the formation of the corresponding triazole derivative of the β-lactam compound.

The present invention also relate to a process for the synthesis of triazole derivatives of β-lactam compounds wherein the precursor obtained at the end of step c) and containing a primary alcohol group is subjected to an oxidation reaction to obtain an aldehyde derivative.

The process according to the present invention allows the carboxylic group of the β-lactam nuclei to be esterified under mild conditions which do not cause the breakage of the β-lactam ring. In particular, the process according to the present invention is characterized by specific reaction conditions which have allowed the azide derivative of the nucleus 7-ACA (II) to be isolated in quantities identical to those of the nucleus 6-APA (1).

For this purpose, the use of 0.25 ml of anhydrous 1,4-dioxane per mmole of nucleus I and II and molecular sieves, allowed not only the volume of diethyl ether to be reduced from 15 ml to 10 ml, but also increased the reaction yield up to 40% even in the case of the 7-aminocephalosporanic nucleus. Furthermore, the reaction is carried out at a temperature ranging from -5°C to 0°C until the nucleophile is added. The mixture is then left at room temperature for a time ranging from 24 to 52 hours, preferably from 24 to 36 hours.

With respect to the amidation process, on the other hand, the reaction conditions are the following. The solvent used is anhydrous dichloromethane (DCM). In particular, a solution is prepared of thionyl chloride in DCM at 0°C, to which a mixture in anhydrous DCM is added dropwise, containing carboxylic acid and the primary or secondary amine in equimolar quantities with the thionyl chloride, in the presence of an excess of triethylamine (TEA) or diisopropylethylamine (DIPEA) (3-5 equivalents).

Step a) of the process according to the present invention which provides for the protection of the amino group in position 6 of APA or in position 7 of ACA by the formation of a carbamate can be carried out by reaction with a dicarbonate, which can be an aromatic or cyclic alkyl dicarbonate. These dicarbonates can be optionally functionalized with groups which exert their biological activity or which then allow further derivatization in this position 6 or 7, possibly to confer a different pharmacological activity.

Examples of possible dicarbonates are the following: di-tert-butyl dicarbonate, bis (oxiran-2-ylmethyl) dicarbonate, bis (trifluoromethyl) dicarbonate, bis (2-ethoxycyclohexyl) dicarbonate or dibenzyl dicarbonate:

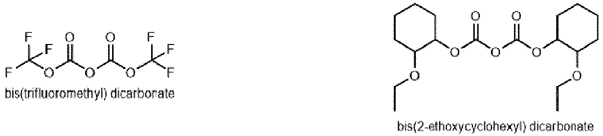

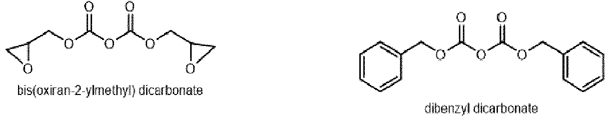

Step a) is preferably carried out by forming the carbamate with di-tert-butyl dicarbonate. More preferably, step a) of the process according to the present invention is carried out at room temperature (20-30°C), for a time ranging from 10 to 32 hours, preferably for a time equal to about 24 hours in a solvent which is a 1:1 mixture by volume of 1,4-dioxane and water or a 1:1 mixture by volume of THF and water.

The (tert-butyl-dicarbonate) reagent is present in a molar ratio of 2 to 1, preferably from 1.3 to 1, with respect to the β-lactam compound.

As far as the esterification process is concerned, step b) is carried out under anhydrous conditions using anhydrous diethyl ether in mixture with anhydrous 1,4-dioxane or anhydrous THF in ratios by volume of 10/0.25 in the presence of an amine selected from triethylamine (anhydrified on molecular sieves), diethylpropylamine (DIPEA), anhydrous pyridine and dimethylaminopyridine.

The carboxylic acid and alcohol are in an equimolar ratio, whereas the base used is present in a ratio of 2 to 1 with respect to the beta-lactam nucleus.

Step b) of the reaction is carried out at a temperature ranging from -5°C to 0°C until the nucleophile is added, and is subsequently left to react at room temperature for a time ranging from 24 to 52 hours, preferably from 24 to 36 hours.

The esterification of nuclei I and II with an alcohol having an azide group, in particular 3-azidopropan-1-ol (1) or with an alcohol with two azide groups, in particular 1,3-azidopropanol (2 ), allowed compounds IA, a mixture of IIA and IIA' in a ratio of 85/15, IJ and a mixture of IIJ and IIJ' in a ratio of 85/15 (Scheme 2 and Scheme 2b indicated above) to be obtained.

These compounds, which have the appearance of dense whitish oils, were isolated and characterized by NMR (1-H, 13C, COSY, HSQC, HMBC) and Mass Spectroscopy.

The esters containing an azide group were produced with a yield ranging from 10 to 40%. The highest yields coincide with anhydrous conditions for both the solvent and for the products. The water present even in small quantities drastically reduces the reaction yield and in fact, using non-anhydrous solvents, the desired products are not formed. With respect to the cephalosporanic nucleus 7-ACA (IIA), with the esterification process there is the formation of a mixture of the two isomers IIA (85) and IIA' (15%) according to the above scheme 2.

The 65% percentage of IIA and 35% of IIA' that is found in NMR is the consequence of purification on a chromatographic column.

With respect to the esters with two azide groups, the product IJ was obtained with a yield of about 10%.

The derivative esterified with an alcohol that brings one or two azide groups into the carboxyl position of the β-lactam nucleus, obtained at the end of step b), allows, for example, a *"copper(I)-catalyzed azide-alkyne cycloaddition (CuAAC) reaction"* to be carried out with the consequent introduction of different, appropriate and selected structures with a triazole ring.

In particular, in order to create a library of beta-lactam derivatives containing at least one triazole group, the compounds IA, IIA and IIA' were tested in the cycloaddition reaction with two different substrates to obtain new derivatives, indicated hereunder and characterized.

In particular, the reaction of IA, IIA and IIA' with propargyl alcohol 3 (scheme 3 indicated above) allowed compound IB and the mixture of isomers IIB and IIB' to be produced. This reaction allowed a hydroxyl group to be introduced not only with the aim of modulating the lipophilicity of the molecule, but as a possible starting point for further modifications.

In the same way, the reactions of IA and IIA with the propargyl ester of 6-APA (compound 4) allowed the product IC and the mixture of isomers IIC and IIC' to be obtained (scheme 4 indicated above). In this way, it was possible to obtain a functionalized triazole with two nuclei of 6-APA or one nucleus of 6 APA and one of 7-ACA.

These compounds are certainly interesting for studying the effect of a double beta-lactam, containing a triazole group, both in the interaction with bacterial defense mechanisms (beta-lactamase, efflux pumps etc.), and also with the enzymes responsible for the production of peptidoglycan such as transpeptidase.

It should be noted that these molecules could only exist through the preparation of the azide ester of the nuclei 6-APA and 7-ACA according to the present invention (IA and IIA and IIA').

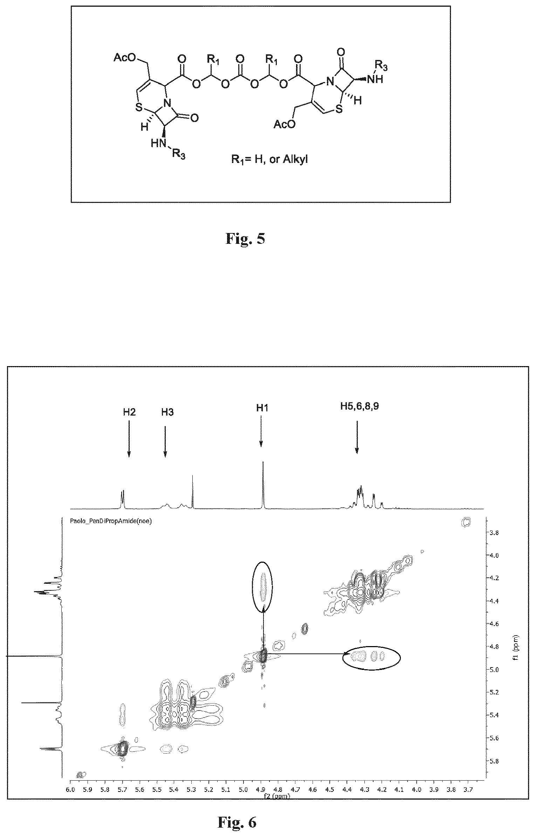

With respect to the presence of di-penicillin and di-cephalosporin esters, there are some patents, in which the two nuclei are bound through carbonate groups (Figure 5, US3928595).

The cycloaddition reaction catalyzed by copper between the esters IA, IIA and IIA' with propargyl ether of glycidol (compound 5) also allows the preparation of beta-lactam derivatives containing an epoxy group (Scheme 6 indicated above). The introduction of an epoxy group is important for various reasons.

Step b) also consists in the synthesis of tertiary amide derivatives IN, IIN and IIN' containing a double azide group and, (not according to the invention, of tertiary amides IG and IIG and IIG' containing a double propargyl group), and of secondary amide derivatives IL, IIL and IIL' containing an azide group.

With respect to the amidation reaction of the cephalosporanic nucleus with compound 14 (3-azidopropylammonium chloride, scheme 8), on the other hand, the product obtained, a secondary amide having an azide group, is once again a mixture of the two isomers IIL and IIL' with the second product present at 70%.

Purification by chromatography on silica in direct phase, allowed the product IIL' to be purified, with a yield of about 10%, in a practically pure form. Step c) of the synthesis process of triazole derivatives according to the present invention is a cycloaddition reaction, more specifically it is a 1,3 dipolar cycloaddition, wherein the organic azides (esters and secondary and tertiary amides of nuclei I and II) obtained in step b) as described above, are reacted with a compound having a terminal alkynyl group (-C=CH), preferably having a propargyl group, with the formation of the corresponding triazole derivative of the β-lactam compound. In the same way, the tertiary amides of nuclei I and II, containing a double propargyl group, again obtained in step b), are reacted with an organic azide, in particular with 3-azidopropanol (compound 1) to obtain the corresponding triazole derivatives. The cycloaddition reaction is mediated by copper in oxidation state I.

Step c) is carried out in a mixture of water and at least one C₁-C₄ aliphatic alcohol, preferably in water and isopropanol (IPA) 1 : 1 by volume, at a temperature ranging from 55 to 60°C, preferably from 58 at 60°C, for a time ranging from 4 to 10 hours, preferably from 4.5 to 6 hours.

The process according to the present invention is also of particular interest for the preparation of compounds having an aldehyde group.

The aldehyde group is found in numerous compounds having a pharmacological activity including some molecules with an antimicrobial activity. The antimicrobial activity appears to be linked to the capacity of interacting with the cytoplasm membrane. Furthermore, the aldehyde reacts with amino groups to form Schiff bases. These bases have been extensively studied for their versatility in different Wail Al Zoubi applications, *"*Biological Activities of Schiff Bases and Their Complexes: A Review of Recent Works" International Journal of Organic Chemistry "2013, 3, 73-95.

In order to introduce the aldehyde group, for example, compounds IB and IIB were oxidized using 2-iodoxybenzoic acid (IBX) applying the conditions reported in literature: Jesse D. More et al.; "A Simple and Advantageous Protocol for the Oxidation of Alcohols with O-Iodossibenzoic Acid (IBX)" Org. Lett., Vol. 4, No. 17, 2002.

Compounds IF and IIF, which have the appearance of transparent oils having an intense fruity fragrance, were obtained and characterized by NMR and mass and are presented in scheme 8 above. In particular, the oxidizing agent is difficult to remove by a direct phase chromatography column, especially in the case of compound IF. The formation of the aldehyde group, however, is confirmed by the appearance of a peak around 10 ppm in 1-H NMR analysis and a peak at 185 ppm in 13-C analysis. In addition, the disappearance of the methylene group (CH₂) carrying the alcohol group, and the presence of peaks typical of beta-lactam structures intact, confirm the completed oxidation and non-degradation of the beta-lactam rings.

The oxidation of these substrates allows a 1,2,3-triazo-4-carbaldehyde to be generated. A similar structure is present in different molecules with an antibacterial and antimycobacterial activity. S. Sharma et al.; "Synthesis of 2,3,6-trideoxy sugar triazole hybrids as potential new broad spectrum antimicrobial agents" European Journal of Medicinal Chemistry, 2014, vol. 83, pages 474 - 489, P. Suman et al. "Synthesis and antimycobacterial activity of 2-chloronicotinaldehydes based novel 1H-1,2,3-triazolylbenzohydrazides" Bioorganic and Medicinal Chemistry Letters, 2015, vol. 25, # 11, pages 2390 - 2394.

The present invention therefore also relates to amide derivatives of nuclei I and II (Scheme 8). In particular, secondary amides were produced containing an azide group and tertiary amides containing a double azide group or a double propargyl group (Scheme 9). An essential reason for preparing these amide derivatives is linked to the importance of the amide group from both a chemical point of view and also from the point of view of biological activity.

The amide group is undoubtedly more resistant than the ester group, especially towards the hydrolytic action exerted by the esterases present in the human body.

Furthermore, amide groups are found in numerous compounds having a pharmaceutical activity, in natural products and in polymers (RM Lanigan et al., "Direct amidation of unprotected amino acids using B (OCH2CF3) 3" Chem. Commun. 2016, 52, 8846-8849). Biomolecules containing amide groups are of great interest in the pharmaceutical and biotechnological fields (A. Goswami et al., "Enzymatic Strategies and Biocatalysts for Ammide Bond Formation: Tricks of the Trade Outside of the Ribosome" Mol Biosyst. 2015, 20; 11 (2 ), 338-353).

There are some examples in literature of beta-lactam derivatives in which the carboxylic group has been converted into amide, but no derivative is effective for the preparation of triazole derivatives. An amide of benzyl penicillin containing a propargyl group was recently produced by means of a copper-catalyzed reaction described in literature (S. V. Ley et al., "Rapid Asymmetric Synthesis of Disubstituted Allenes by Coupling of Flow-Generated Diazo Compounds and Propargylated Amines", Angew. Chem.Int.Ed, 2017, vol.56, (7), 1864-1868). Furthermore, an amide containing a single propargyl group on a cephalosporin was described in May 2016. (B. Gold et al.; "Novel Cephalosporins Selectively Active on Nonreplicating Mycobacterium tuberculosis" J. Med. Chem. 59, 13, 6027 -6044).

Beta-lactam derivatives of the nuclei 6-APA and 7-ACA in which the carboxyl group has been converted into a secondary amide containing an azide group, or a tertiary amide containing two azide groups or two propargyl groups, however, are not described in literature. The advantage of having a double azide or a double propargyl with respect to a single azide or propargyl group is extraordinarily important, as it allows branched and much more complex structures to be obtained. As already mentioned with respect to the product IJ.

In the case of secondary amide derivatives containing an azide group, the same argument applies, already reported for the azide esters mentioned above. The azide group, in fact, not only allows cycloaddition reactions, but also offers the advantage of being a substrate for other synthetic modifications such as the reduction of the azide group to amine, (S. Liu et al.; «Staudinger Reactions for Selective Functionalization of Polysaccharides : A Review »Biomacromolecules, 2015, 16 (9), pages 2556-2571) or the Villarasa reaction to form a new peptide bond. (J. Vilarrasa et al., «Catalytic Staudinger-Vilarrasa Reaction for the Direct Ligation of Carboxylic Acids and Azides ". J. Org. Chem., 2009, 74, 2203-2206*).*

For this reason, using a modification of a procedure known in literature based on the use of thionyl chloride (A. Leggio et al., "One-pot synthesis of ammonium from carboxylic acids activated using thionyl chloride" RSC Adv., 2016.6, 34468-34475), the nuclei 6-APA and 7-ACA, protected as terbutylcarbamates on the amino group, were subjected to amidation reaction using dipropargylamine to form tertiary amides (Scheme 11).

In particular, IG was obtained with a yield of 20%, IIG and IIG', as a mixture of isomers, with a yield of 7-10%.

With respect to the secondary amides IL and the mixture of isomers IIL and IIL', the same procedure was used with a yield of 23% in the case of IL and with a yield of 15% in the case of the mixture of the two isomers IIL and IIL'. To produce these compounds, nuclei I and II were reacted with compound 14 which was prepared as already indicated, following the synthesis procedure known in literature.

The amidation reaction was carried out as follows: a solution containing one millimole of acid, one millimole of compound 14 and a excess of TEA or DIPEA (5 equivalents) was added dropwise to a solution of one millimole of thionyl chloride in 3 ml of anhydrous DCM at 0°C. The reaction is then left under stirring for a period ranging from 30 minutes to three hours. The isolation of the products is effected by purification through a direct phase chromatographic column on silica.

NMR and mass spectroscopy analyses confirm the desired structure for both compounds. In particular NOESY analysis shows that even in the compound IG, the proton H1 does not "see" the protons H2 and H3. This analysis shows that the H1 proton is on the plane of the molecule, whereas the carboxyl group is below the plane (Figure 6).

The tertiary amide products containing the double azide group (or the double propargyl group not according to the invention), and the secondary amide derivatives containing an azide group, can be subsequently functionalized to obtain new beta-lactam derivatives by means of the cycloaddition reaction catalyzed by copper I (Scheme 9).

The product IH not according to the invention, for example, was obtained with a yield of 45%, by reacting IG with two equivalents of 3-azidopropanol (compound 1). This product has a double functionalization of the penicillanic nucleus via two triazole groups.

Viceversa, the product IM was obtained, again by means of the cycloaddition reaction catalyzed by copper I, by reacting the substrate IL with propargyl alcohol (compound 3), with a yield of 60%. The compound IP obtained with a yield of 30% was prepared in the same way.

Compounds IH (not according to the invention), IM and IP, which appear as white foamy solids, were characterized by means of NMR and mass spectroscopy analysis (except for IP) (Scheme 9).

A further object of the present invention relates to triazole derivatives having the following names and the following structural formulae:
3-azidopropyl(2S, 5R, 6R)-6-((tert-butoxycarbonyl)-amino)-3,3-dimethyl-7-oxo-4-thio-1-azabicyclo[3.2.0]heptane-2- carboxylate **IA** 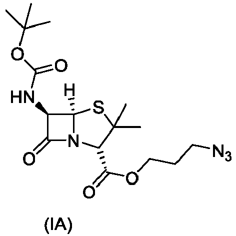
3-azidopropyl-(2R, 6R, 7R)-3-(acetoxymethyl)-7-((tert-butoxy-carbonyl)-amino)-8-oxo-5-thio-1-azabicyclo [4.2.0] oct-3-ene-2-carboxylate **IIA** 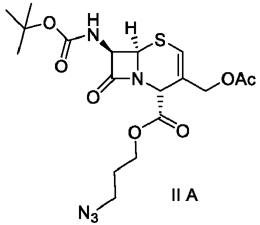
3-azidopropyl-(6R,7R)-3-(acetoxymethyl)-7-((tert-butoxy-carbonyl)-amino)-8-oxo-5-thio-1-azabicyclo[4.2.0]oct-2-ene-2-carboxylate **IIA**' 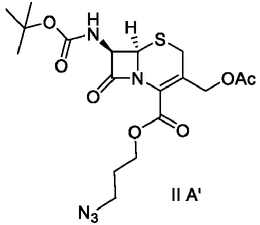
3-(4-(hydroxymethyl)-1H-1,2,3-triazol-1-yl)-propyl(2S,SR,6R)-6-((tertbutoxycarbonyl)amino)-3,3-dimethyl-7-oxo-4-thio-1-azabicyclo[3.2.0]heptan-2-carboxylate **IB** 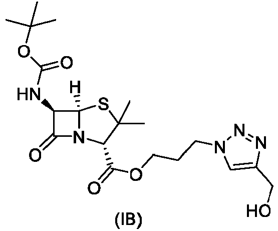
3-(4-(hydroxymethyl)-1H-1,2,3-triazol-1-yl)-propyl-(2R,6R, 7R)-3-(acetoxymethyl)-7-((tert-butoxycarbonyl)-amino)-8-oxo-5-thio-1-azabicyclo[4.2.0]oct-3-ene-2-carboxylate **IIB** 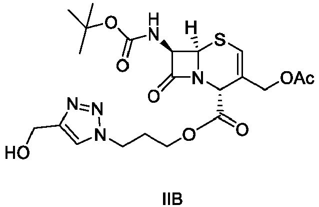
3-(4-(hydroxymethyl)-1H-1,2,3-triazol-1-yl)-propyl-(6R,7R)-3-(acetoxymethyl)-7-((tert-butoxycarbonyl)-amino)-8-oxo-5-thio-1-azabicyclo[4.2.0]oct-3-ene-2-carboxylate **IIB**' 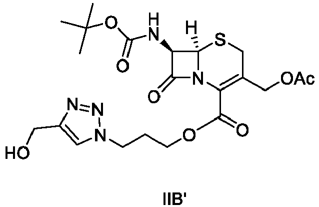
3-(4-((((2S,SR,6R)-6-((tert-butoxycarbonyl)-amino)-3,3-di-methyl-7-oxo-4-thio-1-azabicyclo[3.2.0]heptane-2-carbonyl) oxy)-methyl)-1H-1,2,3-triazol-1-yl)-propyl-(2S,5R,6R)-6- ((tert-butoxycarbonyl)-amino)-3,3-dimethyl-7-oxo-4-thio-1-azabicyclo [3.2.0]heptane-2-carboxylate **IC** 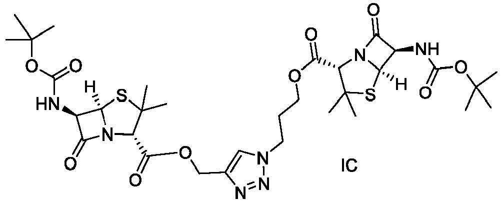
3-(4-((((2S,SR,6R)-6-((tert-butoxycarbonyl)-amino)-3,3-dimethyl-7-oxo-4-thio-1-azabicyclo[3.2.0]heptane-2-carbonyl) oxy)-methyl)-1H-1,2,3-triazol-1-yl)-propyl(2R,6R,7R)-3-(acetoxymethyl)-7-((tert-butoxycarbonyl)-amino)-8-oxo-5-thio-1-azabicyclo[4.2.0]oct-3-ene-2-carboxylate **IIC** 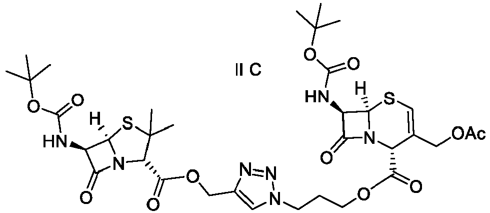
3-(4-((((2S,SR,6R)-6-((tert-butoxycarbonyl)-amino)-3,3-dimethyl-7-oxo-4-thio-1-azabicyclo[3.2.0]heptane-2-carbonyl) oxy)-methyl)-1H-1,2,3-triazol-1-yl)-propyl-(6R,7R)-3-(acetoxymethyl)-7-((tert-butoxycarbonyl)-amino)-8-oxo-5-thio-1-azabicyclo[4.2.0]oct-2-ene-2-carboxylate **IIC**' 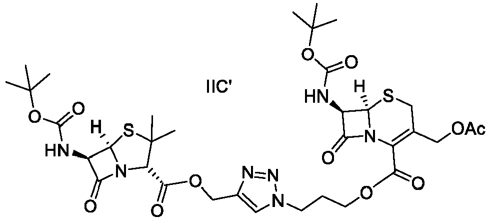
3-(4-((oxiran-2-yl-methoxy)-methyl)-1H-1,2,3-triazol-1-yl)-propyl-(2S,SR,6R)-6-((tertbutoxycarbonyl)-amino)-3,3-dimethyl-7-oxo-4-thio-1-azabicyclo[3.2.0]heptane-2-carboxylate **ID** 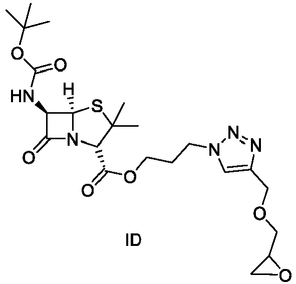
3-(4-((oxiran-2-ylmethoxy)-methyl)-1H-1,2,3-triazol-1-yl)-propyl-(2R,6R,7R)-3-(acetoxymethyl)-7-((tert-butoxycarbonyl)-amino)-8-oxo-5-thio-1-azabicyclo [4.2.0]oct-3-ene-2-carboxylate **IID** 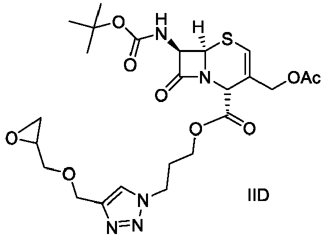
3-(4-((oxiran-2-ylmethoxy)-methyl)-1H-1,2,3-triazol-1-yl)-propyl-(6R,7R)-3-(acetoxymethyl)-7-((tert-butoxycarbonyl)-amino)-8-oxo-5-thio-1-azabicyclo[4.2.0]oct-2-ene-2-carboxylate **IID**' 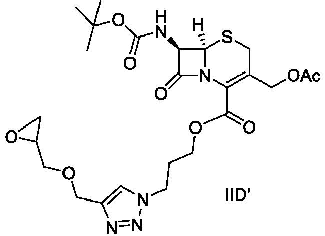
3-(4-formyl-1H-1,2,3-triazol-1-yl)-propyl-(2S,SR,6R)-6-((tert-butoxycarbonyl)-amino)-3,3-dimethyl-7-oxo-4-thio-1-azabicyclo [3.2.0]heptane-2-carboxylate **IF** 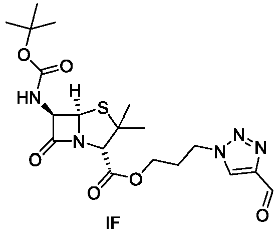
3-(4-formyl-1H-1,2,3-triazol-1-yl-propyl-(2R,6R,7R)-3- (acetoxymethyl)-7-((tert-butoxycarbonyl)-amino)-8-oxo-5-thio-1-azabicyclo[4.2.0]oct-3-ene-2-carboxylate **IIF** 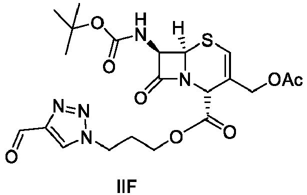
3-(4-formyl-1H-1,2,3-triazol-1-yl)-propyl-(6R,7R)-3- (acetoxymethyl)-7-((tert-butoxycarbonyl)amino)-8-oxo-5-thio-1-azabicyclo[4.2.0]oct-2-ene-2-carboxylate **IIF**' 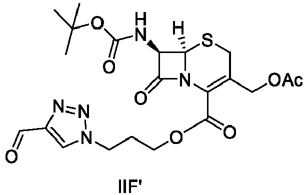
1,3-diazidopropan-2-yl (2S,5R,6R)-6-((tert-butoxycarbonyl)-amino)-3,3-dimethyl-7-oxo-4-thio-1-azabicyclo[3.2.0]-heptane-2-carboxylate **IJ** 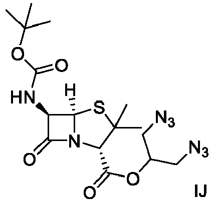
tert-butyl-((2S,SR,6R)-2-((3-azidopropyl)carbamoyl)-3,3-dimethyl-7-oxo-4-thio-1-azabicyclo[3.2.0]heptan-6-yl) carbamate **IL** 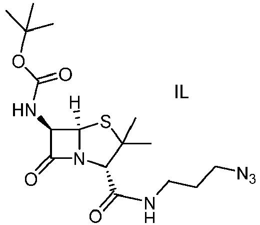
((2R,6R,7R)-2-((3-azidopropyl)carbamoyl)-7-((tert-butoxycarbonyl)amino)-8-oxo-5-thio-1-azabicyclo[4.2.0]oct-3-en-3-yl)methyl acetate **IIL** 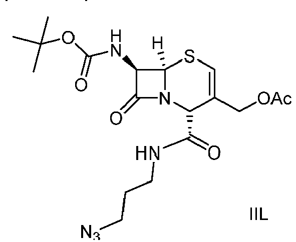
((6R,7R)-2-((3-azidopropyl)carbamoyl)-7-((tert-butoxy-carbonyl)amino)-8-oxo-5-thio-1-azabicyclo[4.2.0]oct-2-en-3-y)methyl acetate **IIL**' 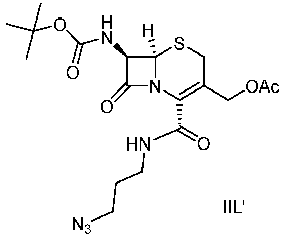
tert-butyl-((2S,SR,6R-2-((3-(4-(hydroxymethyl)-1H-1,2,3-triazol-1-yl)propyl)carbamoyl)-3,3-dimethyl-7-oxo-4-thio-1-azabicyclo[3.2.0]heptan-6-yl) carbamate **IM** 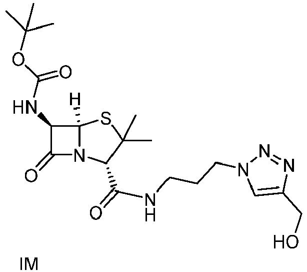
tert-butyl-((2S,SR,6R)-2-(bis(2-azidoethyl)carbamoyl)-3,3-dimethyl-7-oxo-4-thia-1-azabicyclo[3.2.0]heptan-6-yl) carbamate **IN** 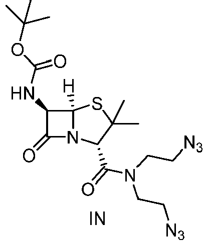
((6R,7R)-2-(bis(2-azidoethyl)carbamoyl)-7-((tert-butoxy-carbonyl)amino)-8-oxo-5-thia-1-azabicyclo[4.2.0]oct-2-en-3-yl)methyl acetate **IIN**' 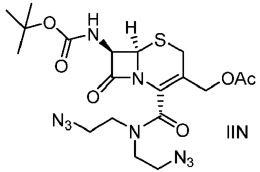
tert-butyl((2S,SR,6R)-2-(bis(2-(4-(hydroxymethyl)-1H-1,2,3-triazol-1-yl)ethyl)carbamoyl)-3,3-dimethyl-7-oxo-4-thia-1-azabicyclo[3.2.0]heptan-6-yl)carbamate **IP**

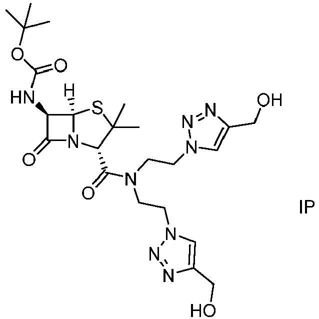

These intermediates or precursors can then be functionalized in very different ways by means of appropriate reactions.

In one embodiment, the present invention provides a compound among those listed above, or a mixture of said compounds, for use as a medicament or as a precursor of a medicament, preferably an antibiotic, antifungal and/or as an anticancer drug.

The following examples are representative of the present invention, without limiting its scope in any way.

Unless otherwise specified, chemical compounds and solvents were bought from Sigma Aldrich (St-Quentin Fallavier, France) and used without further purification. The abbreviations are as follows: anhydrous dichloromethane (DCM dry), ethyl acetate (EtOAc), di-tert-butyl dicarbonate (Boc), methanol (MeOH), N,N-dimethylformamide (DMF), anhydrous diethyl ether (Et₂O dry), 1,4 dioxane anhydrous (dioxane dry), tetrahydrofuran anhydrous (THF dry), isopropanol (IPA), triethylamine (TEA), diisopropylethylamine (DIPEA). Thin layer chromatography (TLC) was performed using aluminum sheets coated with silica gel 60 F254 purchased from Merck and verified by examination under UV light (254 and 366 nm). Nuclear magnetic resonance spectroscopy (NMR) 1 H, 13C, COSY, HSQC, NOESY and HMBC NMR: the analyses were recorded with a Bruker Avance 400 MHz using CDCl₃, CD₂Cl₂, and the chemical shifts (δ) were indicated in parts per million (ppm). The coupling constants (J) are in Hz, and the multiplicities are described as singlet (s), doublet (d), triplet (t), quadruplet (q), double doublet (dd), double triplet (dt), broad (br), multiplet (m), and double doublet of doublets (ddd). The molecular weights were determined using the electrospray ionization technique (ESI-MS) using an Agilent QTOF 6538 (Wilmington, DE), coupled with an Agilent 1200 with a high resolution liquid chromatography (LC) system. Mobile phase 100% ACN for HPLC analysis: the samples were injected into the DUAL ESI at a rate of 400 µL min and analyzed in the presence of standards. The capillary voltage is +3,800 V and -3,800V. The ion source was at a temperature of 300°C with a flow of 121/min of nitrogen.

### Example 1

### A) General procedure for the esterification of carboxylic acids with azidopropanol (1) and with 1,3-diazidopropan-2-ol (2).

1 mmole of carboxylic acid is dissolved in 0.5 mL of anhydrous 1,4-dioxane in a 25 mL flask in an atmosphere of N₂ and under stirring. 10 mL of anhydrous diethyl ether or anhydrous THF or anhydrous 1,4-dioxane (preferably diethyl ether) are added later.

1 mmole of diisopropylethylamine (DIPEA), or triethylamine (TEA) or anhydrous pyridine, is added to the solution which is left to react for 5 minutes at 0°C.

1 mmole of paratoluenesulfonyl chloride or mesyl chloride or diethyl phosphate chloride is subsequently added to the solution which is left under stirring for 30 minutes at 0°C.

A solution of anhydrous diethyl ether containing a millimole of azidopropanol (1) or a millimole of 1,3-diazidopropan-2-ol (2) and a millimole of DIPEA, TEA or anhydrous pyridine, is then added dropwise to the solution containing the carboxylic acid maintained at 0°C.

The reaction mixture is reacted for 24 hours and monitored via TLC using a mixture of Hexane/Acetone 7/3.

The solvent is evaporated using a rotavapor and the purification of the reaction mixture on silica, using Hexane/Acetone 9/1 as eluent, allows the desired product to be isolated with a yield ranging from 30 to 40%.

### B) General procedure for the cycloaddition reaction catalyzed by copper

One millimole of the azide ester and one millimole of the selected substrate containing a terminal alkyne are added to 3 mL of a 50% mixture of water and isopropyl alcohol at room temperature and under magnetic stirring.

10% by moles of sodium ascorbate and 2-5% by moles of copper (II) sulfate (% with respect to moles of azide substrate), are added to the solution.

The reaction is carried out at a temperature ranging from 20 to 70°C for 2-48 hours and monitored via TLC with a 7/3 hexane/acetone mixture.

Once the solvent has been removed by means of the rotavapor, the reaction raw product is purified on silica using a mixture of DCM/Acetone 8/2 or DCM/MeOH 8/2 for the more hydrophilic compounds, to obtain the desired product either as a transparent oil or as a white solid with a yield ranging from 30 to 60%.

### C) General procedure for the oxidation of a primary alcohol to aldehyde.

One millimole of alcohol and 3 equivalents of IBX (7) are mixed in 3 mL of acetonitrile at 80°C. The reaction is monitored via TLC using a 7/3 mixture of DCM/acetone.

The mixture is cooled to 0°C and filtered to remove part of the oxidizing reagent. Purification on a direct phase chromatographic column using silica and a mixture of hexane/acetone 6/4 does not allow the reaction by-products to be completely eliminated.

### D) Procedure for the synthesis of secondary and tertiary amide derivatives

A solution of 1 mmole of acid plus 1 mmole of 3-azidopropan-1-ammonium chloride (14) or 1 mmole of bis (2-azidoethyl) amines (15) and 5 mmoles of DIPEA or TEA dissolved in 5mL of anhydrous dichloromethane, is added dropwise to a solution of 1 millimole of thionyl chloride in 3 mL of anhydrous DCM and in the presence of molecular sieves at 0°C. The reaction is left under stirring for a time ranging from 40 minutes to 24 hours at room temperature and is monitored via TLC with Hexane 7/Acetone 3 as mobile phase. At the end of the reaction, the solvent is evaporated and the product is purified by means of chromatography to obtain the amide with a yield ranging from 7 to 23%.

The following compounds were prepared using the general procedures indicated above: 3-azidopropyl (2S,5R,6R)-6-((tert-butoxycarbonyl)-amino)-3,3-dimethyl-7-oxo-4-thio-1-azabicyclo[3.2.0]heptane-2-carboxylate IA
1 mmole of carboxylic acid is dissolved in 0.5 mL of anhydrous 1,4-dioxane in a 25 mL flask in an atmosphere of N₂ and under stirring. 10 mL of anhydrous diethyl ether are subsequently added; 1 mmole of diisopropylethylamine (DIPEA) is added to the solution which is left to react for 5 minutes at 0°C.
1 mmole of diethyl phosphate chloride is subsequently added to the solution which is left under stirring for 30 minutes at 0°C.

A solution of anhydrous diethyl ether containing one millimole of azidopropanol (1) and one millimole of DIPEA is then added dropwise to the solution containing the carboxylic acid maintained at 0°C.

The reaction mixture is reacted for 24 hours and monitored via TLC using a mixture of Hexane/Acetone 7/3.

The solvent is evaporated by means of the rotavapor and purification of the reaction mixture on silica, using Hexane/Acetone 9/1 as eluent, allows the desired product to be isolated with a yield of 40%.

The product; which has the appearance of a transparent oil, was characterized by NMR analysis and mass spectroscopy (Figure 7).
¹H NMR (CDCl₃, 400 MHz): δ 5.52 (d, *J* = 4.2 Hz, 1H, H2), 5.48 (dd, *J* = 9.3, 3.6 Hz, 1H, H3), 5.29 (d, *J* = 9.3 Hz, 1H, H4), 4.42 (s, 1H, H1), 4.29 (dt, *J* = 11.3, 6.2 Hz, 1H,H5), 4.21 (dt, *J* = 11.2, 6.2 Hz, 1H,H6), 3.42 (t, *J* = 6.6 Hz, 2H,H9-H10), 1.94 (m, 2H, H7-H8), 1.65 (s, 3H), 1.50 (s, 3H), 1.44 (s, 9H)
¹³C NMR (CDCl₃, 100 MHz): δ 27.26 (s, 1C, C3), 28.07 (s, 1C, C10), 28.33 (s,3C,14), 31.74 (s, 1C, C4), 48.10 (s, 1C, C11), 60.37 (s, 1C, C6), 62.69 (s, 1C, C9), 64.71 (s, 1C, C2), 68.54 (s, 1C, C5), 70.68 (s, 1C, C1), 81.15 (s, 1C, C13), 154.25 (s, 1C, C12), 167.94 (s, 1C, C8), 174.90 (s, 1C, C7)
MS (ESI): *m*/*z*; found [M+Na]⁺ = 422,1488, theoretical [M+Na]⁺ = 422,1469 3-azidopropy(6R,7R)-3-(acetoxymethyl)-7-((tert-butoxycarbo-nyl)-amino)-8-oxo-5-thio-1-azabicyclo[4.2.0]oct-3-ene-2-carboxylate **IIA** 3-azidopropyl-(6R,7R)-3-(acetoxymethyl)-7-((tert-butoxycarbo-nyl)amino)-8-oxo-5-thio-1-azabicyclo[4.2.0]oct-2-ene-2-carboxylate **IIA**'

The product has the appearance of a transparent oil and was obtained with a 30% yield. The characterization was effected by NMR analysis and mass spectroscopy (Figure 8). 1 mmole of carboxylic acid is dissolved in 0.5 mL of anhydrous 1,4-dioxane in a 25 mL flask in an atmosphere of N₂ and under stirring. 10 mL of anhydrous diethyl ether are added later. 1 mmole of diisopropylethylamine (DIPEA) is added to the solution which is left to react for 5 minutes at 0°C.

Subsequently 1 mmole of diethyl phosphate chloride is added to the solution which is left under stirring for 30 minutes at 0°C.

A solution of anhydrous diethyl ether containing one millimole of azidopropanol (1) and one millimole of DIPEA is subsequently added dropwise to the solution containing the carboxylic acid maintained at 0°C.

The reaction mixture is reacted for 24 hours and monitored via TLC using a mixture of Hexane/Acetone 7/3.

The solvent is evaporated by means of the rotavapor and purification of the reaction mixture on silica, using Hexane /Acetone 9/1 as eluent, allows the desired product to be isolated with a 30% yield.
¹H NMR (CDCl₃, 400 MHz): δ 6,47 (dt, J = 1,7, 0,8 Hz, 1H, H2), 5,64 (dd, J = 9,7, 4,9 Hz, 1H, H4'), 5,47 (dd, J = 9,4, 3,5 Hz, 1H, H4), 5,32 (d, J = 9,3 Hz, 1H, H5), 5,25(d, J = 3,9 Hz, 1H, H3), 5,22 (d, J = 9,8 Hz, 1H, H5'), 5,14 (d, J = 13,5 Hz, 1H, H6'),5,02 (ddd, J = 1,8, 1,1, 0,7 Hz, 1H, H1), 4,97 (d, J = 4,9 Hz, 1H, H3'), 4,84 (d, J = 13,5 Hz, 1H, H7'), 4,71 (d, J = 12,6 Hz, 1H, H6), 4,59 (d, J = 12,7 Hz, 1H, H7), 4,47-4,41 (m, 1H, H8'), 4,34 - 4,22 (m, 3H, H9-H8, H9'), 3,58 (d, J = 18,4 Hz, 1H, H1'), 3,42 (m, 5H, H2',12-H13, H12'-H13'), 2,09 (s, 3H, OAc'), 2,07 (3H, OAc), 2,01-1,89 (m, 4H, H11-H10, H11'-H10'), 1,45 (s, 18H, Boc)
N.B. The ratio between the two isomers in the NMR analysis, after the chromatographic column, is about 60% IIA and 40% IIA'. Therefore when the signal of a proton is identified, in the case of the isomer IIA' it is about 0.4 of the total signal.
¹³C NMR (CDCl₃, 100 MHz): δ 170,70 (s, 1C, C15'), 170,61 (s, 1C, C15), 167,27 (s, 1C, C8), 165,64 (s, 1C, C6'), 165,25 (s, 1C, C6), 161,44 (s, 1C, C8'), 154,65 (s, 1C, C9'), 154,49 (s, 1C, C9), 126,10 (s, 1C, C2),125,62 (s, 1C, C2'), 122,63 (s, 1C, C3), 119,28 (s, 1C, C1'), 81,46 (s, 1C, C10'), 81,34 (s, 1C, C10), 65,54 (s, 1C, C7), 63,46 (s, 1C, C12) 63,21 (s, 1C, C7'), 63,19 (s,C,C12') 62,19 (s, 1C, C5), 61,15 (s, 1C, C5'), 57,92 (s, 1C, C4'), 53,95, (s, 1C, C4), 50,02 (s, 1C, C1), 48,12 (s, 1C, C14), 48,09 (s, 1C, C14'), 28,33 (s,6C,C11-C11'), 28,14 (s, 1C, C13'), 28,06 (s, 1C, C13), 26,54 (s, 1C, C3'), 20,99 (s, 1C, C16), 20,90 (s, 1C, C16'),
MS (ESI): *m*/*z*; found [M+Na]⁺ = 478,1391, theoretical [M+Na]⁺ = 478,1368 3-(4-(hydroxymethyl)-1H-1,2,3-triazol-1-yl)-propyl(2S,SR,6R)-6-((tert-butoxycarbonyl)amino)-3,3-dimethyl-7-oxo-4-thio-1-azabicyclo [3.2.0] heptane-2-carboxylate **IB**

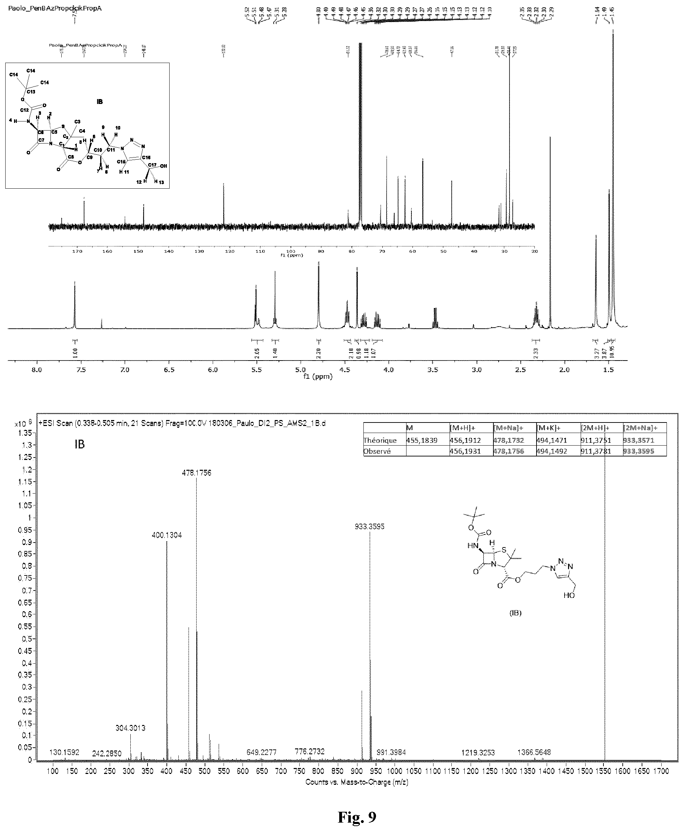

The product has the appearance of a transparent oil and is obtained with a yield of 55%. Characterization (Figure 9).
¹H NMR (CDCl₃, 400 MHz): δ 7.57 (s, 1H, H11), 5.51-5.48 (m, 2H, H2-H3), 5.29 (d, J = 9.3 Hz, 1H, H4), 4.80 (s, 2H, H12-H13), 4.47 (td, J = 6.7, 2.1 Hz, 2H, H9-H10), 4.3 (s, 1H, H1), 4.32-4.26 (m, 1H, H5), 4.16-4.10 (m , 1H, H6), 2.74 (br, 1H, OH), 2.35 - 2.28 (m, 2H, H7-H8), 1.64 (s, 3H, CH3), 1.49 (s, 3H, CH3), 1.45 (s, 9H, 3CH3Boc)
¹³C NMR (CDCl₃, 100, MHz): δ 27.25 (s, 1C, C3), 28.41 (s, 3C, C14), 29.30 (s, 1C, C10), 31.70 (s, 1C, C4), 47.16 (s, 1C, C11), 56.66 (s, 1C, C17), 60.37 (s, 1C, C6), 62.43 (s, 1C, C9), 64.72 (s, 1C, C2), 68.50 (s, 1C, C5) , 70.61 (s, 1C, C1), 81.22 (s, 1C, C13), 122.02 (s, 1C, C15), 148.17 (s, 1C, C16) 154.27 (s, 1C, C12), 167.78 (s, 1C , C8), 175.08 (s, 1C, C7)
MS (ESI): *m*/*z*; found [M + Na] + = 478,1756, theoretical [M + Na] + = 478,1732 3-(4-(hydroxymethyl)-1H-1,2,3-triazol-1-yl)-propyl (6R, 7R)-3-(acetoxymethyl)-7-((tertbutoxycarbonyl)amino)-8-oxo-5-thio-1-azabicyclo [4.2.0] oct-3-ene-2-carboxylate **IIB**
3- (4-(hydroxymethyl)-1H-1,2,3-triazol-1-yl)-propyl-(6R, 7R)-3- (acetoxymethyl)-7-((tert-butoxycarbonyl)-amino)-8-oxo-5-thio-1-azabicyclo[4.2.0]oct-2-ene-2-carboxylate **IIB'**

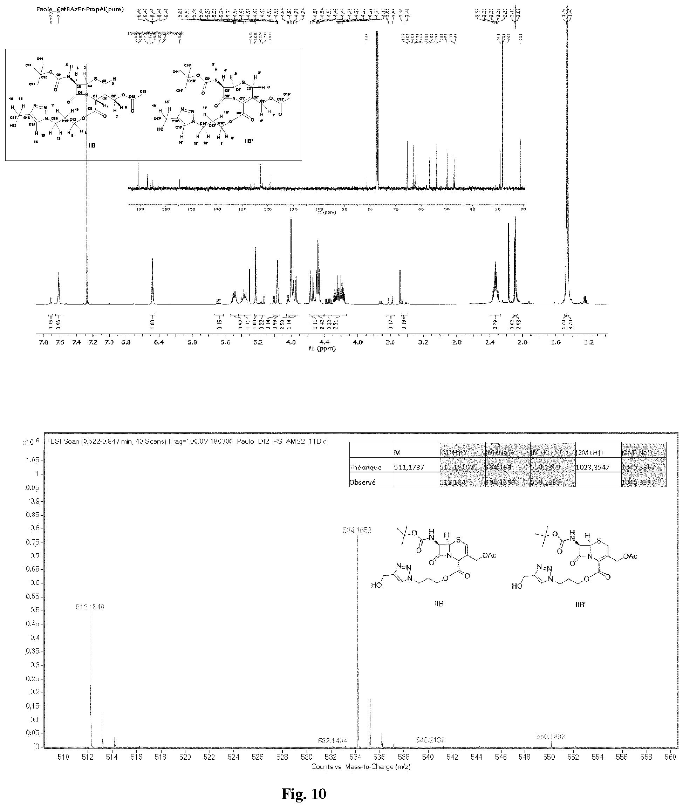

The product has the appearance of a transparent oil whose composition is equal to about 85% IIB and 15% IIB'. The reaction yield is 52%. Characterization (Figure 10):
¹H NMR (CDCl₃, 400 MHz): δ 7.71 (s, 1H, H14'), 7.62 (s, 1H, H14), 6.48 (dt, J = 1.7, 1.0 Hz, 1H, H2), 5.68 (dd, J = 9.8, 4.8 Hz, 1H, H4'), 5.49 (dd, J = 9.4, 3.5 Hz, 1H, H4), 5.40-5.35 (m, 2H, H5, H5'), 5.23 (d, J = 3.9 Hz , 1H, H3), 5.15 (d, J = 13.6 Hz, 1H, H6 '), 5.00 (d, J = 5.0 Hz, 1H, H3'), 4.96 (ddd, J = 1.8, 1.1, 0.7 Hz, 1H , H1) 4.84-4.80 (m, 5H, H16-H15, H16'-H15', H7'), 4.76 (d, J = 12.7 Hz, 1H, H6), 4.55 (d, J = 12.8 Hz, 1H, H7), 4.48 (t, J = 6.6 Hz, 4H, H12-H13, H12'-H13'), 4.35 (m, 1H, H9'), 4.31 - 4.14 (m, 3H, H9-H8, H8') , 3.60 (d, J = 18.6 Hz, 1H, H1'), 3.44 (d, J = 18.5 Hz, 1H, H2'), 2.44 - 2.26 (m, 4H, H10-H11, H10'-H11 '), 2.10 (s, 3H, OAc '), 2.09 (s, 3H, OAc), 1.47 (s, 9H, 3CH3Boc), 1.46 (s, 9H, 3CH3 Boc)
¹³C NMR (CDCl₃, 100 MHz): δ 170.82 (s, 1C, C18 '), 170.76 (s, 1C, C18), 167.14 (s, 1C, C8), 165.97 (s, 1C, C6'), 165.38 (s, 1C, C6), 161.40 (s, 1C, C8'), 154.51 (s, 2C, C9-C9'), 126.61 (s, 1C , C2), 125.26 (s, 1C,C2'), 122.74 (s, 1C,C3), 122.23 (br, 2C, C15-C15') 119.28 (s,1C, Cl'), 81.37 (s,1C, C10-C10), 65.48 (s,1C, C7), 63.20 (s, 1C, C7'), 63.11 (s, 1C, C12), 62 , 61 (s, 1C, C12'), 62.17 (s, 1C, C5), 61.09 (s, 1C, C5') 58.00 (s, 1C, C4 '), 56.68 (s, 2C, C17-C17'), 53.88 (s, 1C,C4), 49.90 (s, 1C,C1), 47.13 (s, 2C, C14-C14'), 29.13 (s, 2C, C13-C13'), 28.32 (s, 6C, C11-C11'), 26.53 (s, 1C,C3'), 21.04 (s, 2C,C19-C19')
MS (ESI): *m*/*z*; found [M + Na]⁺ = 534,1658, theoretical [M + Na]⁺ = 534.163 3-(4-((((2S,SR,6R)-6-((tert-butoxycarbonyl)-amino)-3,3-dime-thyl-7-oxo-4-thio-1-azabicyclo [3.2.0] heptane-2-carbonyl) oxy)-methyl)-1H-1,2,3-triazol-1-yl)-propyl(2S,5R,6R)-6-((tert-butoxycarbonyl)-amino)-3,3-dimethyl-7-oxo-4-thio-1-azabicyclo [3.2.0] heptane-2-carboxylate IC

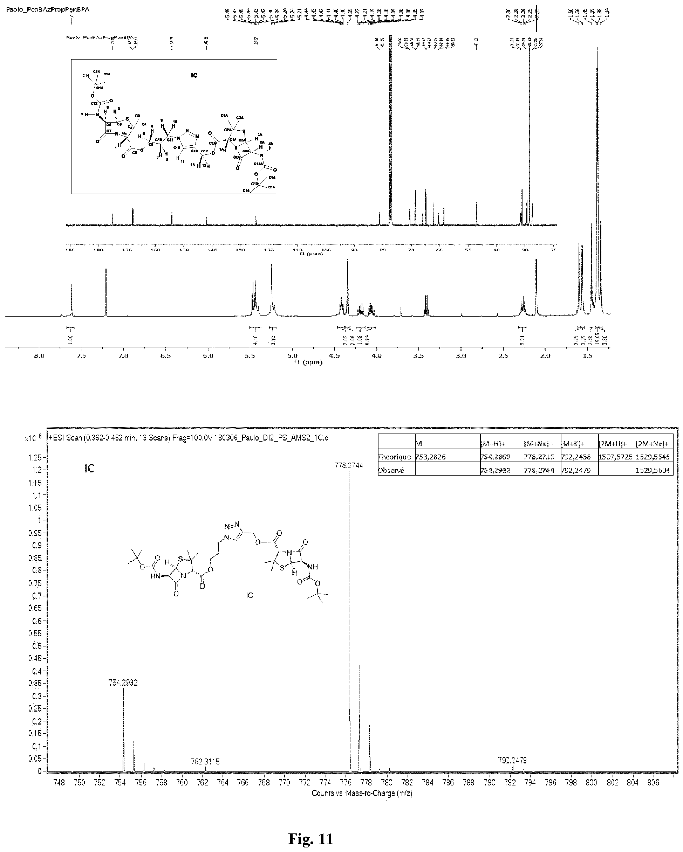

The product has the appearance of a foamy white solid. 60% yield. Melting point 125-127°C. Characterization (Figure 11)
¹H NMR (CDCl₃, 400 MHz): δ 7.62 (d, J = 15.3 Hz, 1H, H11), 5.48-5.39 (m, 4H, H2-H3, H2A-H3A), 5 ,26-5.19(m, 4H, H12-H13, H4-H4A), 4.42 (td, J = 6.8, 2.9 Hz, 2H, H9-H10), 4.35 (s, 2H, H1-H1A), 4.19 (m, 1H, H5), 4.06 (m, 1H, H6), 2.31 - 2.22 (m, 2H, H7-H8), 1.60 (s, 3H, CH3), 1.56 (s, 3HCH3), 1.45 (s, 3H, CH3), 1.39 (s, 9H, 3CH3), 1.38 (s, 9H, 3CH3), 1 ,34 (s, 3H, CH3).
¹³C NMR (CDCl₃, 100 MHz): δ 27.25 (d,2C,C3), 28.32 (s,6C, C14), 29.29 (s 1C C10), 31.51 (d,2C,C4), 47.12 (s,1C,C11), 58.53 (s,1C,C17), 60.29 (d,2C,C6-C6A), 62.06 (s,1C,C9), 64.77 (d,2C,C2-C2A), 68.45 (d,2C,C5-C5A), 70.61 (d,2C,C1-C1A), 81.17 (d, 2C, C13-C13A), 124.57 (s, 1C, C15), 142.10 (s, 1C, C16), 154.24 (s, 2C, C12), 167.79 (s, 1C, C8A), 167.97 (s, 1C, C8) 175.00 (s, 2C, C7-C7A)
MS (ESI): *m*/*z*; found [M + Na]⁺ = 776,2744, theoretical [M + Na]⁺ = 776,2719 3-(4-((((2S,5R,6R)-6-((tert-butoxycarbonyl)-amino)-3,3-dimethyl-7-oxo-4-thio-1-azabicyclo [3.2.0] heptane-2-carbonyl) oxy)-methyl)-1H-1,2,3-triazol-1-yl)-propyl-(2R,6R,7R)-3- (acetoxymethyl)-7-((tert-butoxycarbonyl)-amino)-8-oxo-5-thio-1-azabicyclo [4.2.0]oct-3-ene-2-carboxylate **IIC**
3-(4-((((2S,5R,6R)-6-((tert-butoxycarbonyl)-amino)-3,3-dimethyl-7-oxo-4-thio-1-azabicyclo [3.2.0] heptane-2-carbonyl) oxy)-methyl)-1H-1,2,3-triazol-1-yl)-propyl-(6R,7R)-3- (acetoxymethyl)-7-((tert-butoxycarbonyl)-amino)-8-oxo-5-thio-1-azabicyclo[4.2.0]oct-2-ene-2-carboxylate **IIC**'

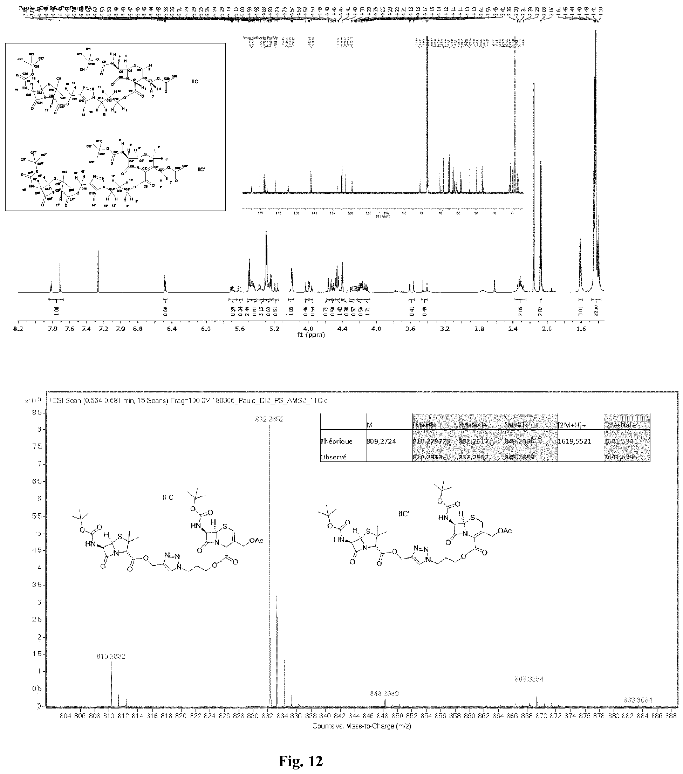

The compound, a white solid, is obtained with a yield of 58%. Melting point 112-115°C, mass spectrum and 1H NMR in Figure 12
¹H NMR (CDCl₃, 400 MHz): δ 7.82 (s,1H,H14'),7.71(s,1H, H14),6.48(dt, J = 1.8, 0.9 Hz, 1H, H2), 5.69 (dd, J = 9.5, 4.7 Hz, 1H, H4'), 5.61 (d, J = 9.5 Hz,1H,5'),5.50-5.49(m, 3H, H3, H18-H18 '), 5.48 - 5.42 (m, 3H, H4, H18-H18'), 5.37 (d, J = 9.5 Hz, 2H, H5-H5 '), 5.32 - 5.26 (m, 6H, H16-H15, H16'-H15', H20-H20'), 5.24 (d, J = 3.9 Hz, 2H, H19-H19'), 5.17 (d, J = 13.7 Hz, 1H, H6'), 5.02 - 4.97 (m, 2H, H3'-H1), 4.81 (d , J = 13.7 Hz, 1H, H7 '),4.77 (d, J = 12.7 Hz, 1H,H7),4.55 (d, J = 12.8 Hz, 1H,H6), 4.50 (t, J = 6.5 Hz,2H, H12'-H13 '), 4.46 (t, J = 6.8 Hz,2H, H12-H13), 4.41 (s, 1H, H17'), 4.40 (s, 1H, H17),4.31-4.07(m, 4H9-H8, H8'-H9'), 3.59 (d, J = 18.6 Hz, 1H,H1'), 3.43(d, J = 18.6 Hz, 1H, H2'), 2.31 (dt, J = 13.2, 6.6 Hz, 4H, H10-H11 'H10'- H11'), 2.08 (s, 3H, OAc'), 2.07 (s, 3H, OAc), 1.61 (m, 3H, CH3, CH3), 1.49 - 1.37 (m, 22H, 18H Boc, CH₃)
¹³C NMR (CDCl₃,100 MHz): δ 174.97,174.85, 170.74, 170.69, 167.94, 167.77, 167.18, 16.22, 165.25, 161.34, 154, 79, 154.45, 154.29, 154.23, 142.04, 141.76, 127.07, 125.09, 124.67, 122.79, 119.13, 81.44, 81.29, 81.12, 70.56, 69.62, 68.44, 68.39, 65.44, 64.96, 64.87, 63.21, 62.68, 62.23, 62.08, 61, 09, 60.25, 58.87, 58.55, 57.99, 46.93, 46.67, 31.58, 31.41, 29.79, 29.37, 29.33, 29.20, 28.31, 27.22, 26.52.
MS (ESI): *m*/*z*; found [M + Na]⁺ = 832,2652, theoretical [M + Na]⁺ = 832,2617 3-(4-((oxiran-2-yl-methoxy)-methyl)-1H-1,2,3-triazol-1-yl)-propyl-(2S,5R,6R)-6-((tertbutoxycarbonyl)-amino)-3,3-dimethyl-7-oxo-4-thio-1-azabicyclo[3.2.0]heptane-2-carboxylate **ID**

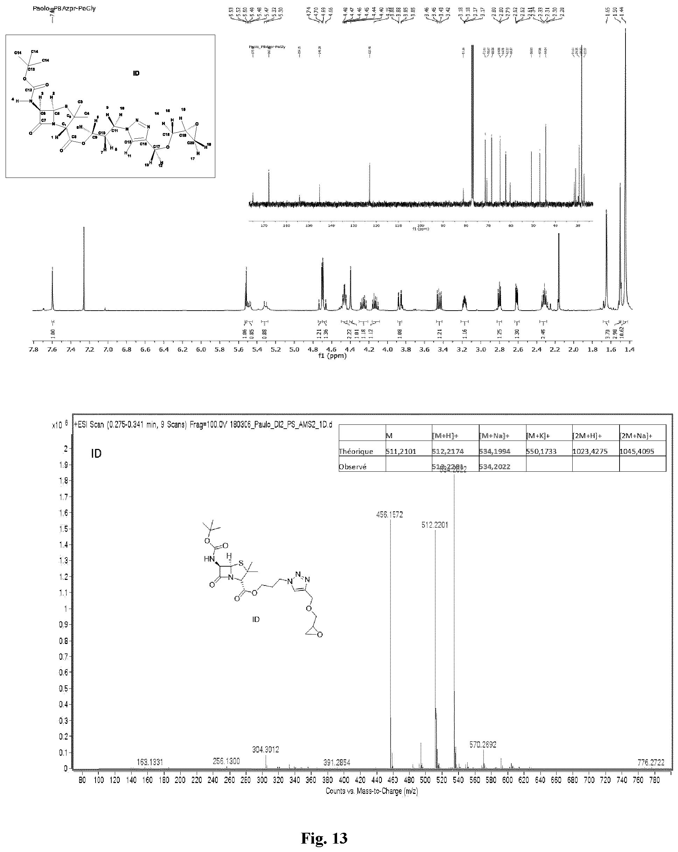

The product, obtained with a 60% yield, has the appearance of a transparent oil. Characterization (Figure 13):
¹H NMR (CDCl₃, 400MHz): δ 7.60 (s,1H,H11), 5.52 (d, J = 4.2 Hz,1H,H2),5.49 (dd, J=9.9, 4.1 Hz,1H,H3), 5.31 ( d, J = 8.7 Hz,1H,H4), 4.72(d, J = 12.3 Hz,1H,H12), 4.68 (d, J = 12.3 Hz,1H,H13), 4.46 (td, J = 6.8,2.0 Hz,2H,H9-H10), 4.40 (s, 1H,H1), 4.26 (m,1H,H5), 4.13 (m,1H,H6), 3.86 (dd, J = 11.5,2.7 Hz,1H,H14) , 3.44(dd, J =11.5,6.2 Hz,2H,H15), 3.17 (m,1H,H16), 2.80 (dd, J = 4.9, 4.2 Hz,1H,H17), 2.61 (dd, J = 5.0, 2.7 Hz,1H,H18), 2.35 - 2.27 (m,2H,H7-H8), 1.65 (s, 3H), 1.50 (s,3H), 1.44 (s,9H).
¹³C NMR (CDCl₃, 100MHz): δ 27.27 (s,lC,C3), 28.33 (s, 3C, C14), 29.35 (s,1C,C10), 31.61(br,1C,C4), 44.34(s,lC, C20), 47.06(s,1C,C11), 50.83(s,1C,C19), 60.37,(s,lC,C5) 62.27(s, 1C,C9), 64.70(s,1C,C17), 64.80(s,lC,C2), 68.50 (s,lC,C6), 70.67(s,lC,C1), 71.41(s,1C,C18), 81.16 (s,1C,C13), 122.90 (s,1C,C15), 145.28(s,1C,C16), 154.25(s,1C,C12), 167.81 (s, 1C,C8), 175.00(s,1C,C7)
MS (ESI) *m*/*z*; found [M + Na]⁺ = 534,2022, theoretical [M + Na]⁺ = 534,1994 3-(4-((oxiran-2-ylmethoxy)-methyl)-1H-1,2,3-triazol-1-yl)-propyl-(2R,6R,7R)-3-(acetoxymethyl)7-((tert-butoxycarbonyl)-amino)-8-oxo-5-thio-1-azabicyclo[4.2.0]oct-3-ene-2-carboxylate **IID**
3-(4-((oxiran-2-yl-methoxy)-methyl)-1H-1,2,3-triazol-1-yl)-propyl-(6R,7R)-3-(acetoxymethyl)-7-((tert-butoxycarbonyl)-amino)-8-oxo-5-thio-1-azabicyclo[4.2.0]oct-2-ene-2-carboxylate **IID**'

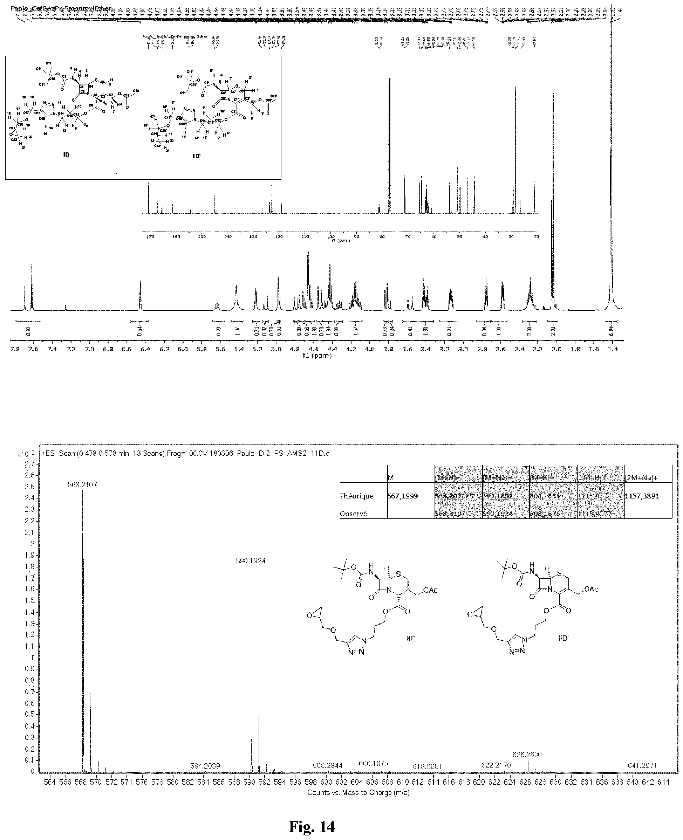

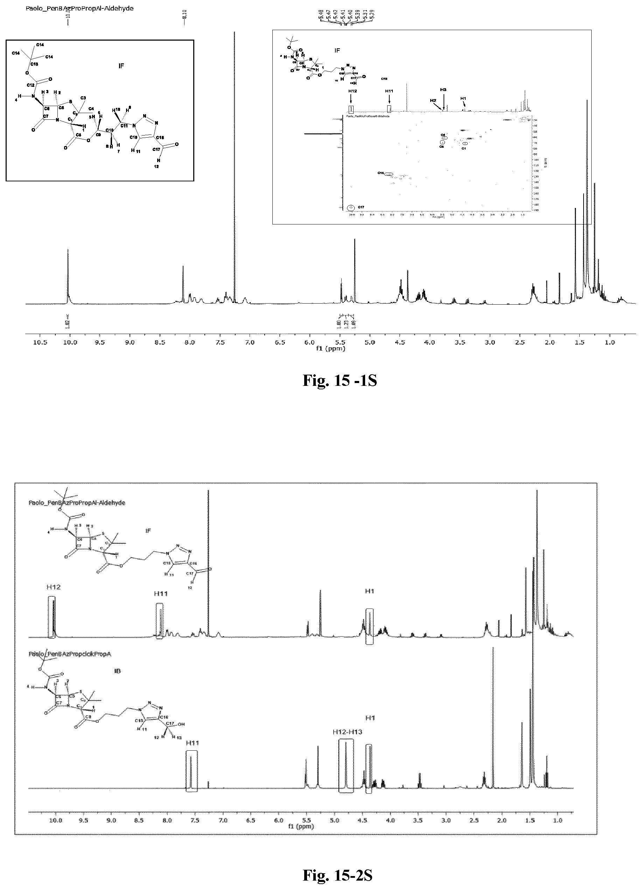

The product has the appearance of a transparent oil. 50% yield. Characterization (Figure 14):
¹H NMR (CDCl₃, 400 MHz):δ 7.69(s,1H,H14'), 7.62(s,1H, H14), 6.46(dt, J=1.8, 0.9Hz,1H,H2), 5.64(dd, J =9.6,4.8 Hz, 1H,H4'), 5.43(br,3H,H5',H5,H4), 5.23-5,21 (m,1H,H3), 5.11 (d, J= 3.6 Hz,1H,H6'), 4.98(d, J=1.8 Hz,1H,H1), 4.97( d, J = 4.6Hz,1H,H3'), 4.79(d, J=13.6Hz,1H,H7'),4.73(d, J=12.7Hz, 1H,H6), 4.71-4.61(m,4H,H16-H15,H16'-H15') 4.53(d, J = 12.7Hz,1H,H7), 4.52-4.38 (m,4H,H12-H13,H12'-H13'), 4.33 (ddd,J= 11.7,7.6,4.2Hz,1H,H9'),4.22-4.08(m,3H,H9-H8,H8'), 3.82(dd, J = 1.5,2.8 Hz, 2H,H17,H17'), 3.57 (d, J = 18.6 Hz, 1H,H1'), 3.43-3.38(m,3H,H2',H18-H18'), 3.15 - 3.11 (m, 2H, H19,H19'), 2.77 - 2 , 74(m,2H,H21,H21'), 2.63-2.53 (m,2H, H20-H20'), 2.35-2.21 (m,4H,H10-H11,H10'-H11'), 2.05 (s, 3H,OAc'), 2.04 (s,3H,OAc), 1.42 (s,9H,Boc', 1.41 (s,9H, Boc).
¹³C NMR (CDCl₃, 100 MHz): δ 170.61(s,21C,C18'-C18), 167.11 (s,lC,C8),165.84 (s,1C,C6'),165.38(s,1C,C6), 161.31 (s,1C,C8'),154.62(s,C,C9'),154.40(s,1C,C9),145.06(s,1C,16), 144.53 (s,1C,C16') 126.66 (s,lC,C2), 125.14 (s,lC,C2'), 123.69 (s,lC,C3), 123,03 (s,1C,C15), 122.65 (s,1C,C15') 119.10 (s,1C,C1'), 81.32 (s,1C,C10'), 81.14 (s,1C,C10), 71.23 (s,lC,C20), 71.06 (s,1C,1C20'), 65.39 (s,lC,C7), 64.64 (s,1C,C17) , 64.59 (s,1C,C17'), 63.08 (s,lC,C7'), 62.63 (s,1C,C12), 62.40 (s,1C,C12'), 62,11 (s,lC,C5), 61.00 (s,lC,C5') 57.89 (s,lC,C4'), 53.82 (s,1C,C4), 50.72 (s,2C, C21-C21') 49.84 (s,1C,C1), 46.70 (s,2C,C14-C14'), 44.31, (s,1C,C22 ') 44.25 (s,lC,C22), 29.33 (s,1C,C13'), 29.14 (s, 1C,C13) 28.21 (s,6C,C11-C11'), 26.43(s,1C,C3'), 20.91 (s, 1C,C19), 20.80 (s,1C,C19')
MS (ESI): *m*/*z*; found [M + Na]⁺ = 590.1924 theoretical [M + Na]⁺ = 590,1892 3-(4-formyl-1H-1,2,3-triazol-1-yl)-propyl(2S,SR,6R)-6-((tert-butoxycarbonyl)-amino)-3,3-dimethyl-7-oxo-4-thio-1-azabicyclo[3.2.0]heptane-2-carboxylate **IF** (Figure 15-1S,2S)

The impure product which is difficult to purify on silica in direct phase, has the appearance of a transparent oil with a fruity fragrance. In the 1H-NMR spectra, the formation of a proton can be noted at 10.04 ppm typical of an aldehyde. In addition, this proton combines, HSQC analysis (Figure 15-1S hereunder), with a carbon at ppm 185.35 ppm, typical of a carbaldehyde. As can be seen from Figure 15-2S (overlapping of the ¹H-NMR of IB with the ¹H-NMR of IF, the protons H12-H13 of IB, following oxidation, disappear to give the proton H12 of IF. The oxidation of C17 from alcohol to aldehyde, moreover, involves the «shift» of H11 of the triazole ring at ppm 8.10.

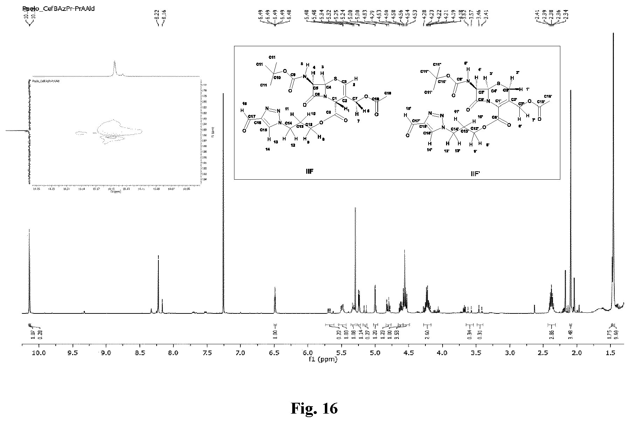

¹H NMR (CDCl₃, 400 MHz): δ 10.04 (1H, H12), 8.11 (s, 1H, H11), 5.48 (d, J = 4.2 Hz, 1H, H2), 5.40 (dd, J = 9.6, 4.0 Hz, 1H, H3), 5.30 (d, J = 8.1 Hz, 1H, H4), 4.37 (s, 2H) 4.37 (s, 1H, H1), 4.19 (m,1H,H5), 4.09 (m,1H,H6), 2.28 (m,2H,H7-H8), 1.57 (s,3H,CH3), 1.43 (s,3H,CH3), 1.38 (s,9H, 3CH3Boc) 3-(4-formyl-1H-1,2,3-triazol-1-yl)-propyl-(2R,6R,7R)-3- (acetoxymethyl)-7-((tert-butoxycarbonyl)-amino)-8-oxo-5-thio-1-azabicyclo [4.2.0] oct-3-ene-2-carboxylate IIF 3-(4-formyl-1H-1,2,3-triazol-1-yl)-propyl(6R,7R)-3-(acetoxymethyl)-7-((tert-butoxycarbonyl)amino)-8-oxo-5-thio-1-azabicyclo[4.2.0]oct-2-ene-2-carboxylate IIF' (Figure 16)

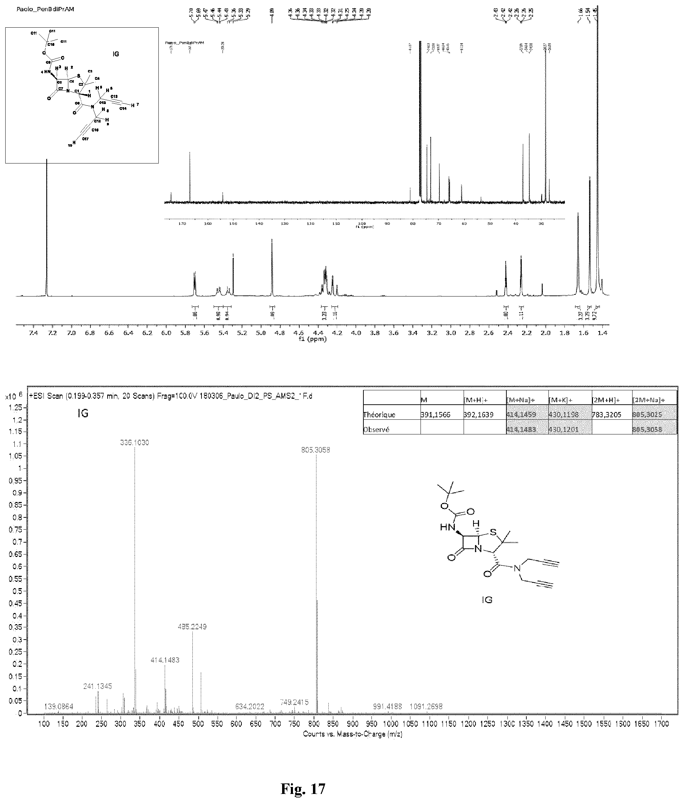

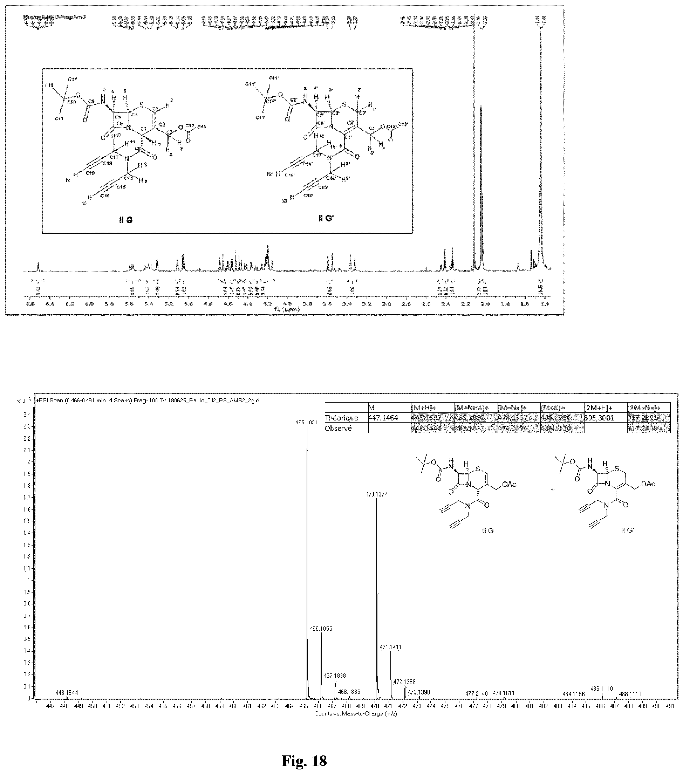

The product IIF also has the appearance of a transparent oil with a fruity odour as a mixture of the two isomers IIF and IIF' in a ratio of about 85:15 in percentage terms. In the ¹H-NMR (Figure 16) the presence can be seen of the proton at 10.15 and 10.13 of the protons H15 and H15'. As can be seen from the HSQC analysis H14 and H14' couple with two carbons at ppm 185.31 and 185.17 ppm
¹H NMR (CDCl₃, 400 MHz): δ 10.15 (s,1H,H15), 10.13 (s, 1H H15'), 8.22 (s,1H,H14), 8.16 (s,1H,H14'), 7.71 (s, 1H, H14'), 6.49 (dt, J = 1.7, 1.0 Hz, 1H,H2), 5.69 (dd, J = 9, 8, 4.8 Hz, 1H,H4 '), 5.49 (dd, J = 9.1, 3.4 Hz, 1H,H4), 5.32 (m, 2H, H5-H5'), 5,24 (d, J = 4.0 Hz, 1H, H3), 5.15 (d, J = 13.6 Hz, 1H, 6'), 5.00-4.98, (m, 2H, H1 -H3'), 4.81 (d, J = 12.7 Hz, 1H, H6), 4.80 (d, J = 13.6 Hz, 1H, H7'), 4.62 (t, J = 6.8 Hz, 2H, H12-H13), 4.56 (t, J = 6.9 Hz, 3H, H12-H13), 4.54 (d, J = 13.1 Hz,1H,H7,) 4.34-4.17 (m,4H,H8-H9,H8'-H9'), 3.59 (d, J = 18.6 Hz, 1H,H1'), 3.43 (d, J = 18.5 Hz, 1H,H2'), 2.45-2.31 (m, 4H,H10-H11, H10'-H11'), 2.10 (s,3H,OAc'), 2.09 (s,3H,OAc), 1.47 (s,9H,3CH3Boc), 1.46 (s,9H, 3CH3 Boc),
¹³C NMR (CDCl₃, 100 MHz): δ 185.17 (s, 2C,C17-C17'), 170.83 (s,1C,C18'), 170.76 (s,1C,C18), 167.21 (s,lC,C8), 165.56 (s,lC,C6'), 165.34 (s,lC,C6), 161.33 (s,lC,C8'), 154.47 (d,2C,C9-C9'), 148.001 (s,2C,C16-C16') 126.81 (s, 1C,C2), 126.70 (s,lC,C2'), 125.79 (s,2C,C15-C15'), 122.91 (s,lC,C3), 119.28 (s,1C,C1'), 81.62 (s,1C,C10'), 81.40 (s, 1C,C10) , 65.57 (s,1C,C7), 63.23 (s,lC,C7'), 62.53 (s,2C, C12-C12'), 62.26 (s,1C,C5), 61,23 (s,lC,C5') 58.01 (s,1C, C4'), 53.91 (s,1C,C4), 49.74 (s,1C,C1), 47.51, (s,lC,Cl4'), 47.32 (s,2C,C14), 29.20 (s,2C,C13-C13'), 28.33 (s,6C,C11-C11'), 26.53 (s,lC,C3'), 21.04 (s,2C,C19), 20.92 (s,1C, C19') ((2R,6R,7R)-7-((tert-butoxycarbonyl)amino)-2-(di(prop-2-in-1-yl)carbamoyl)-8-oxo-5-thio-1-azabicyclo[4.2.0]oct-3-en-3-yl) methyl acetate **IG** (not according to the invention) The product has the appearance of a transparent oil. 20% yield. Characterization (Figure 17):
   ¹H NMR (CD₂ Cl₂, 400 MHz): δ 5.70 (d, J = 4.1 Hz, 1H, H2), 5.45 (dd, J = 10.0, 3.6 Hz, 1H, H3), 5.35 (d, J = 10.0 Hz, 1H , H4), 4.89 (s, 1H, H1), 4.37 - 4.29 (m, 3H, H8, H5-H6), 4.22 (dd, J = 18.6, 2.4 Hz, 1H, H9), 2.42 (t, J = 2.4 Hz, 1H, H10), 2.26 (t, J = 2.5 Hz, 1H, H7), 1.66 (s, 3H), 1.54 (s, 3H), 1.45 (s, 9H)
   ¹³C NMR (CDCl₃, 100 MHz): δ 26.89 (s, 1C, C3), 28.37 (s, 3C, C11), 34.58 (s, 1C, C12), 34.64 (s, 1C, C4), 37.09 (s, 1C, 15), 61.14 (s, 1C, C6), 65.75 (s, 1C, C2), 66.04 (s, 1C, Cl), 69.77 (s, 1C, C5), 73.00 (s, 2C, C17-C14 ), 74.63 (s, 2C, C16-C13), 81.07 (s, 1C) 154.24 (s, 1C, C9), 167.10, (s, 1C, C8), 174.33 (s, 1C, C7)
   MS (ESI): *m*/*z*; found [2M + Na]⁺ = 805,3025 theoretical [2M + Na]⁺ = 805,3058 ((2R,6R,7R)-7- ((tert-butoxycarbonyl) amino)-2- (di (prop-2-in-1-yl)carbamoyl)-8-oxo-5-thio-1-azabicyclo [4.2.0] oct-3-en-3-yl) methyl acetate **IIG** (not according to the invention) ((6R,7R) -7-((tert-butoxycarbonyl) amino)-2-(di (prop-2-in-1-yl) carbamoyl)-8-oxo-5-thio-1-azabicyclo[4.2.0]oct-2-en-3-yl) methyl acetate **IIG'** (not according to the invention) Transparent oil. 10% yield. Mixture of two isomers IIG and IIG' in a 30% to 70% ratio. Characterization (Figure 18):
      ¹H NMR (CD₂Cl₂ , 400 MHz): δ 6.52 (dt, J = 1.8, 1.0 Hz, 1H, H2), 5.57 (dd, J = 9.8, 4.8 Hz, 1H , H4 '), 5.45 - 5.36 (m, 3H, H5-H4, H5'), 5.31 (d, J = 1.4 Hz, 1H, H1), 5.11 (d, J = 3.7 Hz, 1H, H3), 5.05 (d, J = 5.0 Hz, 1H, H3 '), 4.67 (d, J = 12.9 Hz, 1H, H6'), 4 , 64 - 4.55 (m, 2H, H6, H8 '), 4.50 (d, J = 12.9 Hz, 1H, H7'), 4.45 (d, J = 12.7 Hz, 1H , H6), 4.42 - 4.35 (m, 2H, H8-H9), 4.32-4.30 (m, 1H, H10), 4.27 - 4.13 (m, 4H, H9 ' -H10'-H11 ', H11), 3.57 (d, J = 17.8 Hz, 1H, H1'), 3.34 (d, J = 17.8 Hz, 1H, H2 '), 2, 45 (t, J = 2.4 Hz, 1 H, H 13), 2.41 (t, J = 2.5 Hz, 1 H, H 13 '), 2.35 (t, J = 2.4 Hz, 1H, H12), 2.34 (t, J = 2.5Hz, 1H, H12 '), 2.05 (s, 3H, OAc'), 2.03 (s, 3H, OAc), 1.44 (s , 9H, 3CH3 '), 1.43 (s, 9H, 3CH3)
      MS (ESI): *m*/*z*; found [M + Na]⁺ = 470,1374 theoretical [M + Na]⁺ = 470,1357 tert-Butyl((2S,SR,6R)-2-(bis((1-(3-hydroxypropyl)-1H-1,2,3-triazol-3-yl) methyl) carbamoyl)-3,3-dimethyl-7-oxo-4-thio-1-azabicyclo [3.2.0]heptan-6-yl) carbamate **IH** (not according to the invention)

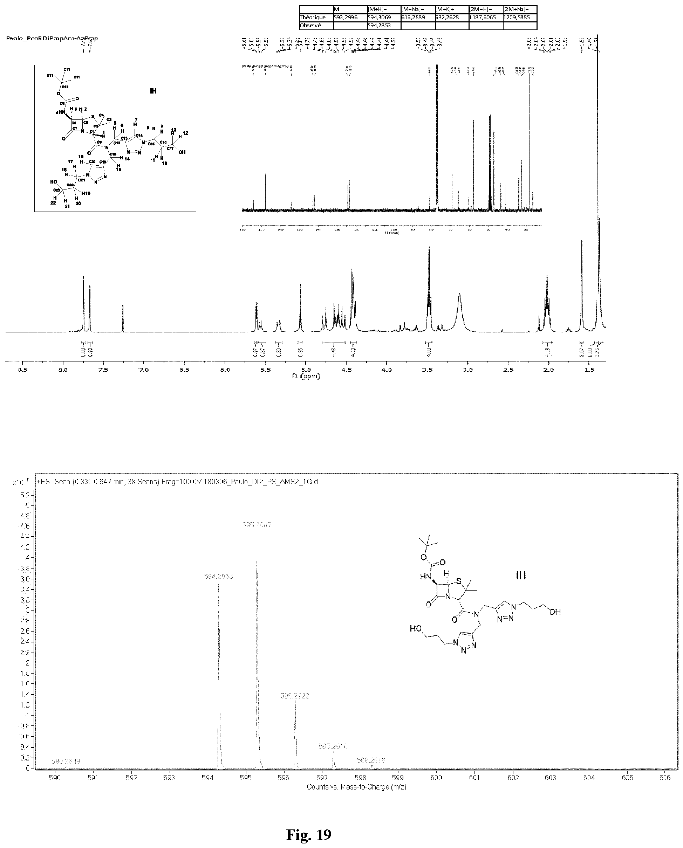

The product is obtained as a white solid with a yield of 43%. Melting point 153-158°C. Characterization (Figure 19):
1H NMR (CDCl₃, 400 MHz): δ 7.75 (s, 1H, H7), 7.67 (s, 1H, H16), 5.61 (d, J = 4.1 Hz, 1H, H2), 5.56 (d, J = 9.8 Hz, 1H, H4), 5.33 (dd, J = 9.3, 4.5 Hz, 1H, H3), 5.07 (s, 1H, H1) , 4.77 (d, 16.6 Hz, 1H, H5), 4.63 (d, J = 16.6 Hz, 1H, H6), 4.61 (d, J = 14.9 Hz, 1H, H14), 4.54 (d, J = 14.8 Hz, 1H, H15), 4.43 (t, J = 6.7 Hz, 2H, H8-H9), 4.41 (t, J = 6.8 Hz, 2H, H17-H18), 3 , 50 - 3.45 (m, 4H, H12-H13, H21-H22), 2.07-1.96 (m, 4H, H8-H9, H17-H18), 1.59 (s, 3H, CH3 ), 1.40 (s, 9H, 3CH3), 1.37 (s, 3H, CH3)
¹³C NMR (CDCl₃, 100 MHz): δ 26.40 (s, 1C, C3), 28.12 (s, 3C, Boc), 32.30 (s, 1C, C16), 32.41 (s, 1C , C22), 41.07 (s, 1C, C18), 43.58 (s, 1C, C12), 47.11 (s, 2C, C15-C21), 57.95 (s, 2C, C17-C23 ), 60.68 (s, 1C, C6), 65.52 (s, 1C, C2), 66.01 (s, 1C, C1), 69.26 (s, 1C, C5), 81.07 ( s, 1C, C10), 123.70 (s, 1C, C20), 124.41 (s, 1C, C14), 142.13 (s, 1C, C19), 142.82 (s, 1C, C13) , 154.39 (s, 1C, C9), 167.94 (s, 1C, C8), 174.31 (s, 1C, C7) MS (ESI): *m*/*z*; found [M + H]⁺ = 594,2853 theoretical [M + H]⁺ = 594,3069 *tert*-butyl((2S,5R,6R)-2-((3-azidopropyl)carbamoyl)-3,3-dimethyl-7-oxo-4-thio-1-azabicyclo[3.2.0]heptan-6-yl) carbamate **IL**

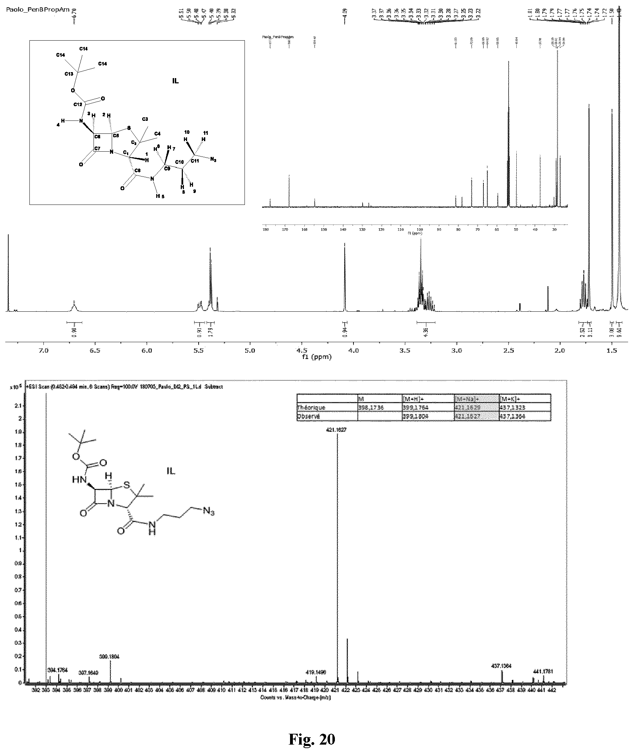

The product has the appearance of a transparent oil and is obtained with a yield of 23%. Characterization (Figure 20):
NMR (CD₂ Cl₂, 400MHz): δ 6.70 (s, 1H, H5), 5.49 (dd, J = 9.9, 4.1 Hz, 1H, H3), 5.40-5.38 (m, 2H, H2-H4), 4.09 (s, 1H, H1), 3.42 - 3.19 (m, 4H, H6-H7, H10-H11), 1.81 - 1.73 (m, 2H, H8-H9), 1.72 (s, 3H, CH3), 1.50 (s, 3H, CH3), 1.43 (s, 9H, 3CH3) .13C NMR (CD2Cl2, 100 MHz): □ 26.96 (C3), 28.44 (C14), 28.81 (C4), 29.19 (C10), 37.40 (C9), 49.84 (C11 ), 59.55 (C6), 64.92 (C2), 66.99 (C5), 73.09 (C1), 81.33 (C13), 154.68 (C12), 169.01 (C8), 177.79 (C7)
MS (ESI): *m*/*z*; found [M + Na]⁺ = 421,1627 theoretical [M + Na]⁺ = 421,1629 ((2R,6R,7R)-2-((3-azidopropyl)carbamoyl)-7-((tert-butoxycar-bonyl) amino) -8-oxo-5-thio-1-azabicyclo [4.2.0] oct-3 -en-3-yl) methyl acetate **IIL**
((6R,7R)-2-((3-azidopropyl)carbamoyl)-7-((tert-butoxy-carbonyl) amino) -8-oxo-5-thio-1-azabicyclo [4.2.0] oct-2-en-3-yl) methyl acetate **IIL'**

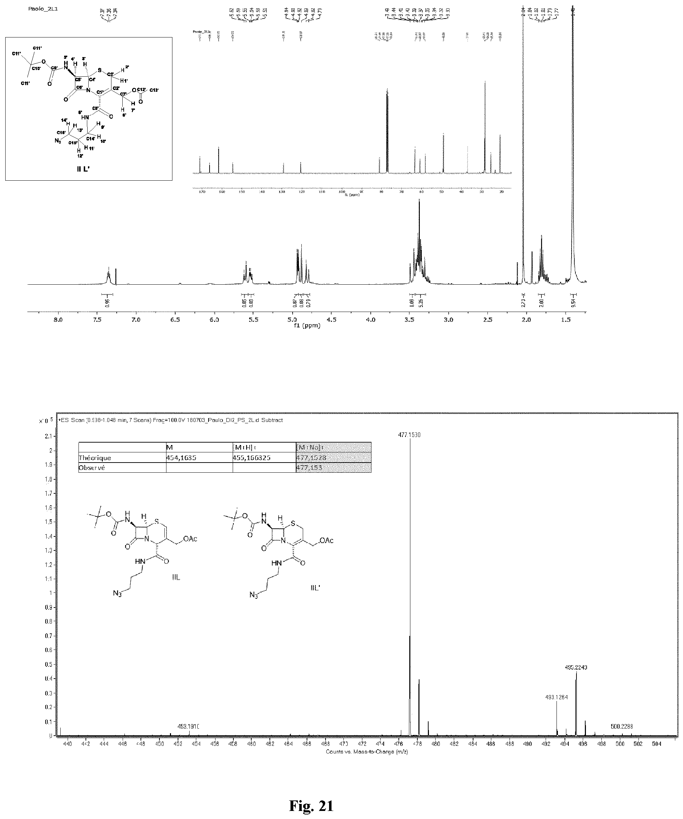

The reaction product is a mixture of the two isomers IIL and IIL' in a ratio of 30 to 70 in percentage terms with a yield of 15%. The product has the appearance of a transparent oil. Purification by direct phase chromatography allowed the product IIL' to be isolated with a 10% yield. Characterization (Figure 21).

¹H NMR (CDCl₃, 400 MHz): δ 7.35 (t, J = 5.7 Hz, 1H, NH8'), 5.60 (d, J = 9.6 Hz, 1H, NH5'), 5.53 (dd, J = 9.5, 4.7 Hz , 1H, H4 '), 4.93 (d, J = 4.8 Hz, 1H, H3'), 4.90 (d, J = 13.2 Hz, 1H, H6'), 4.81 (d, J = 13.2 Hz, 1H, H7' ), 3.47 (d, J = 18.1 Hz, 1H, H1'), 3.42 - 3.29 (m, 5H, H9'-H10', H13'-H14 ', H2'), 2.04 (s, 3H, CH3), 1.81 (p, J = 6.7 Hz, 2H, H12'-Hl1'), 1.41 (s, 9H, 3CH3). ¹³C NMR (CDCl₃, 100 MHz): δ 20.84 (C13'), 25.44 (C3'), 28.20 (Cll '), 28.41 (C15'), 37.18 (C14'), 49.86 (C16'), 58.07 ( C4'), 60.87 (C5'), 63.41 (C7'), 81.21 (C10'), 120.57 (C1'), 129.15 (C2'), 154.72 (C9'), 161.70 (C8'), 166.19 (C6'), 171.12 (C12'). MS (ESI): *m*/*z*; found [M + Na]⁺ = 477,153 theoretical [M + Na]⁺ = 477,1528 *tert*-butyl((2S,5R,6R)-2-((3-(4-(hydroxymethyl)-1H-1,2,3-triazol-1-yl)propyl)carbamoyl)-3,3-dimethyl-7-oxo-4-thio-1-azabicyclo[3.2.0] heptan-6-yl)carbamate **IM**

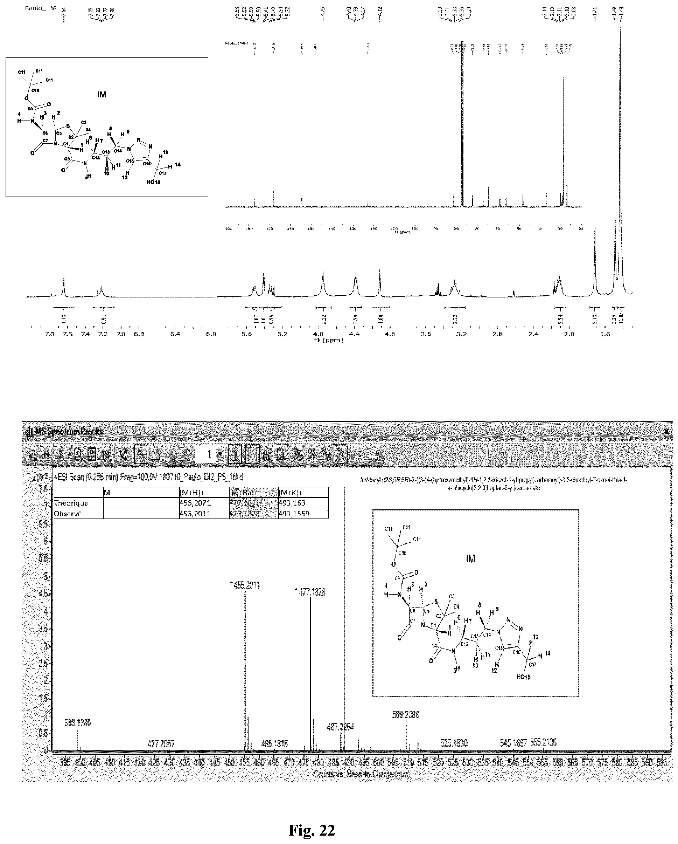

The product, obtained with a 60% yield, has the appearance of a white solid. Melting point 118-121°C. Characterization (Figure 22):
¹H NMR (CD₂Cl₂, 400 MHz): δ 7.64 (s, 1H, H12), 7.31 - 7.08 (m, 1H, H5), 5.51 (dd, J = 9.6, 3.7 Hz, 1H, H3), 5.41 (d, J = 4.5 Hz, 1H, H2), 5.33 (d, J = 9.8 Hz, 1H, H4), 4.75 (s, 2H, H13-14), 4.39 (t, J = 6.2 Hz, 2H, H8-H9) , 4.12 (s, 1H, H1) 3.38 - 3.16 (m, 2H, H6-H7), 21.14-2.06 (m, 2H, H10-H11), 1.71 (s, 3H, CH3), 1.49 (s, 3H, CH3), 1.43 (s, 9H, 3CH3),
¹³C NMR (CD₂Cl₂, 100 MHz): δ 177.18 (s, 1C, C7), 168.23 (s, 1C, C8), 154.40 (s, 1C, C9), 148.08 (s, 1C, C16), 122.79 (s, 1C, C15), 81.20, (s, 1C, C10), 72.26 (s, 1C, C1), 66.80 (s, 1C, C5), 64.63 (s, 1C, C2), 59.11 (s, 1C, C6), 56.09 (s, 1C, C17), 48.18 (s, 1C, C14), 36.68 (s, 1C, C12), 29.83 (s, 1C, C13), 28.99 (s, 1C, C4), 28.28 (s, 3C, C11), 26.75 (s, 1C, C3)
MS (ESI): *m*/*z*; found [M + Na]⁺ = 477, 1528 theoretical [M + Na]⁺ = 477,1891 1,3-diazidopropan-2-yl(2S,5R,6R)-6-((tert-butoxycarbonyl) amino)-3,3-dimethyl-7-oxo-4-thia-1-azabicyclo [3.2.0] heptane-2-carboxylate **IJ**

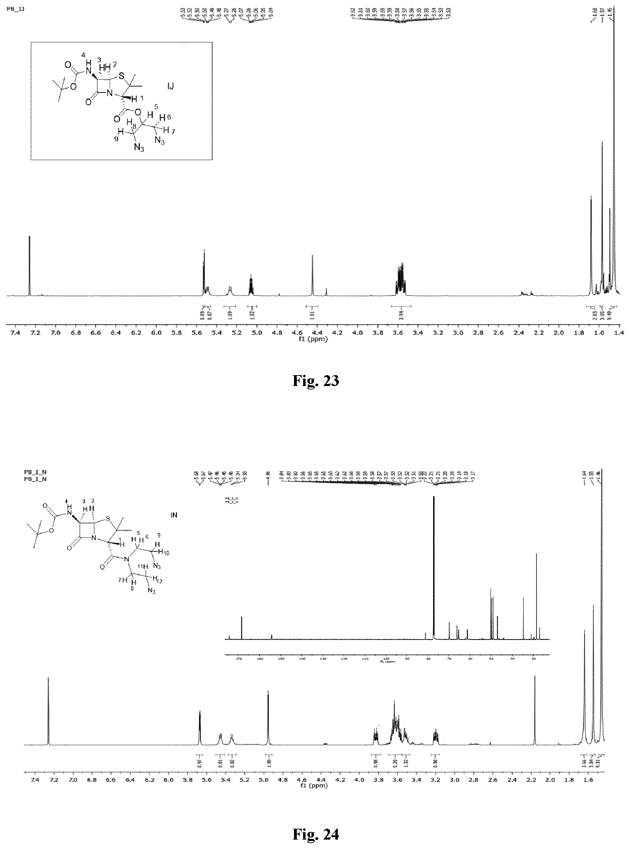

The product has the appearance of a transparent oil obtained, after purification on silica in direct phase, with a yield of 10%. Characterization (Figure 23):
¹H NMR (CD₂Cl₂, 600 MHz): δ 5.53 (d, J = 4.3 Hz, 1H,), 5.49 (dd, J = 9.6, 3.9 Hz, 1H,), 5.26 (d, J = 9.4 Hz, 1H), 5.10-5.02 (m, 1H), 4.45 (s, 1H), 3.63 - 3.51 (m, 4H) 1.68 (s, 3H), 1.57 (s, 3H), 1.45 (s, 9H) tert-butyl((2S,5R,6R)-2-(bis(2-azidoethyl)carbamoyl)-3,3-dimethyl-7-oxo-4-thia-1-azabicyclo[3.2.0]heptan-6-yl) carbamate **IN**

The product has the appearance of a pale-yellow oil and is obtained with a yield of 23%. Characterization (Figure 24):
¹H NMR 600 (MHz) CD₃Cl: δ 5.67 (d, J = 4.1 Hz, 1H), 5.46 (dd, J = 9.7, 3.1 Hz, 1H), 5.34 (d, J = 9.2 Hz, 1H), 4.96 (s, 1H), 3.83 (dt, J = 13.9, 4.7 Hz, 1H),3.68 - 3.55 (m,5H), 3.54 - 3.48 (m, 1H), 3.20 (ddd, J = 13.8, 8.2, 4.5 Hz, 1H), 1.64 (s, 3H), 1.55 (s, 3H), 1.46 (s, 9H)
((6R,7R)-2-(bis(2-azidoethyl)carbamoyl)-7-((tert-butoxycarbo-nyl)amino)-8-oxo-5-thia-1-azabicyclo[4.2.0]oct-2-en-3-yl) methyl acetate **IIN'**

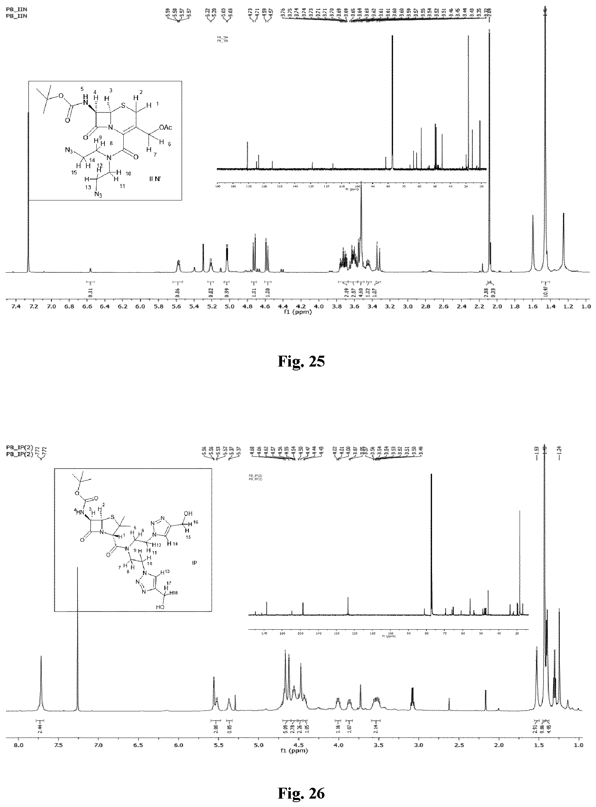

The product, still impure due to the unreacted starting amine and difficult to eliminate by direct phase column chromatography, but free of the isomer IIN, has the appearance of a reddish oil. Obtained with a 7% yield. Characterization (Figure 25):
¹H NMR 600 (MHz) CD₃Cl: δ 5.58 (dd, J = 9.1, 4.7 Hz, 1H), 5.21 (d, J = 9.4 Hz, 1H), 5.03 (d, J = 4.9 Hz, 1H), 4.78 (d , J = 12.9 Hz, 1H), 4.58 (d, J = 12.9 Hz, 1H), 3.78 - 3.68 (m, 2H), 3.67-3.56 (m, 3H) 3.56 - 3.51 (m, 4H), 3.46-3.43 (m, 1H), 3.33 (d, J = 17.8 Hz, 1H), 2.09 (s, 3H), 1.46 (s, 9H) tert-butyl ((2S,5R,6R)-2-(bis(2- (4- (hydroxymethyl) -1H-1,2,3-triazol-1-yl)ethyl)carbamoyl)-3,3-dimethyl-7-oxo-4-thia-1-azabicyclo [3.2.0] heptan-6-yl)carbamate **IP**

The product has the appearance of a whitish semisolid. Obtained with a 30% yield. Also in this case some impurities are difficult to eliminate by purification on silica in direct phase. Characterization (Figure 26):
¹H NMR 600 (MHz) CDCl₃: δ 7.72 (s, 1H), 7.71 (s, 1H), 5.56 (d, J = 3.9 Hz, 1H), 5.52 (d, J = 8.7 Hz, 1H), 5.37 (d , J = 5.0 Hz, 1H), 4.72 - 4.38 (m, 11H), 4.05 - 3.97 (m, 1H), 3.90 - 3.82 (m, 1H), 3.59 - 3.46 (m, 2H), 1.53 (s, 3H), 1.43 (s, 9H), 1.24 (s, 3H)

## Claims

1. A compound selected from the following:
3-azidopropyl-(2S,SR,6R)-6-((tert-butoxycarbonyl)-amino)-3,3-dimethyl-7-oxo-4-thio-1-azabicyclo[3.2.0]heptane-2-carboxylate **IA** 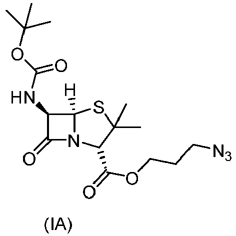
3-azidopropyl-(2R,6R,7R)-3-(acetoxymethyl)-7-((tert-butoxycarbonyl)-amino)-8-oxo-5-thio-1-azabicyclo [4.2.0]oct-3-ene-2-carboxylate **IIA** 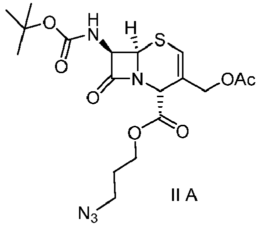
3-azidopropyl-(6R,7R)-3-(acetoxymethyl)-7-((tert-butoxycarbonyl)-amino)-8-oxo-5-thio-1-azabicyclo[4.2.0]oct-2-ene-2-carboxylate **IIA'** 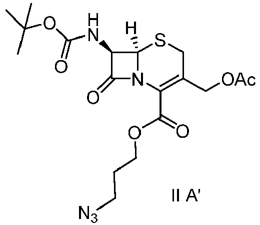
3-(4-(hydroxymethyl)-1H-1,2,3-triazol-1-yl)-propyl-(2S,SR,6R)-6-((tert-butoxycarbonyl)-amino)-3,3-dimethyl-7-oxo-4-thio-1-azabicyclo[3.2.0]heptane-2-carboxylate **IB** 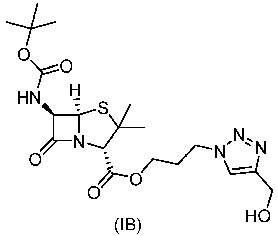
3-(4-(hydroxymethyl)-1H-1,2,3-triazol-1-yl)-propyl-(2R,6R,7R)-3-(acetoxy-methyl)-7-((tert-butoxycarbonyl)-amino)-8-oxo-5-thio-1-azabicyclo[4.2.0]oct-3-ene-2-carboxylate IIB 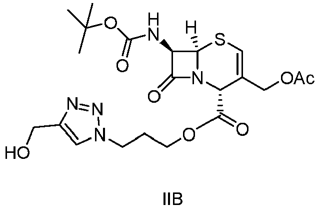
3-(4-(hydroxymethyl)-1H-1,2,3-triazol-1-yl)-propyl-(6R,7R)-3-(acetoxymethyl)-7-((tert-butoxycarbonyl)-amino)-8-oxo-5-thio-1-azabicyclo[4.2.0]oct-2-ene-2-carboxylate IIB' 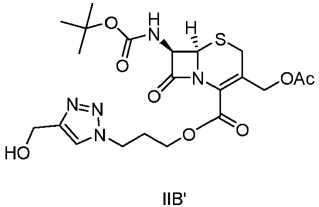
3-(4-((((2S,SR,6R)-6-((tert-butoxycarbonyl)-amino)-3,3-dimethyl-7-oxo-4-thio-1-azabicyclo[3.2.0]heptane-2-carbonyl)oxy)-methyl)-1H-1,2,3-triazol-1-yl)-propyl-(2S,SR,6R)-6-((tert-butoxycarbonyl)-amino)-3,3-dimethyl-7-oxo-4-thio-1-azabicyclo[3.2.0]heptane-2-carboxylate **IC** 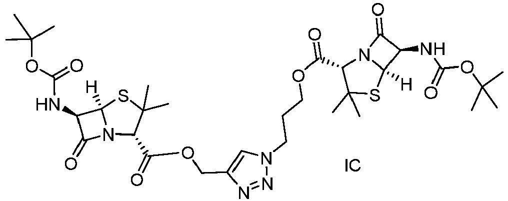
3-(4-((((2S,SR,6R)-6-((tert-butoxycarbonyl)-amino)-3,3-dimethyl-7-oxo-4-thio-1-azabicyclo[3.2.0]heptane-2-carbonyl)oxy)-methyl)-1H-1,2,3-triazol-1-yl)-propyl-(2R,6R,7R)-3-(acetoxymethyl)-7-((tert-butoxycarbonyl)-amino)-8-oxo-5-thio-1-azabicyclo[4.2.0]oct-3-ene-2-carboxylate IIC 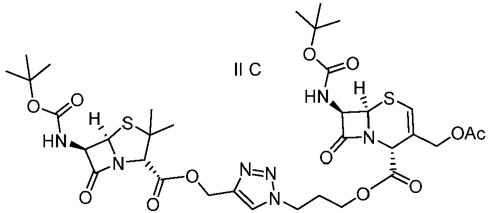
3-(4-((((2S,SR,6R)-6-((tert-butoxycarbonyl)-amino)-3,3-dimethyl-7-oxo-4-thio-1-azabicyclo[3.2.0]heptane-2-carbonyl)oxy)-methyl)-1H-1,2,3-triazol-1-yl)-propyl-(6R,7R)-3-(acetoxymethyl)-7-((tert-butoxycarbonyl)-amino)-8-oxo-5-thio-1-azabicyclo [4.2.0] oct-2-ene-2-carboxylate **IIC'** 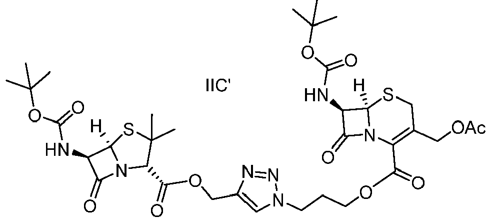
3-(4-((oxyran-2-yl-methoxy)-methyl)-1H-1,2,3-triazol-1-yl)-propyl-(2S,SR,6R)-6-((tert-butoxycarbonyl)-amino)-3,3-dimethyl-7-oxo-4-thio-1-azabicyclo-[3.2.0]heptane-2-carboxylate **ID** 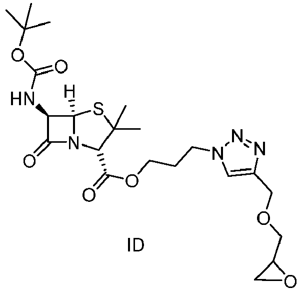
3-(4-((oxyran-2-yl-methoxy)-methyl)-1H-1,2,3-triazol-1-yl)-propyl-(2R,6R,7R)-3-(acetoxymethyl)-7-((tert-butoxycarbonyl)-amino)-8-oxo-5-thio-1-azabicyclo-[4.2.0]oct-3-ene-2-carboxylate **IID** 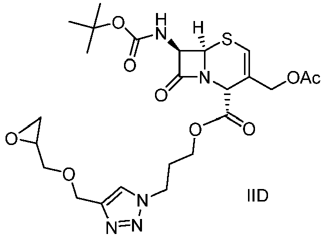
3-(4-((oxyran-2-yl-methoxy)-methyl)-1H-1,2,3-triazol-1-yl)-propyl-(6R,7R)-3-(acetoxymethyl)-7-((tert-butoxycarbonyl)-amino)-8-oxo-5-thio-1-azabicyclo-[4.2.0]oct-2-ene-2-carboxylate **IID'** 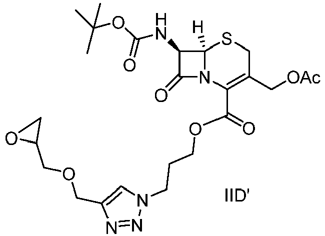
3-(4-formyl-1H-1,2,3-triazol-1-yl)-propyl-(2S,SR,6R)-6-((tert-butoxycarbonyl)-amino)-3,3-dimethyl-7-oxo-4-thio-1-azabicyclo[3.2.0]heptane-2-carboxylate **IF** 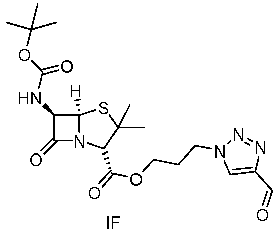
3-(4-formyl-1H-1,2,3-triazol-1-yl)-propyl-(2R,6R,7R)-3-(acetoxymethyl)-7- ((tert-butoxycarbonyl)-amino)-8-oxo-5-thio-1-azabicyclo[4.2.0]oct-3-ene-2-carboxylate IIF 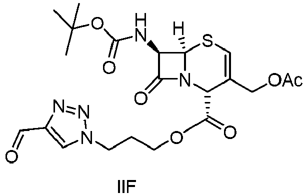
3-(4-formyl-1H-1,2,3-triazol-1-yl)-propyl-(6R,7R)-3-(acetoxymethyl)-7-((tert-butoxycarbonyl)-amino)-8-oxo-5-thio-1-azabicyclo[4.2.0]oct-2-ene-2-carboxylate IIF' 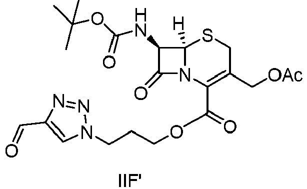
1,3-diazidopropan-2-yl(2S,5R,6R)-6-((tert-butoxycarbonyl) amino)-3,3-dimethyl-7-oxo-4-thia-1-azabicyclo[3.2.0]heptane-2-carboxylate IJ 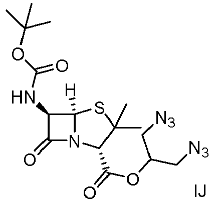
tert-butyl ((2S,SR,6R)-2-((3-azidopropyl)carbamoyl)-3,3-dime-thyl-7-oxo-4-thio-1-azabicyclo[3.2.0]heptan-6-yl)carbamate **IL** 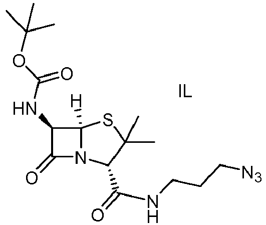
((2R,6R,7R)-2-((3-azidopropyl)carbamoyl)-7-((tert-butoxycarbonyl)amino)-8-oxo-5-thio-1-azabicyclo[4.2.0]oct-3-en-3-yl)methyl acetate **IIL** 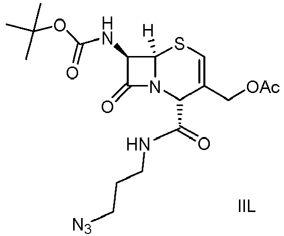
((6R,7R)-2-((3-azidopropyl)carbamoyl)-7-((tert-butoxycarbonyl)amino)-8-oxo-5-thio-1-azabicyclo[4.2.0]oct-2-en-3-yl)methyl acetate **IIL**' 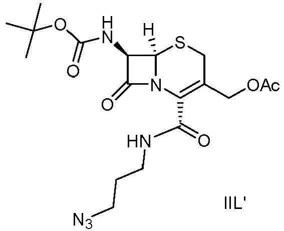
tert-butyl-((2S,SR,6R)-2-((3-(4-(hydroxymethyl)-1H-1,2,3-triazol-1-yl)propyl)carbamoyl)-3,3-dimethyl-7-oxo-4-thio-1-azabicyclo[3.2.0]heptan-6-yl)carbamate **IM** 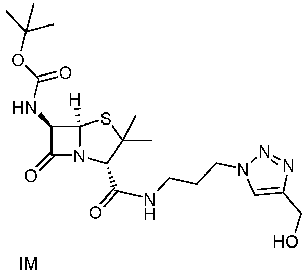
tert-butyl((2S,SR,6R)-2-(bis(2-azidoethyl)carbamoyl)-3,3-dimethyl-7-oxo-4-thia-1-azabicyclo[3.2.0]heptan-6-yl)carbamate **IN** 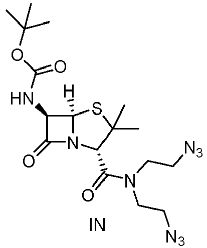
((6R,7R)-2-(bis(2-azidoethyl)carbamoyl)-7-((tert-butoxycarbonyl)amino)-8-oxo-5-thia-1-azabicyclo[4.2.0]oct-2-en-3-yl)methyl acetate IIN' 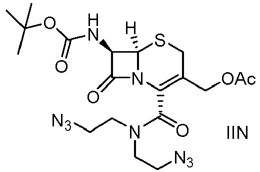
tert-butyl((2S ,5R,6R)-2-(bis(2-(4-(hydroxymethyl)- 1H-1,2,3-triazol-1 -yl)ethyl)-carbamoyl)-3,3-dimethyl-7-oxo-4-thia-1-azabicyclo[3.2.0]heptan-6-yl)carbamate **IP**

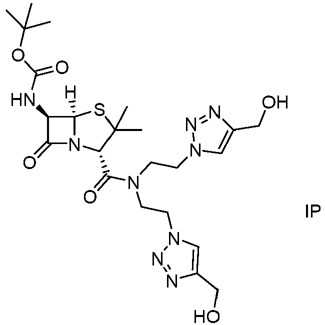

2. A compound according to claim 1, or a mixture comprising two or more compounds according to claim 1, for use as a medicament or as a precursor of a medicament, preferably as an antibiotic medicament or as a beta-lactamase inhibitor, as an antifungal and/or an antitumour agent.

3. A process for the synthesis of precursors of derivatives of β-lactam compounds selected from 6-aminopenicillanic acid (6-APA) and 7-aminocephalosporanic acid (7-ACA), preferably 6-aminopenicillanic acid (6-APA), according to claim 1, which comprises the following steps:
a) protection of the amino group in position 6 of 6-APA or in position 7 of 7-ACA by the formation of a carbamate;
b) esterification of the carboxyl group of the β-lactam compound obtained in step a) by reaction with an aliphatic alcohol, having from 3 to 10 carbon atoms, preferably from 3 to 5, more preferably 3, and containing at least one azide group or amidation of the carboxyl group of the β-lactam compound obtained in step a) by reaction with a primary aliphatic amine having from 3 to 10 carbon atoms, preferably from 3 to 5, more preferably 3, and containing an azide group or with a secondary aliphatic amine having from 4 to 10 carbon atoms, preferably 4, and containing two azide groups to obtain a tertiary amide.

4. A process for the synthesis of triazole derivatives of β-lactam according to claim 1, wherein the precursor obtained according to claim 3 is subjected to the following step c):
c) cycloaddition reaction between the derivative obtained in step b) having at least one azide group and a compound having at least one alkyne group (C≡C), to form the corresponding triazole derivative of the β-lactam compound;
and wherein, optionally, the cycloaddition reaction product of step c) is subjected to the following step d):
d) the precursor obtained at the end of step c) and containing a primary alcohol group is subjected to an oxidation of the primary alcohol group to obtain an aldehyde derivative.

5. The process according to one or more of claims 3-4, wherein step a) is carried out by reaction with a linear or cyclic, or aromatic alkyl dicarbonate, preferably selected from di-tert-butyl dicarbonate, bis-(oxyrane-2-yl-methyl) dicarbonate, bis (trifluoromethyl) dicarbonate, bis(2-ethoxycyclohexyl) dicarbonate o dibenzyl dicarbonate, even more preferably di-tert-butyl dicarbonate.

6. The process according to one or more of claims 3-5, wherein step a) is carried out at a temperature ranging from 20 to 30°C for 10 to 32 hours, preferably for around 24 hours, in a solvent which is a 1:1 mixture by volume of 1,4-dioxane and water or a 1:1 mixture by volume of THF and water, with di (tert-butyl-dicarbonate) present in a molar ratio ranging from 2 to 1, preferably from 1.3 to 1, with respect to the β-lactam compound.

7. The process according to one or more of claims 3-6, wherein step b) is carried out under anhydrous conditions using a solvent selected from diethyl ether, 1,4-dioxane, THF or diethyl ether in a 20:1 ratio by volume with 1,4 dioxane or chloroform or DCM, or THF, in the presence of an amine selected from trimethylamine, dimethylaminopyridine, DIPEA or anhydrous pyridine.

8. The process according to one or more of claims 3-7, wherein step b) is carried out at a temperature ranging from -5°C to 0°C until the addition of the nucleophile, and then at room temperature for a time ranging from 24 to 52 hours, preferably from 24 to 36 hours.

9. The process according to one or more of claims 4-8, wherein step c) is a cycloaddition reaction mediated by copper in oxidation state I, wherein the compound comprising an alkyne group is propargyl alcohol.

10. The process according to one or more of claims 4-9, wherein step c) is carried out in a mixture of water and an alkyl alcohol having from 1 to 3 carbon atoms, preferably in water and isopropanol (IPA) 1:1 by volume, at a temperature ranging from 55 to 60°C, preferably from 58 to 60°C, for a time ranging from 4 to 10 hours, preferably from 4.5 to 6 hours.

## Patentansprüche

1. Verbindung, ausgewählt aus den folgenden:
3-Azidopropyl-(2S,5R,6R)-6-((tert-butoxycarbonyl)-amino)-3,3-dimethyl-7-oxo-4-thio-1-azabicyclo[3.2.0]heptan-2-carboxylat **IA** 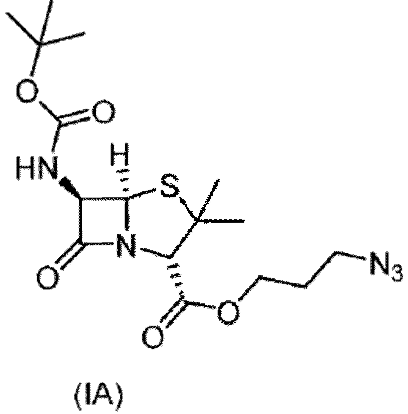
3-Azidopropyl-(2R,6R,7R)-3-(acetoxymethyl)-7-((tert-butoxycarbonyl)-amino)-8-oxo-5-thio-1-azabicyclo[4.2.0]oct-3-en-2-carboxylat **IIA** 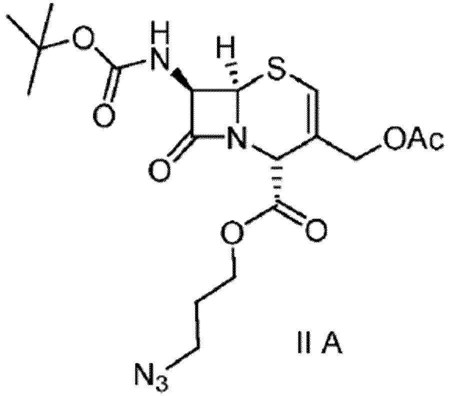
3-Azidopropyl-(6R,7R)-3-(acetoxymethyl)-7-((tert-butoxycarbonyl)-amino)-8-oxo-5-thio-1-azabicyclo[4.2.0]oct-2-en-2-carboxylat IIA' 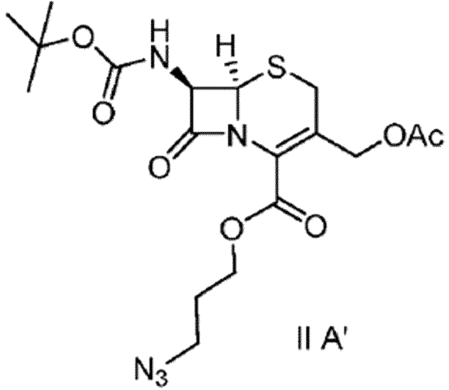
3-(4-(Hydroxymethyl)-1H-1,2,3-triazol-1-yl)-propyl-(2S,5R,6R)-6-((tert-butoxycarbonyl)-amino)-3,3-dimethyl-7-oxo-4-thio-1-azabicyclo[3.2.0]heptan-2-carboxylat **1B** 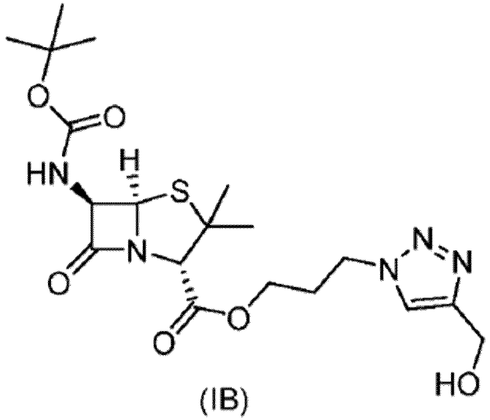
3-(4-(Hydroxymethyl)-1H-1,2,3-triazol-1-yl)-propyl-(2R,6R,7R)-3-(acetoxy-methyl)-7-((tert-butoxycarbonyl)-amino)-8-oxo-5-thio-1-azabicyclo[4.2.0]oct-3-en-2-carboxylat **IIB** 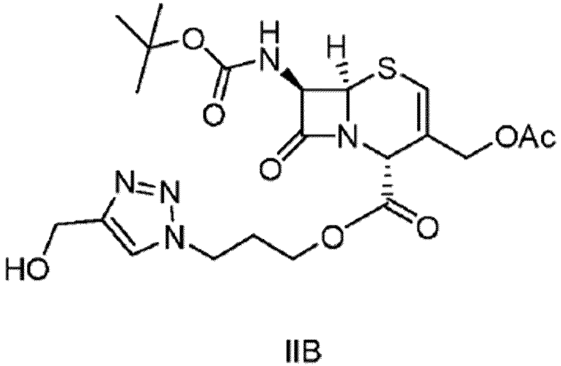
3-(4-(Hydroxymethyl)-1H-1,2,3-triazol-1-yl)-propyl-(6R,7R)-3-(acetoxymethyl)-7-((tert-butoxycarbonyl)-amino)-8-oxo-5-thio-1-azabicyclo[4.2.0]oct-2-en-2-carboxylat **IIB'** 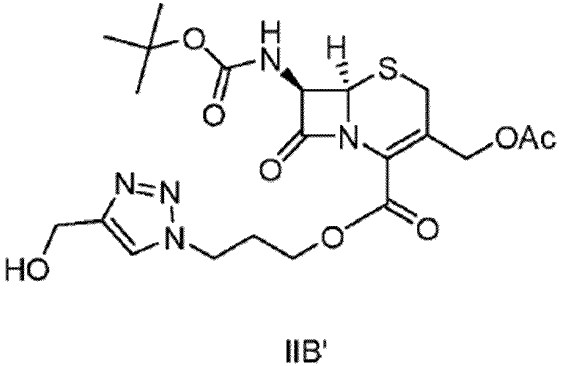
3-(4-((((2S,5R,6R)-6-((tert-Butoxycarbonyl)-amino)-3,3-dimethyl-7-oxo-4-thio-1-azabicyclo[3.2.0]heptan-2-carbonyl)oxy)-methyl)-1H-1,2,3-triazol-1-yl)-propyl-(2S,5R,6R)-6-((tert-butoxycarbonyl)-amino)-3,3-dimethyl-7-oxo-4-thio-1-azabicyclo[3.2.0]heptan-2-carboxylat **IC** 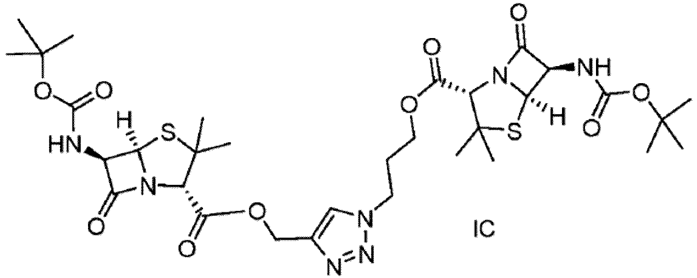
3-(4-((((2S,5R,6R)-6-((*tert*-Butoxycarbonyl)-amino)-3,3-dimethyl-7-oxo-4-thio-1-azabicyclo[3.2.0]heptan-2-carbonyl)oxy)-methyl)-IH-1,2,3-triazol-1-yl)-propyl-(2R,6R,7R)-3-(acetoxymethyl)-7-((tert-butoxycarbonyl)-amino)-8-oxo-5-thio-1-azabicyclo[4.2.0]oct-3-en-2-carboxylat IIC 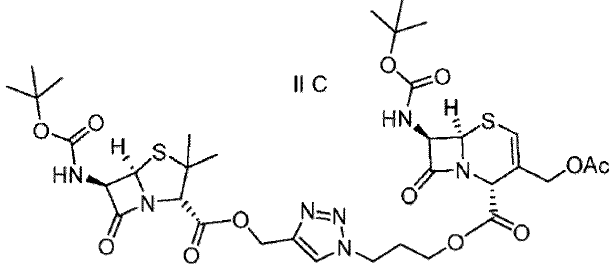
3-(4-((((2S,5R,6R)-6-((tert-Butoxycarbonyl)-amino)-3,3-dimethyl-7-oxo-4-thio-1-azabicyclo[3.2.0]heptan-2-carbonyl)oxy)-methyl)-IH-1,2,3-triazol-1-yl)-propyl-(6R,7R)-3-(acetoxymethyl)-7-((tert-butoxycarbonyl)-amino)-8-oxo-5-thio-1-azabicyclo[4.2.0]oct-2-en-2-carboxylat **IIC'** 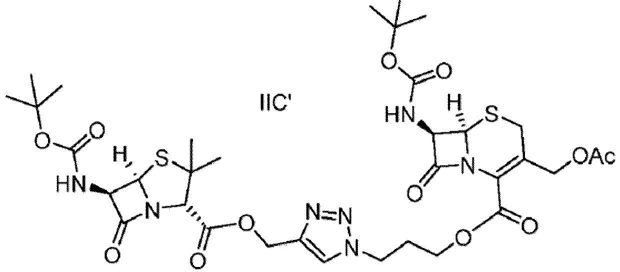
3-(4-((Oxyran-2-yl-methoxy)-methyl)-IH-1,2,3-triazol-1-yl)-propyl-(2S,5R,6R)-6-((tert-butoxycarbonyl)-amino)-3,3-dimethyl-7-oxo-4-thio-1-azabicyclo-[3.2.0]heptan-2-carboxylat **ID** 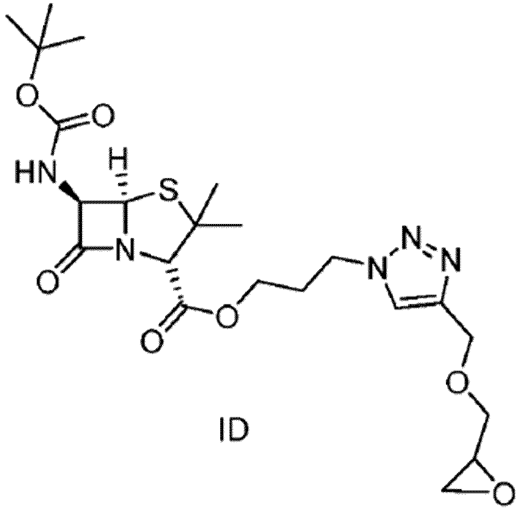
3-(4-((Oxyran-2-yl-methoxy)-methyl)-1H-1,2,3-triazol-1-yl)-propyl-(2R,6R,7R)-3-(acetoxymethyl)-7-((tert-butoxycarbonyl)-amino)-8-oxo-5-thio-1-azabicyclo-[4.2.0]oct-3-en-2-carboxylat **IID** 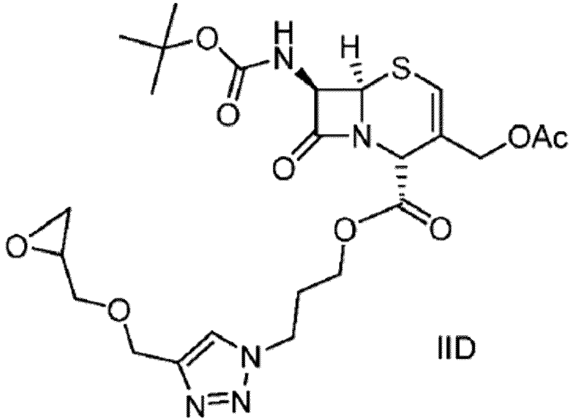
3-(4-((Oxyran-2-yl-methoxy)-methyl)-1H-1,2,3-triazol-1-yl)-propyl-(6R,7R)-3-(acetoxymethyl)-7-((tert-butoxycarbonyl)-amino)-8-oxo-5-thio-1-azabicyclo-[4.2.0]oct-2-en-2-carboxylat **IID'** 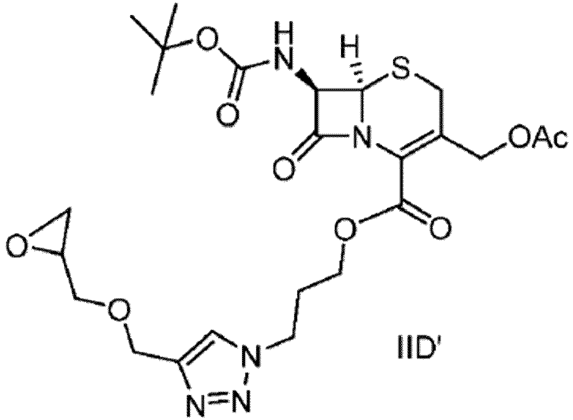
3-(4-Formyl-1H-1,2,3-triazol-1-yl)-propyl-(2S,5R,6R)-6-((tert-butoxycarbonyl)-amino)-3,3-dimethyl-7-oxo-4-thio-1-azabicyclo[3.2.0]heptan-2-carboxylat **IF** 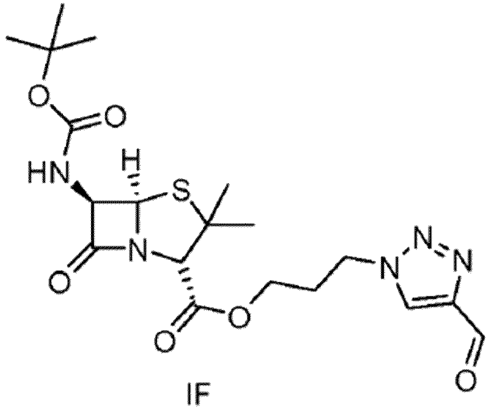
3-(4-Formyl-1H-1,2,3-triazol-1-yl)-propyl-(2R,6R,7R)-3-(acetoxymethyl)-7-((tert-butoxycarbonyl)-amino)-8-oxo-5-thio-1-azabicyclo[4.2.0]oct-3-en-2-carboxylat **IIF** 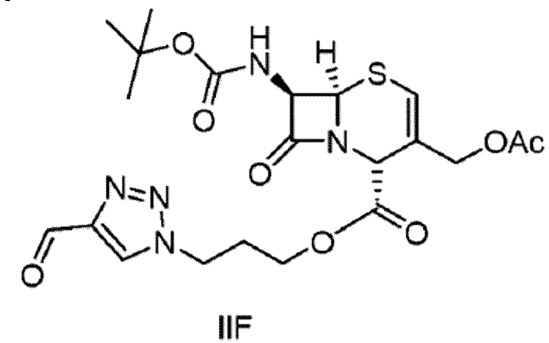
3-(4-Formyl-1H-1,2,3-triazol-1-yl)-propyl-(6R,7R)-3-(acetoxymethyl)-7-((tert-butoxycarbonyl)-amino)-8-oxo-5-thio-1-azabicyclo[4.2.0]oct-2-en-2-carboxylat IIF' 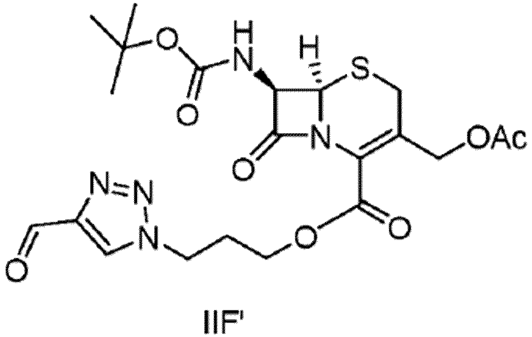
1,3-Diazidopropan-2-yl(2S,5R,6R)-6-((tert-butoxycarbonyl)amino)-3,3-dimethyl-7-oxo-4-thia-1-azabicyclo[3.2.0]heptan-2-carboxylat IJ 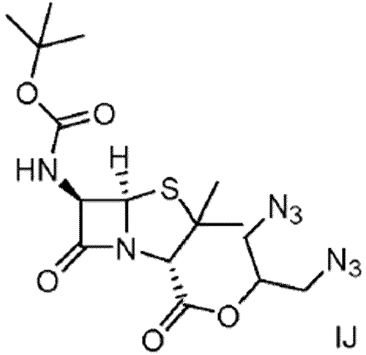
tert-Butyl((2S,5R,6R)-2-((3-azidopropyl)carbamoyl)-3,3-dimethyl-7-oxo-4-thio-1-azabicyclo[3.2.0]heptan-6-yl)carbamat IL 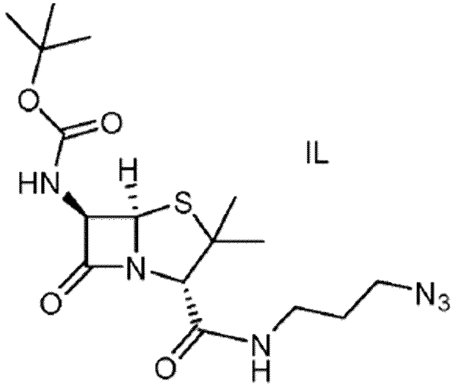
((2R,6R,7R)-2-((3-Azidopropyl)carbamoyl)-7-((tert-butoxycarbonyl)amino)-8-oxo-5-thio-1-azabicyclo[ 4.2.0]oct-3-en-3-yl)methylacetat **IIL** 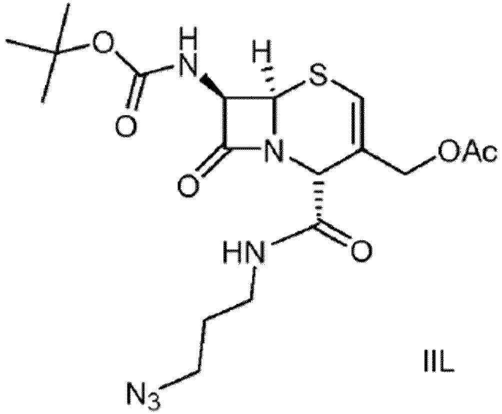
((6R,7R)-2-((3-Azidopropyl)carbamoyl)-7-((tert-butoxycarbonyl)amino)-8-oxo-5-thio-1-azabicyclo[4.2.0]oct-2-en-3-yl)methylacetat IIL' 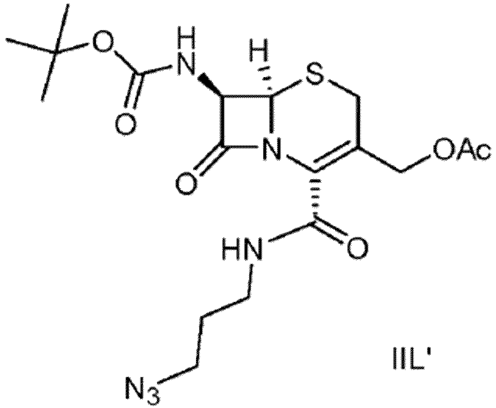
tert-Butyl-((2S,5R,6R)-2-((3-(4-(hydroxymethyl)-1H-1,2,3-triazol-1-yl)propyl)carbamoyl)-3,3-dimethyl-7-oxo-4-thio-1-azabicyclo[3.2.0] heptan-6-yl)carbamat **IM** 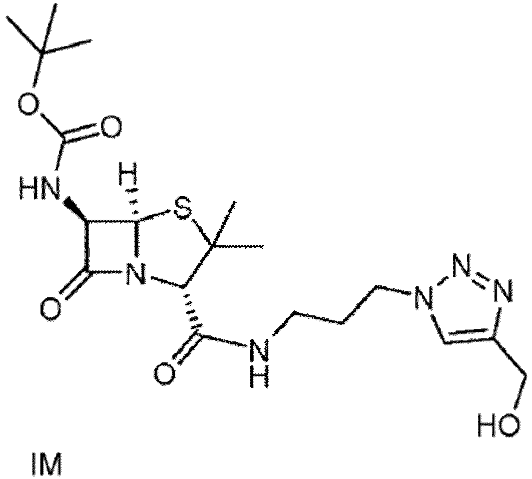
tert-Butyl ((2S,5 R,6 R)-2-(bis(2-azidoethyl)carbamoyl)-3,3-dimethyl-7-oxo-4-thia-1-azabicyclo[3.2.0]heptan-6-yl)carbamat **IN** 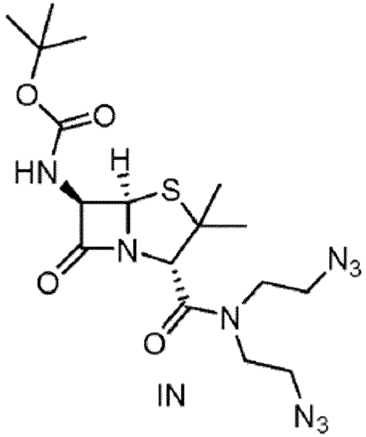
((6R,7R)-2-(Bis(2-azidoethyl)carbamoyl)-7-((tert-butoxycarbonyl)amino)-8-oxo-5-thia-1-azabicyclo[4.2.0]oct-2-en-3-yl)methylacetat **IIN'** 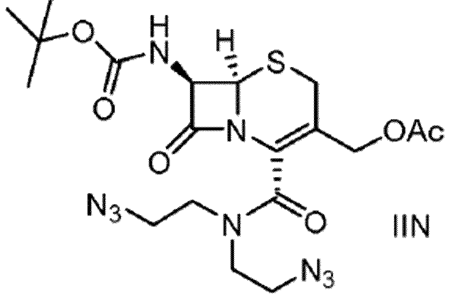
tert-Butyl((2S,5R,6R)-2-(bis(2-(4-(hydroxymethy**l**)-IH-1,2,3-triazol-1-yl)ethy**l)-**carbamoyl)-3,3-dimethy**l**-7-oxo-4-thia-1-azabicyclo[3.2.0]heptan-6-yl)carbamat **IP**

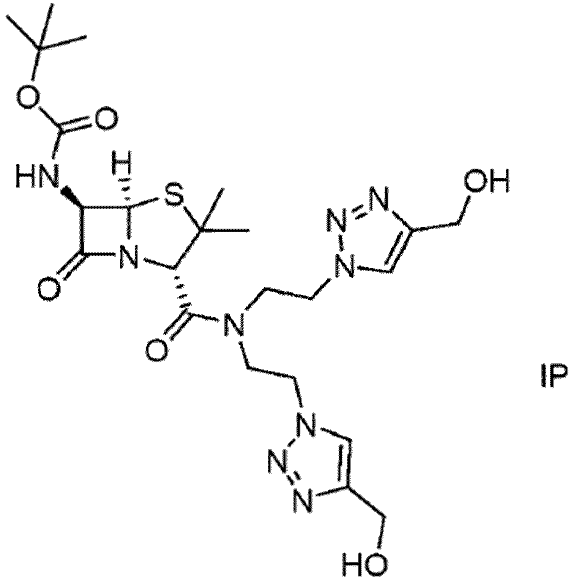

2. Verbindung nach Anspruch 1 oder ein Gemisch, umfassend zwei oder mehr Verbindungen nach Anspruch 1, zur Verwendung als Medikament oder als Vorstufe eines Medikaments, vorzugsweise als antibiotisches Medikament oder als Beta-Lactamase-Inhibitor, als Antimykotikum und/oder Antitumormittel.

3. Verfahren zur Synthese von Vorstufen von Derivaten von -Lactamverbindungen, ausgewählt aus 6-Aminopenicillansäure (6-APA) und 7-Aminocephalosporansäure (7-ACA), vorzugsweise 6-Aminopenicillansäure (6-APA), nach Anspruch 1, umfassend die folgenden Schritte:
a) Schutz der Aminogruppe in Position 6 von 6-APA oder in Position 7 von 7-ACA durch Bildung eines Carbamats;
b) Veresterung der Carboxylgruppe der in Schritt a) erhaltenen -Lactamverbindung durch Umsetzung mit einem aliphatischen Alkohol aufweisend von 3 bis 10 Kohlenstoffatomen, bevorzugt von 3 bis 5, besonders bevorzugt 3, enthaltend mindestens eine Azidgruppe, oder Amidierung der Carboxylgruppe der in Schritt a) erhaltenen - Lactamverbindung durch Umsetzung mit einem primären aliphatischen Amin aufweisend von 3 bis 10 Kohlenstoffatomen, bevorzugt von 3 bis 5, besonders bevorzugt 3, und enthaltend eine Azidgruppe, oder mit einem sekundären aliphatischen Amin aufweisend von 4 bis 10 Kohlenstoffatomen, bevorzugt 4, und enthaltend zwei Azidgruppen, um ein tertiäres Amid zu erhalten.

4. Verfahren zur Synthese von Triazolderivaten von -Lactam nach Anspruch 1, wobei die nach Anspruch 3 erhaltene Vorstufe dem folgenden Schritt e) unterworfen wird:
c) Cycloadditionsreaktion zwischen dem in Schritt b) erhaltenen Derivat aufweisend mindestens eine Azidgruppe und einer Verbindung aufweisend mindestens eine Alkingruppe (C=C), um das entsprechende Triazolderivat der -Lactamverbindung zu erhalten;
und wobei optional das Cycloadditionsreaktionsprodukt von Schritt e) dem folgenden Schritt d) unterworfen wird:
d) die am Ende von Schritt e) erhaltene Vorstufe, die eine primäre Alkoholgruppe enthält, einer Oxidation der primären Alkoholgruppe unterworfen wird, um ein Aldehydderivat zu erhalten.

5. Verfahren nach einem oder mehreren der Ansprüche 3-4, wobei Schritt a) durch Umsetzung mit einem linearen oder cyclischen oder aromatischen Alkyldicarbonat, vorzugsweise ausgewählt aus *Di-*tert-butyldicarbonat, Bis-(oxyrane-2-yl-methyl)dicarbonat, Bis(trifluormethyl)dicarbonat, Bis(2-ethoxycyclohexyl)dicarbonat oder Dibenzyldicarbonat, noch bevorzugter *Di-tert-butyl*dicarbonat, durchgeführt wird.

6. Verfahren nach einem oder mehreren der Ansprüche 3-5, wobei Schritt a) bei einer Temperatur im Bereich von 20 bis 30°C für 10 bis 32 Stunden, vorzugsweise für etwa 24 Stunden, in einem Lösungsmittel durchgeführt wird, das ein 1:1-Volumengemisch aus 1,4-Dioxan und Wasser oder ein 1:1-Volumengemisch aus THF und Wasser ist, wobei Di-(tert-butyldicarbonat) in einem Molverhältnis im Bereich von 2 bis 1, vorzugsweise von 1,3 bis 1, in Bezug auf die-Lactamverbindung vorliegt.

7. Verfahren nach einem oder mehreren der Ansprüche 3-6, wobei Schritt b) unter wasserfreien Bedingungen unter Verwendung eines Lösungsmittels, ausgewählt aus Diethylether, 1,4-Dioxan, THF oder Diethylether in einem Volumenverhältnis von 20:1 mit 1,4-Dioxan oder Chloroform oder DCM oder THF, in Gegenwart eines Amins, ausgewählt aus Trimethylamin, Dimethylaminopyridin, DIPEA oder wasserfreiem Pyridin, durchgeführt wird.

8. Verfahren nach einem oder mehreren der Ansprüche 3-7, wobei Schritt b) bei einer Temperatur im Bereich von -5 °C bis 0 °C bis die Zugabe des Nucleophils und dann bei Raumtemperatur über einen Zeitraum im Bereich von 24 bis 52 Stunden, vorzugsweise von 24 bis 36 Stunden, durchgeführt wird.

9. Verfahren nach einem oder mehreren der Ansprüche 4-8, wobei Schritt e) eine durch Kupfer in der Oxidationsstufe I vermittelte Cycloadditionsreaktion ist, wobei die Verbindung, die eine Alkingruppe umfasst, Propargylalkohol ist.

10. Verfahren nach einem oder mehreren der Ansprüche 4-9, wobei Schritt e) in einem Gemisch aus Wasser und einem Alkylalkohol aufweisend von 1 bis 3 Kohlenstoffatomen, vorzugsweise in Wasser und Isopropanol (IPA) mit 1:1 Volumen, bei einer Temperatur im Bereich von 55 bis 60 °C, vorzugsweise von 58 bis 60 °C, über einen Zeitraum im Bereich von 4 bis 10 Stunden, vorzugsweise von 4,5 bis 6 Stunden, durchgeführt wird.

## Revendications

1. Composé choisi parmi les suivants :
3-azidopropyl-(2S,5R,6R)-6-((tert-butoxycarbonyl)-amino)-3,3-diméthyl-7-oxo-4-thio-1 azabicyclo[3.2.O]heptane-2-carboxylate **IA** 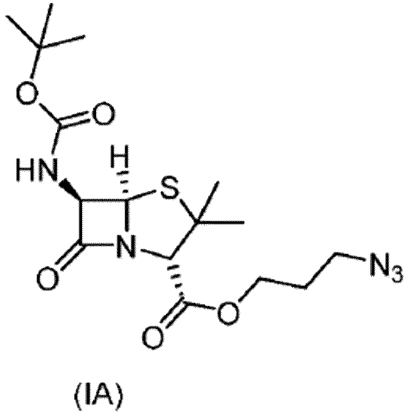
3-azidopropyl-(2R,6R,7R)-3-(acétoxyméthyl)-7-((tert-butoxycarbonyl)-amino)-8-oxo-5- thio-1-azabicyclo[4.2.O]oct-3-ène-2-carboxylate **IIA** 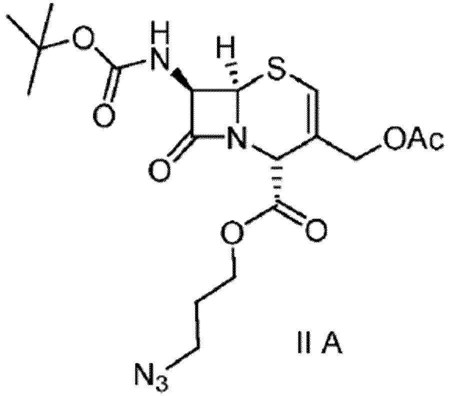
3-azidopropyl-(6R,7R)-3-(acétoxyméthyl)-7-((tert-butoxycarbonyl)-amino)-8-oxo-5-thio-1-azabicyclo[4.2.O]oct-2-ène-2-carboxylate **IIA'** 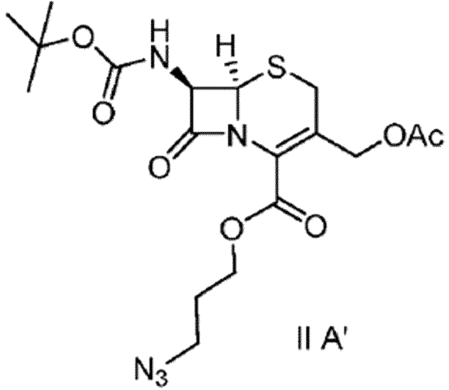
3-(4-(hydroxyméthyl)-1H-1,2,3-triazol-1-yl)-propyl-(2S,5R,6R)-6-((tert-butoxycarbonyl)-amino)-3,3-diméthyl-7-oxo-4-thio- I-azabicyclo[3.2.O]heptane-2-carboxylate **1B** 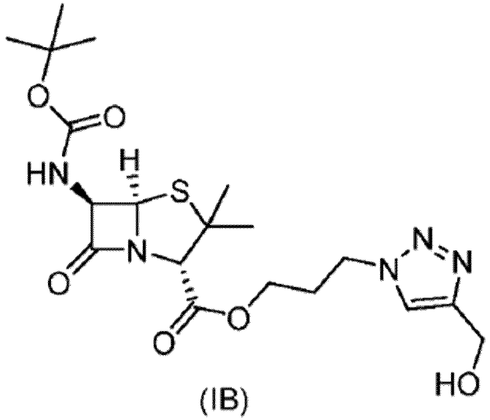
3-(4-(hydroxyméthyl)-1H-1,2,3-triazol-1-yl)-propyl-(2R,6R,7R)-3-(acétoxy-méthyl)-7- ((tert-butoxycarbonyl)-amino)-8-oxo-5-thio-1 - azabicyclo[4.2.0]oct-3-ène-2-carboxylate **IIB** 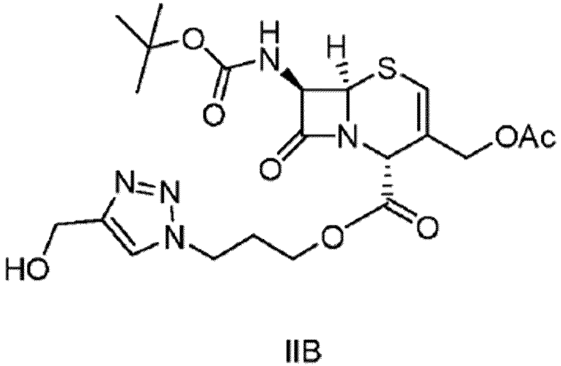
3-(4-(hydroxyméthyl)-1H-1,2,3-triazol-1-yl)-propyl-(6R,7R)-3-(acétoxyméthyl)-7-((tert-butoxycarbonyl)-amino)-8-oxo-5-thio- I-azabicyclo[4.2.0]oct-2-ène-2-carboxylate **IIB'** 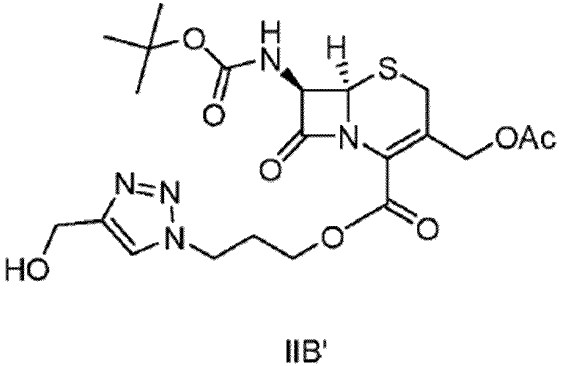
3-(4-((((2S,5R,6R)-6-((tert-butoxycarbonyl)-amino)-3,3-diméthyl-7-oxo-4-thio-1 azabicyclo[3.2.0]heptane-2-carbonyl)oxy)-méthyle)-1H-1,2,3-triazol-1-yl)-propyl-(2S,5R,6R)-6-((tert-butoxycarbonyl)-amino)-3,3-diméthyl-7-oxo-4-thio-1-azabicyclo[3.2.0]heptane-2-carboxylate **IC** 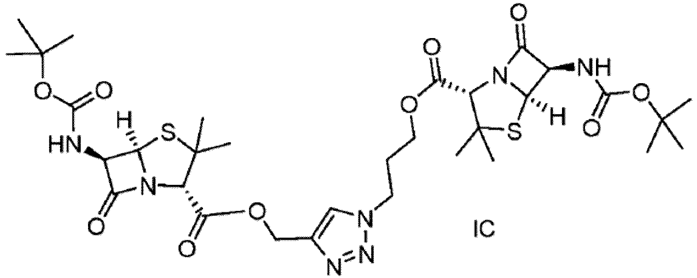
3-(4-((((2S,5R,6R)-6-((tert-butoxycarbonyl)-amino)-3,3-diméthyl-7-oxo-4-thio-1 azabicyclo[3.2.O]heptane-2-carbonyl)oxy)-méthyl)- IH- I,2,3-triazol- I-yl)-propyl (2R,6R,7R)-3-(acétoxyméthyl)-7-((tert-butoxycarbonyl)-amino)-8-oxo-5-thio-1-azabicyclo[4.2.O]oct-3-ène-2-carboxylate IIC 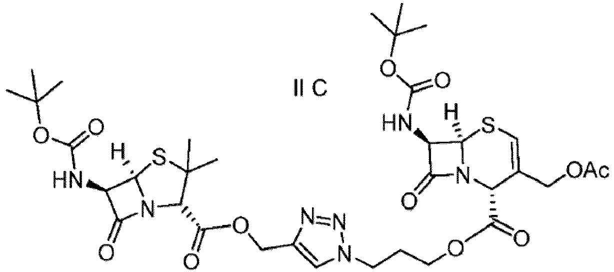
3-(4-((((2S,5R,6R)-6-((tert-butoxycarbonyl)-amino)-3,3-diméthyl-7-oxo-4-thio-1 azabicyclo[3.2.O]heptane-2-carbonyl)oxy)-méthyl)- IH- 1,2,3-triazol- I-yl)-propyl- (6R,7R)-3-(acétoxyméthyl)-7-((tert-butoxycarbonyl)-amino)-8-oxo-5-thio-1-azabicyclo [4.2.0] oct-2-ène-2-carboxylate **IIC'** 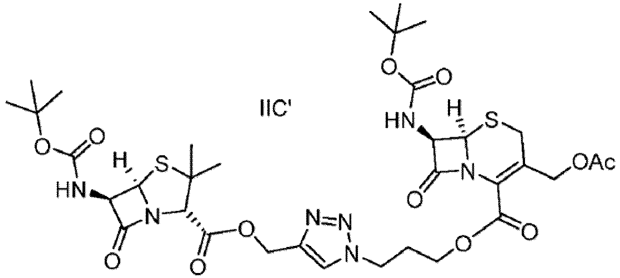
3-(4-((oxyran-2-yl-méthoxy)-méthyl)-IH- I,2,3-triazol- I-yl)-propyl-(2S,5R,6R)-6-((tert butoxycarbonyl)-amino)-3,3-diméthyl-7-oxo-4-thio- I-azabicyclo-[3.2.O]heptane-2- carboxylate **ID** 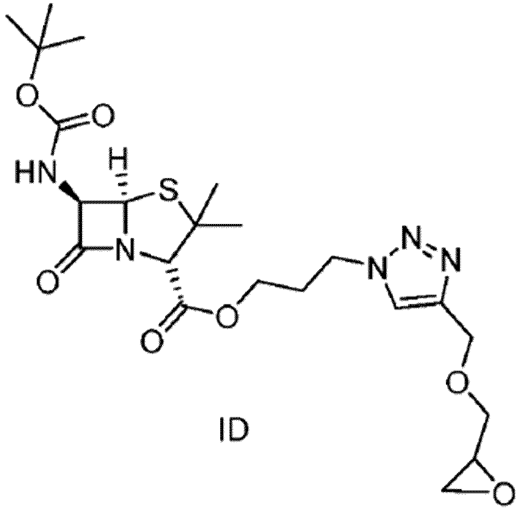
3-(4-((oxyran-2-yl-méthoxy)-méthyl)-1H-1 ,2,3-triazol-1-yl)-propyl-(2R,6R,7R)-3-(acétoxyméthyl)-7-((tert-butoxycarbonyl)-amino)-8-oxo-5-thio-1-azabicyclo-[4.2.O]oct-3-ène-2-carboxylate **IID** 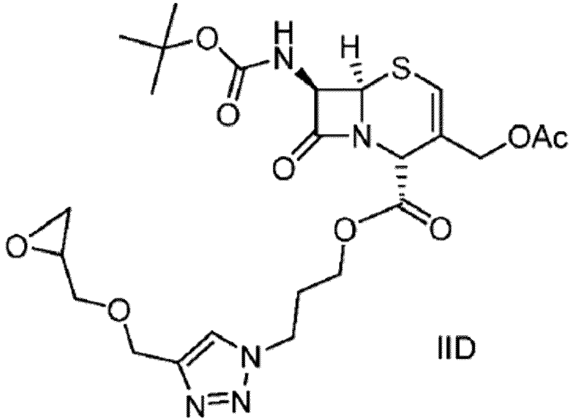
3-(4-((oxyran-2-yl-méthoxy)-méthyl)-1H-1,2,3-triazol-1-yl)-propyl-(6R,7R)-3-(acétoxyméthyl)-7-((tert-butoxycarbonyl)-amino)-8-oxo-5-thio-1-azabicyclo-[4.2.O]oct-2-ène-2-carboxylate **IID'** 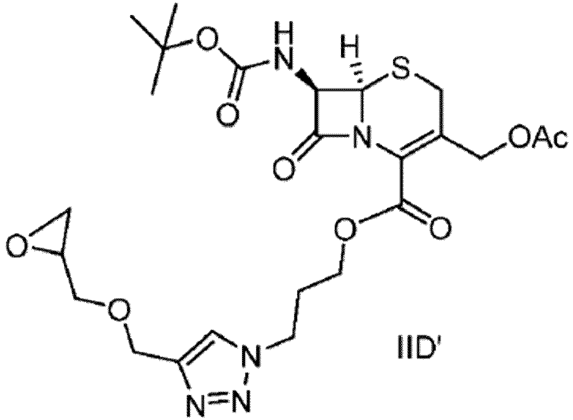
3-(4-formyl-1H-1,2,3-triazol-1-yl)-propyl-(2S,5R,6R)-6-((tert-butoxycarbonyl)-amino)- 3,3-diméthyl-7-oxo-4-thio-1-azabicyclo[3.2.O]heptane-2-carboxylate **IF** 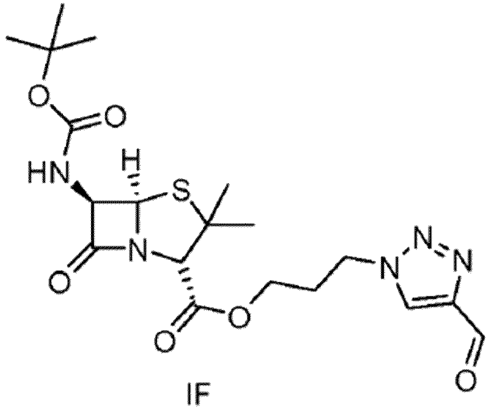
3-(4-formyl-1H-1,2,3-triazol-1-yl)-propyl-(2R,6R, 7R)-3-(acétoxyméthyl)-7-((tert- butoxycarbonyl)-amino)-8-oxo-5-thio-1-azabicyclo[4.2.0]oct-3-ène-2-carboxylate **IIF** 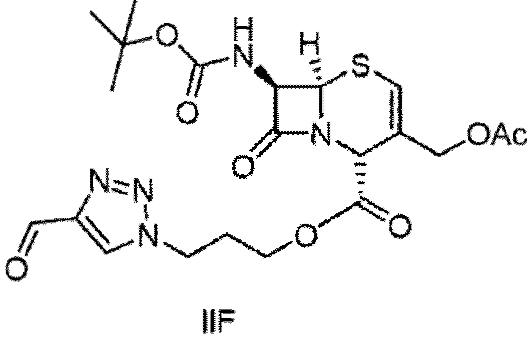
3-(4-formyl-1H-1,2,3-triazol-1-yl)-propyl-(6R,7R)-3-(acétoxyméthyl)-7-((tert butoxycarbonyl)-amino)-8-oxo-5-thio-1-azabicyclo[4.2.0]oct-2-ène-2-carboxylate **IIF'** 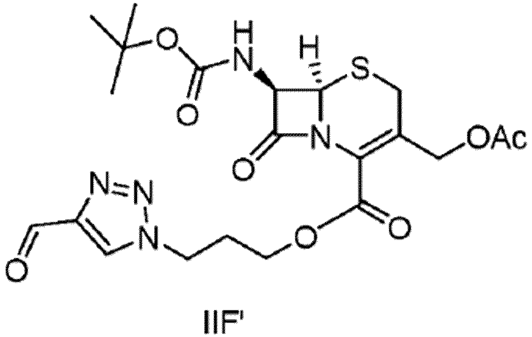
1,3-diazidopropan-2-yl(2S,5R,6R)-6-((tert-butoxycarbonyl) amino)-3,3-diméthyl-7-oxo- 4-thia- I-azabicyclo[3.2.0]heptane-2-carboxylate **IJ** 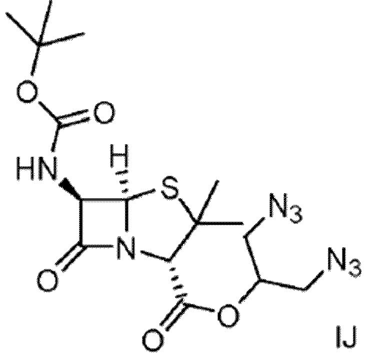
tert-butyl ((2S,5R,6R)-2-((3-azidopropyl)carbamoyl)-3,3-diméthyl-7-oxo-4-thio- I- azabicyclo[3.2.0]heptan-6-yl)carbamate **IL** 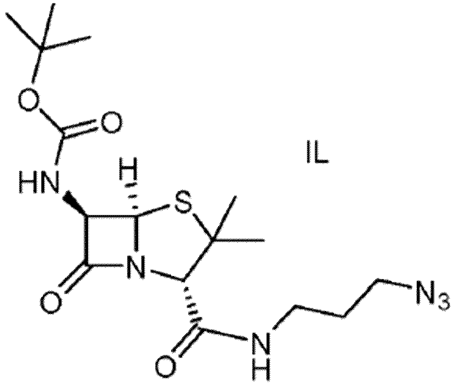
((2R,6R,7R)-2-((3-azidopropyl)carbamoyl)-7-((tert-butoxycarbonyl)amino)-8-oxo-5-thio I-azabicyclo[ 4.2.0]oct-3-én-3-yl)acétate de méthyle **IIL** 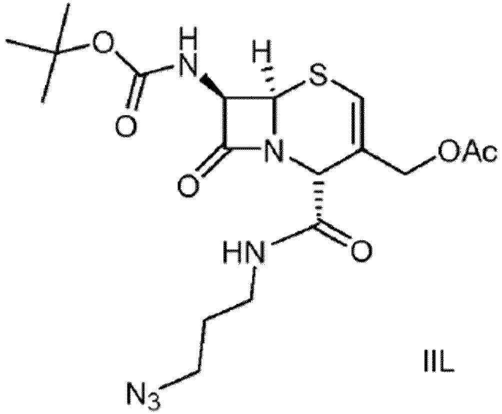
((6R,7R)-2-((3-azidopropyl)carbamoyl)-7-((tert-butoxycarbonyl)amino)-8-oxo-5-thio-1 azabicyclo[4.2.0]oct-2-én-3-yl)acétate de méthyle **IIL'** 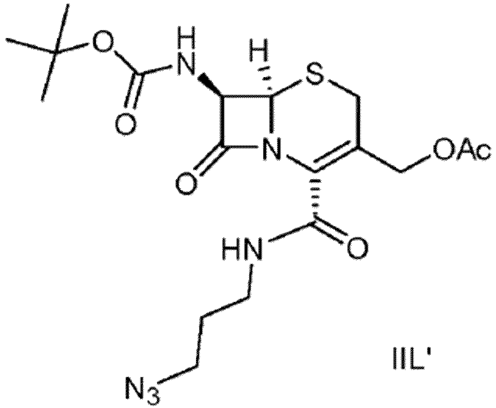
tert-butyl-((2S,5R,6R)-2-((3-(4-(hydroxyméthyl)-IH-I,2,3-triazol-I yl)propyl)carbamoyl)-3,3-diméthyl-7-oxo-4-thio- I-azabicyclo[3.2.0]heptan-6- yl)carbamate **IM** 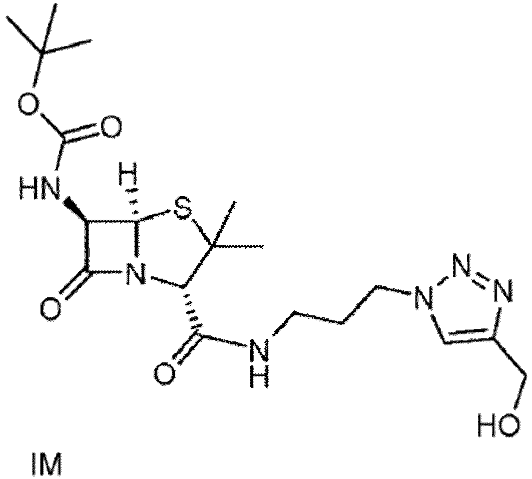
tert-butyl((2S,5R,6R)-2-(bis(2-azidoéthyl)carbamoyl)-3,3-diméthyl-7-oxo-4-thia-I azabicyclo[3.2.O]heptan-6-yl)carbamate **IN** 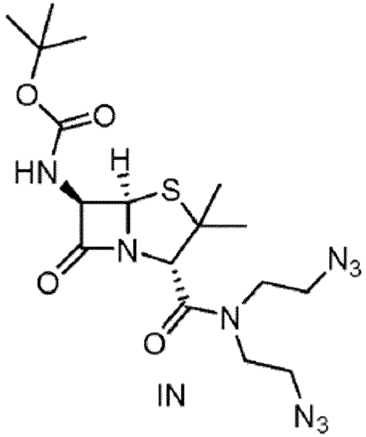
((6R,7R)-2-(bis(2-azidoéthyl)carbamoyl)-7-((tert-butoxycarbonyl)amino)-8-oxo-5-thia-I azabicyclo[4.2.O]oct-2-én-3-yl)acétate de méthyle **IIN'** 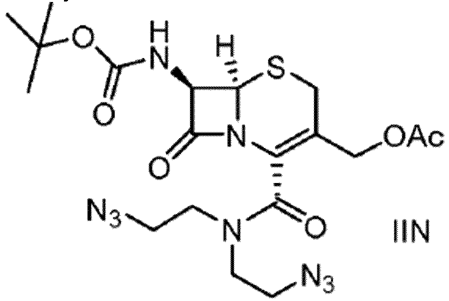
tert-butil((2S,5R,6R)-2-(bis(2-(4-(hydroxyméthy**l)-** IH-1,2,3-triazol-1-yl)éthy**l)** carbamoyl)-3,3-diméthy **I-**7-oxo-4-thia- I -azabicyclo[3 .2.0]heptan-6-yl)carbamate **IP**

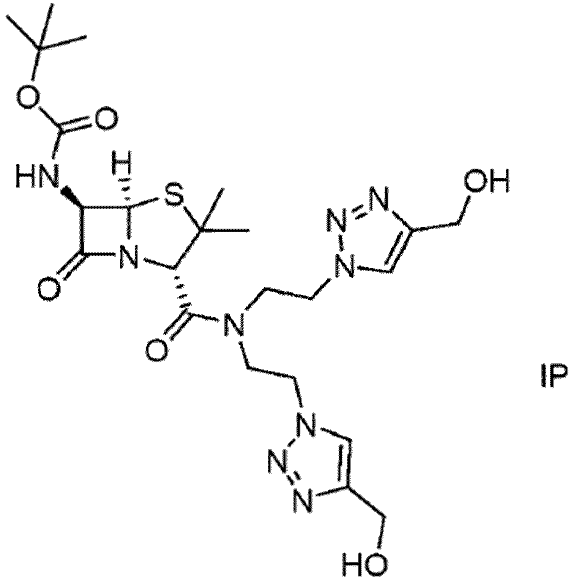

2. Composé selon la revendication 1, ou mélange comprenant deux ou plusieurs composés selon la revendication 1, destiné à être utilisé comme médicament ou comme précurseur de médicament, de préférence comme médicament antibiotique ou comme inhibiteur de bêta-lactamase, comme antifongique et/ou comme agent antitumoral.

3. Procédé de synthèse de précurseurs de dérivés de composés -lactame choisis parmi l'acide 6-aminopénicillanique (6-APA) et l'acide 7-amino-céphalosporanique (7- ACA), de préférence l'acide 6-aminopénicillanique (6-APA), selon la revendication 1, qui comprend les étapes suivantes :
a) protection du groupe amino en position 6 du 6-APA ou en position 7 du 7-ACA par la formation d'un carbamate ;
b) estérification du groupe carboxyle du composé -lactame obtenu à l'étape a) par réaction avec un alcool aliphatique ayant de 3 à 10 atomes de carbone, de préférence de 3 à 5, plus préférentiellement 3, et comprenant au moins un groupe azide ou amidation du groupe carboxyle du composé -lactame obtenu à l'étape a) par réaction avec une amine aliphatique primaire ayant de 3 à 10 atomes de carbone, de préférence de 3 à 5, plus préférentiellement 3, et comprenant un groupe azide ou avec une amine aliphatique secondaire ayant de 4 à 10 atomes de carbone, de préférence 4, et comprenant deux groupes azides pour obtenir un amide tertiaire.

4. Procédé de synthèse de dérivés triazoles de -lactame selon la revendication 1, dans lequel le précurseur obtenu selon la revendication 3 est soumis à l'étape e) suivante :
c) réaction de cycloaddition entre le dérivé obtenu à l'étape b) ayant au moins un groupe azide et un composé ayant au moins un groupe alcyne (C=C), pour former le dérivé triazole correspondant du composé -lactame ;
et dans lequel, éventuellement, le produit de la réaction de cycloaddition de l'étape e) est soumis à l'étape d) suivante :
d) le précurseur obtenu à l'issue de l'étape e) et comprenant un groupe alcool primaire est soumis à une oxydation du groupe alcool primaire pour obtenir un dérivé aldéhydique.

5. Procédé selon une ou plusieurs des revendications 3-4, dans lequel l'étape a) est effectuée par réaction avec un dicarbonate d'alkyle linéaire ou cyclique, ou aromatique, de préférence choisi parmi le dicarbonate de *di-tert-butyl*, le dicarbonate de bis-(oxyrane-2-yl-méthyle), le dicarbonate de bis (trifluorométhyle), le dicarbonate de bis(2-éthoxycyclohexyle) ou le dicarbonate de dibenzyle, de préférence encore le dicarbonate de *di-tert-butyl.*

6. Procédé selon une ou plusieurs des revendications 3 à 5, dans lequel l'étape a) est effectuée à une température allant de 20 à 30°C pendant 10 à 32 heures, de préférence pendant environ 24 heures, dans un solvant qui est un mélange 1:1 en volume de 1,4-dioxane et d'eau ou un mélange 1:1 en volume de THF et d'eau, avec du di (tert-butyl-dicarbonate) présent dans un rapport molaire allant de 2 à 1, de préférence de 1,3 à 1, par rapport au composé de -lactame.

7. Procédé selon une ou plusieurs des revendications 3 à 6, dans lequel l'étape b) est effectuée dans des conditions anhydres en utilisant un solvant choisi parmi l'éther diéthylique, le 1,4-dioxane, le THF ou l'éther diéthylique dans un rapport de 20:1 en volume avec le 1,4-dioxane ou le chloroforme ou le DCM, ou le THF, en présence d'une amine choisie parmi la triméthylamine, la diméthylaminopyridine, la DIPEA ou la pyridine anhydre.

8. Procédé selon une ou plusieurs des revendications 3 à 7, dans lequel l'étape b) est effectuée à une température comprise entre - 5°C et 0°C jusqu'à l'ajout du nucléophile, puis à température ambiante pendant une durée comprise entre 24 et 52 heures, de préférence entre 24 et 36 heures.

9. Procédé selon l'une ou plusieurs des revendications 4 à 8, dans lequel l'étape e) est une réaction de cycloaddition médiée par le cuivre à l'état d'oxydation I, dans lequel le composé comprenant un groupe alcyne est l'alcool propargylique.

10. Procédé selon une ou plusieurs des revendications 4 à 9, dans lequel l'étape e) est effectuée dans un mélange d'eau et d'alcool alkyle ayant de 1 à 3 atomes de carbone, de préférence dans de l'eau et de l'isopropanol (IPA) 1:1 en volume, à une température allant de 55 à 60°C, de préférence de 58 à 60°C, pendant une durée allant de 4 à 10 heures, de préférence de 4,5 à 6 heures.
